(19) Europäisches Patentamt
European Patent Office
Office européen des brevets

(11) EP 4 600 252 A1

(12) EUROPEAN PATENT APPLICATION
published in accordance with Art. 153(4) EPC

(43) Date of publication:
13.08.2025 Bulletin 2025/33

(21) Application number: 22961608.1

(22) Date of filing: 09.10.2022

(51) International Patent Classification (IPC):
*C07H 1/02* (2006.01) *C07H 19/10* (2006.01)
*C07H 19/20* (2006.01) *C07H 21/00* (2006.01)
*C12Q 1/6869* (2018.01)

(52) Cooperative Patent Classification (CPC):
C07H 1/02; C07H 19/10; C07H 19/20; C07H 21/00;
C12Q 1/6869

(86) International application number:
PCT/CN2022/123981

(87) International publication number:
WO 2024/077410 (18.04.2024 Gazette 2024/16)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(71) Applicant: GeneMind Biosciences Company Limited
Shenzhen, Guangdong 518023 (CN)

(72) Inventors:
- CAI, Jinsen
Shenzhen, Guangdong 518023 (CN)
- LI, Yongzhi
Shenzhen, Guangdong 518023 (CN)
- YOU, Xuejiao
Shenzhen, Guangdong 518023 (CN)
- LUO, Ling
Shenzhen, Guangdong 518023 (CN)
- LIU, Lei
Shenzhen, Guangdong 518023 (CN)
- CHEN, Fang
Shenzhen, Guangdong 518023 (CN)
- SUN, Lei
Shenzhen, Guangdong 518023 (CN)

(74) Representative: Schiweck Weinzierl Koch Patentanwälte Partnerschaft mbB
Ganghoferstraße 68 B
80339 München (DE)

# (54) METHOD FOR REDUCING AZIDE METHOXY GROUP, SEQUENCING METHOD, AND KIT

(57) The present application relates to the technical field of metal ion chemistry, and provides a method for reducing an azidomethoxy group, a sequencing method, and a kit. The method for reducing the azidomethoxy group provided in the present application comprises: mixing a compound I comprising the azidomethoxy group, a palladium compound, and an organic phosphine to give a mixed system, and placing the mixed system under a condition suitable for a chemical reaction to reduce the azidomethoxy group of the compound I into hydroxy, wherein the azidomethoxy group has a chemical formula of $-O-CA_1(N_3)-A_2$, and $A_1$ is selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl. According to the method provided in the present application, the palladium compound is introduced into the reaction system, so that an oxidation-reduction reaction between the azidomethoxy group and the organic phosphine is accelerated.

## Description

### TECHNICAL FIELD

The present disclosure belongs to the technical field of metal-catalyzed chemical reactions, and particularly relates to a method for reducing an azidomethoxy group, a sequencing method, and a kit.

### BACKGROUND

Next-generation sequencing, also referred to as high-throughput sequencing or massively parallel sequencing, enables the determination of nucleic acid sequences of multiple samples in one sequencing run. One way to achieve this determination is multiplex sample analysis, also commonly referred to as multiplex library or multiplex sequencing. The more common next-generation sequencing methods include sequencing by ligation (SBL) and sequencing by synthesis (SBS). A platform for achieving nucleic acid sequencing based on sequencing by synthesis (SBS) is based on the base pairing principle, using a DNA polymerase to attach a nucleotide (including a nucleotide analog) to the 3' end of a primer bound to a template so as to controllably achieve single base extension, acquiring signal changes caused by each binding of the nucleotide analog, and further determining a base arrangement of the template based on the change of the acquired signals. A typical SBS method may include the following steps: (1) hybridizing a nucleic acid molecule to be detected with a probe on the surface of a solid carrier to attach a nucleic acid template to be detected to the surface of the solid carrier; (2) under the action of the DNA polymerase and under the condition suitable for a polymerase chain reaction, using the probe as a primer to enable the nucleotide analog (a reversible terminator and dNTPs) with a reversible terminating group and a fluorophore to be incorporated into the nucleic acid template; the reversible terminating group on the nucleotide analog is capable of preventing the nucleotide from being incorporated into the next position of the nucleic acid molecule to be detected, so that the single base extension is achieved; (3) exciting the fluorophore on the nucleotide analog to emit light, and then performing imaging on the surface to acquire a luminescence signal on the surface; (4) removing the reversible terminating group and the fluorophore in the nucleotide analog bound to the nucleic acid strand to be detected; (5) repeating the steps (2) and (4) described above to continue the extension of the nucleic acid strand to be detected. Further, the SBS method further includes step (6): determining the type of nucleotide analogs incorporated into the nucleic acid template in each cycle of the extension by analyzing the optical signal obtained in step (3); and reading the introduced nucleotide analog types in sequence, and finally obtaining all nucleotide sequences of the strand to be detected.

The reversible terminating group can bind to 3'-OH sites of dNTPs to block the incorporation of the nucleotide into the next position of the nucleic acid molecule to be detected. Common 3'-OH site reversible terminating groups are allyl, hemiacetal, amino, sulfydryl, and -O-azidomethoxy, and in step (4) of SBS, the reversible terminating group described above is cleaved by a cleavage reagent to detach it from the nucleotide analog. A 3'-O-azidomethoxy nucleotide reversible terminator (3'-O-azidomethoxy-dNTP) formed by taking -O-azidomethoxy as a reversible terminating group is an effective substrate of the DNA polymerase. In the SBS sequencing cycle, after 3'-O-azidomethyl-dNTP binds to the polymerase, a base is extended on an antisense strand of the nucleotide template through a polymerization reaction, and meanwhile, 3'-O-azidomethoxy in 3'-O-azidomethyl-dNTP can also prevent the polymerization reaction of the next dNTP; after -O-azidomethyl in 3'-O-azidomethyl-dNTP is removed (e.g., cleaved), the base is restored to 3'-OH-dNTP to prepare for the next cycle of the extension; these procedures are repeated to produce a nucleic acid strand in which bases are sequentially recognized. In addition, -O-azidomethyl can also serve as a chemically cleavable group for dNTPs for the purpose of carrying or removing a detectable group (such as a fluorophore) by its chemical cleavability. At present, common cleavage reagents for the 3'-O-azidomethoxy nucleotide are tris(3-hydroxypropyl)phosphine (THP or THPP), tris(2-carboxyethyl) phosphine (TCEP), tris(hydroxymethyl)phosphine (THMP) or tris(2-hydroxyethyl)phosphine (THEP), dithiothreitol (DTT), and the like. However, when cleaving -O-azidomethyl, these cleavage reagents generally require a long time to cleave -O-azidomethyl with high efficiency, resulting in low overall efficiency of the cleavage reaction. Therefore, it is of great significance to find a cleavage reagent capable of rapidly cleaving an O-azidomethyl group.

### SUMMARY

The embodiments of the present application are intended to provide a method for reducing an azidomethoxy group, a sequencing method, and a kit, and aim to solve the problem that when an existing cleavage reagent used for the azidomethoxy group cleaves - O-azidoalkyl, it requires a long time to fully reduce -O-azidoalkyl into hydroxy (the cleavage efficiency is more than 90%) or the cleavage efficiency of reducing -O-azidoalkyl into hydroxy is low, resulting in low overall efficiency of the cleavage reaction.

In order to achieve the purpose of the present application, the technical scheme adopted in the embodiments of the present application is as follows:

In a first aspect, an embodiment of the present application provides a method for reducing an azidomethoxy group, which includes: mixing a compound I containing the azidomethoxy group, a palladium compound, and an organic phosphine to give a mixed system, and placing the mixed system under a condition suitable for a chemical reaction to reduce the azidomethoxy group of the compound I into hydroxy,

wherein the azidomethoxy group has a chemical formula of $-O-CA_1(N_3)-A_2$;

$A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

According to the method provided by the present application, the palladium compound is introduced into the reaction system of the compound I containing the azidomethoxy group and the organic phosphine, and the palladium compound is used for promoting oxidation-reduction reaction between the compound containing the azidomethoxy group and the organic phosphine, so that the reaction process of reducing the azidomethoxy group into hydroxy is accelerated, thereby improving the reaction efficiency.

As one possible embodiment of the method for reducing the azidomethoxy group in the present application, the organic phosphine and the palladium compound in the mixed system are in a molar ratio of 2:1 to 2000:1. When the molar ratio of the two is within the range described above, the palladium compound can effectively improve the reaction efficiency of the organic phosphine for reducing the azidomethoxy group into hydroxy. Specifically, when the organic phosphine and the palladium compound are in a molar ratio of 2:1 to 2000:1, the compound I, the palladium compound, and the organic phosphine are mixed for 20-30 s, and the reduction efficiency of reducing the azidomethoxy group in the compound I into hydroxy can reach 90%-100%.

As one possible embodiment of the method for reducing the azidomethoxy group in the present application, in the mixed system, the palladium compound is selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound. The reaction mechanism of the palladium compound described above may be as follows: the monovalent palladium compound, the divalent palladium compound, the trivalent palladium compound, the tetravalent palladium compound, and the hexavalent palladium compound are first reduced into low-valence palladium, such as the Pd(0) compound, in the reaction process, and the low-valence palladium plays a catalytic role to promote the reaction process of reducing the azidomethoxy group by the organic phosphine.

As one possible embodiment of the method for reducing the azidomethoxy group in the present application, in the mixed system, the palladium compound is selected from at least one of a palladium halate, a palladium halide, an organic palladium carboxylate, and an allylpalladium halide dimer. The palladium compound described above can play a role as a catalyst, accelerate the reduction process of the azidomethoxy group by the organic phosphine, and finally improve the reduction efficiency of the azidomethoxy group in the compound I.

In one possible implementation of this embodiment, the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate. The divalent palladium compound has better stability, and in the process of reducing the azidomethoxy group by the organic phosphine, the divalent palladium compound described above is helpful for accelerating the reduction of the azidomethoxy group in the compound I, thereby shortening the time required for the reduction of the azidomethoxy group into hydroxy.

As one possible embodiment of the method for reducing the azidomethoxy group in the present application, in the mixed system, the organic phosphine is selected from at least one of trivalent organic phosphines. In this case, the azidomethoxy group may form a complex system with the palladium compound, and bind to the trivalent organic phosphine at the same time to form a conjugate system, so that the formed conjugate-complex system may accelerate the rapid removal of one molecule of $N_2$ from the azidomethoxy group and further reduce the azidomethoxy group into a hydroxyl structure.

In one possible implementation of this embodiment, the organic phosphine is selected from at least one of tris(3-hydroxypropyl)phosphine, tris(2-carboxyethyl)phosphine, tris(hydroxymethyl)phosphine, triphenylphosphine, and tris(2-hydroxyethyl)phosphine. Compared with using the organic phosphine described above as a reducing agent alone, using the reaction system of the organic phosphine and the palladium compound described above can improve the reaction efficiency of reducing the azidomethoxy group into hydroxy.

As one possible embodiment of the method for reducing the azidomethoxy group in the present application, the palladium compound accelerates the process of the organic phosphine for reducing the azidomethoxy group into hydroxy under an alkaline condition, that is: the chemical reaction is carried out under the alkaline condition.

As one possible embodiment of the method for reducing the azidomethoxy group in the present application, the chemical reaction is carried out at a temperature of 30-100 °C. In this temperature range, compared with directly subjecting the organic phosphine and the azidomethoxy group to the oxidation-reduction reaction, a reaction system in which the palladium compound is used as a catalyst can improve the reaction efficiency of reducing the azidomethoxy group into hydroxy, and shorten the reaction time required when the cleavage efficiency of the compound I reaches an expected level.

As one possible embodiment of the method for reducing the azidomethoxy group in the present application, the mixed system for reducing the azidomethoxy group of the compound I into hydroxy further comprises metal ions selected from at least one of sodium ions and potassium ions. Maintaining a suitable concentration of sodium ions and/or potassium ions in the reaction system provides a suitable chemical reaction environment for the reduction of the azidomethoxy group by the organic phosphine, which is conducive to improving the cleavage efficiency.

In one possible implementation of this embodiment, the metal ions are derived from at least one of halide salts, carbonates, acetates, phosphates, and borates of the sodium ion and the potassium ion. The salts provided above can provide sodium ions and potassium ions through ionization, and are conducive to improving the reduction efficiency of the organic phosphine and the palladium compound on the azidomethoxy group by regulating the concentration of metal ions; moreover, anions of these salts have reaction inertia in the reaction system, which does not affect the reduction reaction process of the azidomethoxy group in the compound I by adopting the organic phosphine and the palladium compound.

In one possible implementation of this embodiment, the mixed system comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate. In one possible implementation of this embodiment, the metal ions in the mixed system have a concentration of 0.5-2.5 mol/L. When the concentration of the metal ions, i.e., sodium ions and/or potassium ions, is within the range described above, the activity of the organic phosphine catalyzed by the palladium compound is improved, and the reaction efficiency of reducing the azidomethoxy group into hydroxy is correspondingly improved; moreover, within this range, the higher the concentration of the metal ions, the stronger the reduction ability of the organic phosphine to the azidomethoxy group, and the higher the efficiency of the corresponding oxidation-reduction reaction.

As one possible embodiment of the method for reducing the azidomethoxy group in the present application, the mixed system further comprises a buffer. The buffer can provide a stable pH environment for the chemical reaction, is conducive to a stable reaction of reducing the azidomethoxy group into hydroxy, and finally improves the uniformity of the reduction reaction.

In one possible implementation of this embodiment, the buffer is selected from at least one of a primary amine buffer and a tertiary amine buffer. Compared with the primary amine buffer, the tertiary amine buffer can provide a more stable chemical reaction environment for the oxidation-reduction reaction between the compound I and the organic phosphine.

In one possible implementation of this embodiment, the buffer is selected from at least one of tris(hydroxymethyl) aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

As one possible embodiment of the method for reducing the azidomethoxy group in the present application, the buffer in the mixed system has a concentration of 50-200 mmol/L. When the concentration of the buffer is within the range described above, the buffer can provide a stable chemical reaction environment for the reaction of reducing the azidomethoxy group into hydroxy.

As one possible embodiment of the method for reducing the azidomethoxy group in the present application, the compound I has a structural general formula of $A_3$-O-$CA_1(N_3)$-$A_2$, wherein $A_3$ is selected from one of substituted or unsubstituted alkyl, substituted or unsubstituted cycloalkyl, substituted or unsubstituted heteroalkyl, substituted or unsubstituted heterocycloalkyl, substituted or unsubstituted aryl, and substituted or unsubstituted heteroaryl.

In one possible implementation of this embodiment, $A_3$ contains at least one of ribosyl or an analog thereof, deoxyribosyl or an analog thereof, a base or an analog thereof, a phosphate group, and a detectable group. In some implementations, A3 includes at least one of the following structures:

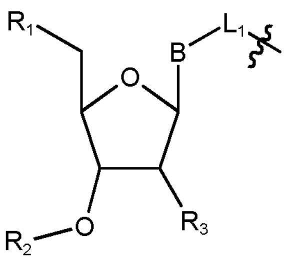

and

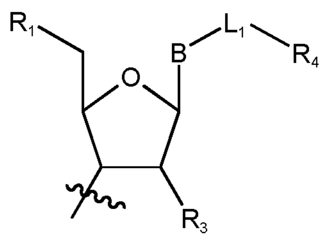

,

wherein B is a base or an analog thereof, $L_1$ is a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ contains a detectable group.

As one possible embodiment of the method for reducing the azidomethoxy group in the present application, at least one of $A_1$ and $A_2$ is hydrogen. In this case, the reactivity between the organic phosphine and the compound I is higher, which may be related to a decrease in steric hindrance of the organic phosphine binding to the azidomethoxy group when $A_1$ is hydrogen. The binding frequency of the two is increased due to the decrease in steric hindrance.

As one possible embodiment of the method for reducing the azidomethoxy group in the present application, $A_2$ is selected from hydrogen, or $A_2$ contains at least one of a group capable of inhibiting a polymerization reaction of the compound I and a detectable group.

As one possible embodiment of the method for reducing the azidomethoxy group in the present application, $A_1$ is selected from hydrogen, and $A_2$ contains at least one of a group capable of inhibiting a polymerization reaction of the compound I and a detectable group. In some embodiments, $A_1$ is selected from hydrogen, and $A_2$ contains a group capable of inhibiting a polymerization reaction of the compound I and a detectable group.

As one possible embodiment of the method for reducing the azidomethoxy group in the present application, the compound I is selected from at least one of the following structural formulas 1-1, 1-2, and 1-3:

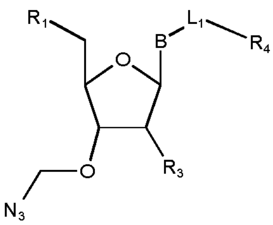

Formula 1-1

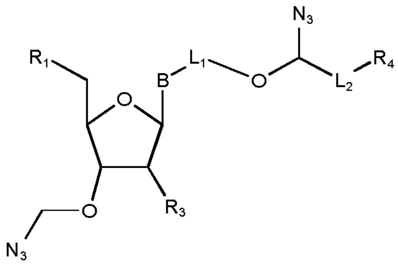

Formula 1-2

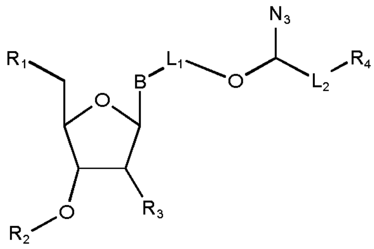

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ contains a detectable group.

In this embodiment, the chemical structures of three types of nucleotides are represented by structural formulas 1-1, 1-2, and 1-3. Among them, the 3'-position of the pentose sugar of the nucleotide of formula 1-1 is substituted with azidomethoxy (-O-$CH_2$-$N_3$). When the palladium compound and the organic phosphine are mixed with the compound I containing formula 1-1 for chemical reaction, the palladium compound and the organic phosphine act on the compound I of formula 1-1 synergistically to rapidly reduce -O-$CH_2$-$N_3$ in the compound I of formula 1-1 into -OH. The 3'-position of the pentose sugar of the nucleotide of formula 1-2 is substituted with azidomethoxy (-O-$CH_2$-$N_3$), and the base or the analog thereof is linked with a substituent containing azidomethoxy (-O-CH($N_3$)-$L_2$-$R_4$) at the same time. When the palladium compound and the organic phosphine are mixed with the compound I containing formula 1-2 for chemical reaction, the palladium compound and the organic phosphine act on the compound I of formula 1-2 synergistically to rapidly reduce -O-$CH_2$-$N_3$ and -O-CH($N_3$)-$L_2$-$R_4$ in the compound I of formula 1-2 into -OH, respectively. In the nucleotide of formula 1-3, the base or the analog thereof is linked with a substituent containing azidomethoxy (-O-CH($N_3$)-$L_2$-$R_4$). When the palladium compound and the organic phosphine are mixed with the compound I containing formula 1-3 for chemical reaction, the palladium compound and the organic phosphine act on the compound I of formula 1-3 synergistically to rapidly reduce -O-CH($N_3$)-$L_2$-$L_4$ in the compound I of formula 1-3 into -OH.

It should be understood that when two or more of structural formulas 1-1, 1-2, and 1-3 are contained in the reaction system at the same time, the palladium compound and the organic phosphine can act on -O-$CH_2$-$N_3$ and -O-CH($N_3$)-$L_2$-$R_4$ of the two or more types of compounds I, and reduce -O-$CH_2$-$N_3$ and -O-CH($N_3$)-$L_2$-$R_4$ into -OH, respectively.

Using the above reactivity of structural formulas 1-1, 1-2, and 1-3, the compound can be used as dNTP for gene sequencing. Specifically, in the formulas, $R_4$ contains a detectable group, and different types of dNTPs can be distinguished under suitable detection conditions by labeling different $R_4$ on the different dNTPs, for example, labeling fluorescent dyes with different colors and/or wavelengths on A nucleotide, T nucleotide, G nucleotide, and C nucleotide, respectively, so as to distinguish four types of nucleotides or analogs thereof under suitable detection conditions. At least one of structural formulas 1-1, 1-2, and 1-3 is added into a sequencing system as a reaction substrate, at least one of structural formulas 1-1, 1-2, and 1-3 extends on a nucleic acid strand through a polymerization reaction, and at the same

time, $-O-CH_2-N_3$ in formulas 1-1 and 1-2 and $-O-R_2$ or $-O-CH(N_3)-L_2-R_4$ in formula 1-3 are used as inhibiting groups for dNTP polymerization reaction, so that the polymerization reaction of the next dNTP at the site of the inhibiting group can be hindered, and the extension of the next nucleotide can be prevented, and therefore, in each cycle of sequencing, to each nucleic acid strand, only one dNTP can be introduced through the polymerization reaction. Further, after the type of the introduced dNTPs is identified, a palladium compound and an organic phosphine are added into the sequencing system, and the inhibiting group $-O-CH_2-N_3$ and/or $-O-CH(N_3)-L_2-R_4$ in the introduced dNTPs in the nucleic acid strand is reduced into hydroxy through a cleavage reaction, so that a polymerization reaction site is provided for the introduction of the next dNTP in the nucleic acid strand. Certainly, the sequencing further includes removing detectable groups on the introduced dNTPs, so as to decrease signal interference caused by signals generated by the detectable groups on the dNTPs when performing signal detection on the next cycle of sequencing, i.e., decrease sequencing background signal or background noise. In the gene sequencing, the palladium compound is added to improve the oxidation-reduction reaction rate between the organic phosphine and the dNTPs, accelerate the reaction of reducing $-O-CH_2-N_3$ and/or $-O-CH(N_3)-L_2-R_4$ into -OH, finally effectively improve the sequencing efficiency, and further be conducive to improving the whole sequencing. Particularly, when the sequencing frequency is increased in the sequencing (i.e., long sequencing is carried out), due to the increase in the reaction rate of each cycle of sequencing, the effect of multiple cycles of sequencing is superposed, the effect of the sequencing rate is improved more significantly, and the error rate caused by the sequencing is decreased under the set automatic sequencing conditions.

In one possible implementation of this embodiment, the palladium compound and the organic phosphine in the mixed system are in a molar ratio of 5:1 to 50:1. When the molar ratio of the two is within the range described above, the compound I, the palladium compound, and the organic phosphine are mixed for 20-30 s, and the reduction efficiency of reducing the azidomethoxy group in the compound I into hydroxy can be up to 95%-100%.

In some implementations, the palladium compound and the organic phosphine in the mixed system are in a molar ratio of 5:1 to 20:1. When the molar ratio of the two is within the range described above, the compound I, the palladium compound, and the organic phosphine are mixed for 20-30 s, and the reduction efficiency of the azidomethoxy group in the compound I can be up to 98%-100%.

In one possible implementation of this embodiment, the chemical reaction of reducing the azidomethoxy group of the compound I into hydroxy is carried out at a pH value of 8.0-10.0. The pH condition is conducive to improving the reduction of $-O-CH_2-N_3$ and/or $-O-CH(N_3)-L_2-R_4$ by the palladium compound and the organic phosphine. Moreover, the pH condition is conducive to the sequencing. Specifically, when the pH of the reaction is greater than 10, a higher pH may hinder the binding of hydrogen bonds during base complementary, which is not conducive to the complementary pairing between dNTPs and a nucleic acid template during the sequencing; when the pH is less than 8, the polymerization reactivity of the dNTPs is decreased, thereby decreasing the sequencing efficiency. When the pH is further decreased, the stability of the nucleic acid strand is decreased and even curling occurs, which is not conducive to the sequencing. In some implementations, the chemical reaction of reducing the azidomethoxy group of the compound I into hydroxy is carried out at a pH value of 9.0-9.5.

As one possible embodiment of the method for reducing the azidomethoxy group in the present application, the chemical reaction is carried out at a temperature of 40-75 °C to reduce the azidomethoxy group of the compound I into hydroxy. When at least one of structural formulas 1-1, 1-2, and 1-3 is used as dNTP for sequencing, the temperature can be used for the sequencing on the premise of not affecting the structural stability of the nucleic acid strand, and effectively improve the reduction efficiency of $-O-CH_2-N_3$ and $-O-CH(N_3)-L_2-R_4$ by the palladium compound and the organic phosphine. In some implementations, the chemical reaction is carried out at a temperature of 45-70 °C to reduce the azidomethoxy group of the compound I into hydroxy.

As one possible embodiment of the method for reducing the azidomethoxy group in the present application, the mixed system comprises a buffer and metal ions selected from at least one of sodium ions and potassium ions. The metal ions and the buffer provide a stable chemical reaction environment, such as a pH condition, for the sequencing, and simultaneously provide a good reaction medium for the reduction of the azidomethoxy group, such as $-O-CH_2-N_3$ and $-O-CH(N_3)-L_2-R_4$, thereby being conducive to charge transfer in the reduction reaction. In some implementations, the metal ions in the mixed system have a concentration of 1.0-2.5 mol/L. High-concentration metal ions are conducive to improving the acceleration of the palladium compound to the reduction process of the azidomethoxy group, such as $-O-CH_2-N_3$ and $-O-CH(N_3)-L_2-R_4$, by the organic phosphine. In some implementations, the metal ions have a concentration of 1.2-2.0 mol/L.

In one embodiment, the mixed system comprises at least sodium chloride at a concentration of 1.0-2.0 mol/L. Moreover, the mixed system may further comprise at least one of other metal salts such as potassium chloride, sodium carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

As one possible embodiment of the method for reducing the azidomethoxy group in the present application, the buffer has a concentration of 50-180 mmol/L. When the concentration of the buffer is within the range described above, the

reduction reaction of $-O-CH_2-N_3$ and $-O-CH(N_3)-L_2-R_4$ by the palladium compound and the organic phosphine is in a stable pH environment, thereby being conducive to the improvement of the uniformity and stability of the reaction. In some implementations, the buffer has a concentration of 100-150 mmol/L.

In a second aspect, the present application provides a sequencing method including the following steps:

(a) under a condition suitable for a polymerization reaction, enabling a compound II to be incorporated into a nucleic acid template to obtain an extension product, wherein the compound II is a nucleotide analog containing an azidomethyl group and a detectable group, and the compound II has a structural formula of formula 1-1, formula 1-2, or formula 1-3:

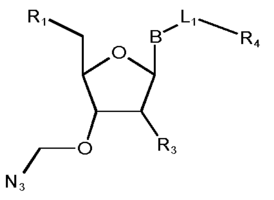

Formula 1-1

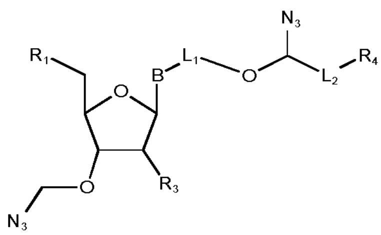

Formula 1-2

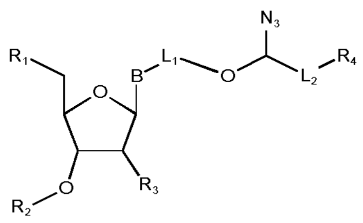

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains a detectable group;

(b) detecting the detectable group and acquiring a detectable signal generated by the detectable group; and

(c) adding a mixed solution comprising a palladium compound and an organic phosphine and placing the mixed solution under a condition suitable for a cleavage reaction to remove the azidomethyl group on the extension product or the azidomethyl group and the detectable group on the extension product.

The sequencing method provided by the present application includes the steps of mixing a palladium compound, an organic phosphine, and a compound II comprising at least one of structural formulas 1-1, 1-2, and 1-3, and carrying out a cleavage reaction on the compound II. In the steps, the palladium compound is used as a catalyst for accelerating an oxidation-reduction reaction between the organic phosphine and the compound, and more specifically, the palladium compound catalyzes the reaction of reducing the azidomethoxy group in the compound into hydroxy. By adopting the sequencing method provided by the present application, the reaction efficiency of reducing the azidomethoxy group in the compound II into hydroxy is improved, so that the sequencing can be accelerated, and the sequencing rate is improved. Particularly, when the sequencing includes a large number of sequencing cycles (for example, long sequencing is carried out), due to the increase in the reaction rate of the steps in each cycle of sequencing, the effect of multiple cycles of sequencing is superposed, and the effect of the sequencing rate is improved more significantly.

As one possible embodiment of the sequencing method in the present application, in step (c), the palladium compound and the organic phosphine are added in an amount satisfying: the palladium compound and the organic phosphine in a molar ratio of 2.5:1 to 100:1. In the sequencing method provided by the present application, compared with the case that no palladium compound is added as a catalyst, when the palladium compound and the organic phosphine are in a molar ratio of 2.5:1 to 100:1, the cleavage efficiency of the compound II, i.e., the reduction efficiency of $-O-CH_2-N_3$ and/or $-O-CH(N_3)-L_2-R_4$ in structural formulas 1-1, 1-2, and 1-3, can be significantly improved. Specifically, the contact time of the compound with a mixed solution of the palladium compound and the organic phosphine is 20-30 s, and the reduction efficiency of reducing $-O-CH_2-N_3$ and/or $-O-CH(N_3)-L_2-R_4$ in formulas 1-1, 1-2, and 1-3 into hydroxy can reach 90%-100%.

In one possible implementation of this embodiment, the palladium compound and the organic phosphine are in a molar ratio of 5:1 to 50:1. When the molar ratio of the two is within the range described above, the compound, the palladium compound, and the organic phosphine are mixed for 20-30 s, the reduction efficiency of $-O-CH_2-N_3$ and/or $-O-CH(N_3)-L_2-R_4$ in structural formulas 1-1, 1-2, and 1-3 is significantly improved, and the reduction efficiency can be up to 95%-100%.

In one possible implementation of this embodiment, the palladium compound and the organic phosphine are in a molar ratio of 5:1 to 20:1. In this case, the mixing time of the compound with the palladium compound and the organic phosphine is 20-30 s, and the reduction efficiency of reducing $-O-CH_2-N_3$ and/or $-O-CH(N_3)-L_2-R_4$ in formulas 1-1, 1-2, and 1-3 into hydroxy can be up to 98%-100%.

As one possible embodiment of the sequencing method in the present application, the palladium compound is

selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound.

As one possible embodiment of the sequencing method in the present application, the palladium compound is selected from at least one of a palladium halate, a palladium halide, an organic palladium carboxylate, and an allylpalladium halide dimer. The palladium compound described above can act together with the organic phosphine on formula 1-1, formula 1-2, or formula 1-3 and form a complex structure with formula 1-1, formula 1-2, or formula 1-3, so that the reduction process of $-O-CH_2-N_3$ and/or $-O-CH(N_3)-L_2-R_4$ by the organic phosphine is accelerated, and the cleavage efficiency in the compound is finally improved.

In one possible implementation of this embodiment, the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate. These divalent palladium compounds synergistically react with the organic phosphine, and contribute to accelerating the reduction of the azidomethoxy group ($-O-CH_2-N_3$ and/or $-O-CH(N_3)-L_2-R_4$) in formulas 1-1, 1-2, and 1-3.

As one possible embodiment of the sequencing method in the present application, the organic phosphine is selected from at least one of trivalent organic phosphines. After the palladium compound and the organic phosphine are mixed with at least one compound of structural formulas 1-1, 1-2, and 1-3, the azidomethoxy group ($-O-CH_2-N_3$ and/or $-O-CH(N_3)-L_2-R_4$) may form a complex system with the palladium compound, and bind to the trivalent organic phosphine at the same time to form a conjugate system, so that the formed conjugate-complex system may accelerate the rapid removal of one molecule of $N_2$ from the azidomethoxy group and further reduce the azidomethoxy group into a hydroxyl structure.

In one possible implementation of this embodiment, the organic phosphine is selected from at least one of tris(3-hydroxypropyl)phosphine, tris(2-carboxyethyl)phosphine, tris(hydroxymethyl)phosphine, triphenylphosphine, and tris(2-hydroxyethyl)phosphine. Compared with the case that no palladium compound is added as a catalyst, the reaction system using the organic phosphine and the palladium compound described above can improve the reaction efficiency of reducing $-O-CH_2-N_3$ and/or $-O-CH(N_3)-L_2-R_4$ in structural formulas 1-1, 1-2, and 1-3 into hydroxy.

As one possible embodiment of the sequencing method in the present application, the cleavage reaction is carried out at a pH value of 8.0-10.0. This is because the sequencing method includes a process of synthesizing a complementary strand based on the base complementary pairing principle using a nucleic acid strand as a template (accordingly, the nucleic acid strand is referred to as a nucleic acid template), and the pH environment in the sequencing may affect the stability of the nucleic acid strand during the sequencing, hinder the binding of hydrogen bonds during base complementary, and further affect the pairing performance of newly added nucleotides. The pH value of the cleavage reaction provided by the present application is 8.0-10.0, and in this case, the nucleic acid strand can keep good stability, thereby being conducive to forming hydrogen bonds by the nucleotides or nucleotide analogs represented by structural formulas 1-1, 1-2, and 1-3 and the corresponding nucleotides on the nucleic acid template, and achieving the base complementary pairing; furthermore, the chemical environment with the pH value of 8.0-10.0 can achieve the reduction of $-O-CH_2-N_3$ and/or $-O-CH(N_3)-L_2-R_4$ in structural formulas 1-1, 1-2, and 1-3 by the palladium compound and the organic phosphine, so that the sequencing efficiency is substantially improved.

In one possible implementation of this embodiment, the cleavage reaction is carried out at a pH value of 9.0-9.5. In one aspect, the pH condition can provide a better chemical environment for achieving the introduction of nucleotides or nucleotide analogs, and in another aspect, the pH condition is more favorable for the palladium compound and the organic phosphine to reduce $-O-CH_2-N_3$ and/or $-O-CH(N_3)-L_2-R_4$ in structural formulas 1-1, 1-2, and 1-3 into hydroxy, and improves the reaction efficiency.

As one possible embodiment of the sequencing method in the present application, the cleavage reaction is carried out at a temperature of 40-75 °C. Under the temperature condition, the nucleic acid strand has better stability, thereby being conducive to ensuring the stability and the accuracy of the sequencing; in addition, the temperature condition is conducive to improving the reduction efficiency of the organic phosphine for reducing $-O-CH_2-N_3$ and/or $-O-CH(N_3)-L_2-R_4$ catalyzed by the palladium compound. In one possible implementation of this embodiment, the cleavage reaction is carried out at a temperature of 45-70 °C.

As one possible embodiment of the sequencing method in the present application, the mixed solution further comprises metal ions, and the metal ions are selected from at least one of sodium ions and potassium ions. The sodium ions and the potassium ions provide a good salt environment for the sequencing, which is conducive to improving the stability of the nucleic acid strand, so that the stability and the accuracy of the sequencing are improved; meanwhile, the salt environment provides a good reaction medium for the reduction of the azidomethoxy group ($-O-CH_2-N_3$ and/or $-O-CH(N_3)-L_2-R_4$), and may be conducive to charge transfer in the oxidation-reduction reaction.

In one possible implementation of this embodiment, the metal ions are derived from at least one of halide salts, carbonates, acetates, phosphates, and borates of the metal ions. The salts described above for providing the metal ions

can provide metal ions, and can improve the reduction efficiency of the organic phosphine and the palladium compound on the azidomethoxy group (-O-$CH_2$-$N_3$ and/or -O-CH($N_3$)-$L_2$-$R_4$) by regulating the concentration of the metal ions; moreover, anions of these salts have reaction inertia in the reaction system, neither affecting the polymerization reaction between nucleotides in the sequencing nor affecting the reduction reaction of the azidomethoxy group in the nucleotide by adopting the organic phosphine and the palladium compound.

In some implementations, the mixed solution comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

As one possible embodiment of the sequencing method in the present application, the metal ions in the mixed solution have a concentration of 0.5-2.5 mol/L. When the concentration of the metal ions, i.e., sodium ions and/or potassium ions, is within the range described above, the reaction efficiency of the organic phosphine and the palladium compound for reducing -O-$CH_2$-$N_3$ and/or -O-CH($N_3$)-$L_2$-$R_4$ in structural formulas 1-1, 1-2, and 1-3 into hydroxy is improved; moreover, within this range, the higher the concentration of the metal ions, the stronger the reduction ability of the organic phosphine and the palladium compound to -O-$CH_2$-$N_3$ and/or -O-CH($N_3$)-$L_2$-$R_4$ in structural formulas 1-1, 1-2, and 1-3, and the higher the efficiency of the corresponding reduction reaction.

In one possible implementation of this embodiment, the metal ions have a concentration of 1.0-2.5 mol/L. High-concentration metal ions are conducive to improving the reduction efficiency of -O-$CH_2$-$N_3$ and/or -O-CH($N_3$)-$L_2$-$R_4$ in structural formulas 1-1, 1-2, and 1-3 by the palladium compound and the organic phosphine. In some implementations, the metal ions have a concentration of 1.2-2.0 mol/L.

In one embodiment, the mixed system comprises at least sodium chloride at a concentration of 1.0-2.0 mol/L. Moreover, the mixed system may further comprise at least one of other metal salts such as potassium chloride, sodium carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

As one possible embodiment of the sequencing method in the present application, the mixed solution further comprises a buffer. The buffer can stabilize the pH environment of the sequencing, and is conducive to improving the stability of the sequencing, particularly the stability of nucleotide polymerization reaction and the stability of binding of hydrogen bonds during base pairing; meanwhile, the pH environment is conducive to the stable reaction of reducing -O-$CH_2$-$N_3$ and/or -O-CH($N_3$)-$L_2$-$R_4$ in structural formulas 1-1, 1-2, and 1-3 into hydroxy.

In one possible implementation of this embodiment, the buffer is selected from at least one of a primary amine buffer and a tertiary amine buffer. Compared with the primary amine buffer, the tertiary amine buffer can provide a better and stable reaction environment for the reduction of the azidomethoxy group into hydroxy.

In some implementations, the buffer is selected from at least one of tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

As one possible embodiment of the sequencing method in the present application, the nucleic acid template binds to the surface of a solid carrier, and the mixed solution flows through the surface of the solid carrier. In the current sequencing method, immobilizing the nucleic acid template on the surface of the solid carrier is a common method for sequencing. A solid carrier on which a nucleic acid template is immobilized is also referred to as a biochip. In the sequencing, after a nucleic acid template is immobilized on the surface of a solid carrier, when nucleotides or nucleotide analogs of structural formulas 1-1, 1-2, and 1-3 are introduced on the surface of the solid carrier, bases in the nucleotides or the nucleotide analogs achieve base complementary pairing with the nucleic acid template through hydrogen bonds, and a polymerization reaction is carried out on a complementary strand of the nucleic acid template, so that the nucleotides or the nucleotide analogs of structural formulas 1-1, 1-2, and 1-3 are incorporated into the nucleic acid template. For each polymerization reaction, one nucleotide is added to the complementary strand of the nucleic acid template, i.e., one extension is achieved, and the corresponding cycle of reaction is referred to as one cycle of extension, thereby allowing the complementary strand to be extended sequentially. Since the nucleic acid template is immobilized and the nucleotides of structural formulas 1-1, 1-2, and 1-3 introduced in each cycle of sequencing bind to the nucleic acid template, the nucleotides of structural formulas 1-1, 1-2, and 1-3 introduced are also substantially equivalent to being immobilized on the surface of the solid carrier. The embodiment achieves the reduction of -O-$CH_2$-$N_3$ and/or -O-CH($N_3$)-$L_2$-$R_4$ in the nucleotides of structural formulas 1-1, 1-2, and 1-3 and improves the reduction efficiency on the basis of not changing the basic principle and the conventional operations of sequencing by synthesis by flowing a mixed solution comprising a palladium compound and an organic phosphine through the surface of the solid carrier. That is, the method provided herein for reducing -O-$CH_2$-$N_3$ and/or -O-CH($N_3$)-$L_2$-$R_4$ in the nucleotides of structural formulas 1-1, 1-2, and 1-3 by the palladium compound and the organic phosphine can be used for the current sequencing method for immobilizing the nucleic acid template on the surface of the solid carrier, and can improve the reduction efficiency of - O-$CH_2$-$N_3$ and/or -O-CH($N_3$)-$L_2$-$R_4$ in the nucleotides of structural formulas 1-1, 1-2, and 1-3.

In one possible implementation of this embodiment, the solid carrier comprises a channel structure capable of communicating one end of the solid carrier to the other end, the nucleic acid template is immobilized in the channel structure, and the mixed solution flows through the surface of the channel structure. In such implementation, by introducing the mixed solution into one end of the solid carrier, the mixed solution contacts and reacts with the nucleotides of structural formulas 1-1, 1-2, and 1-3 immobilized in the channel when the mixed solution flows through the channel structure, and $-O-CH_2-N_3$ and/or $-O-CH(N_3)-L_2-R_4$ is reduced into hydroxy. The method can meet the requirements of automatic sample introduction and continuous sequencing when the current sequencer carries out automatic sequencing.

In some implementations, the channel structure has a width of 5-15 mm and a height of 50-200 μm, the flow velocity of the mixed solution is 1000-2000 μL/s, and the volume of flow of the mixed solution is 50-500 μL. When the channel structure has a width of 5-15 mm and a height of 50-200 μm, the volume of flow of the mixed solution flowing through each site is 50-500 μL by regulating the flow velocity of the mixed solution to be 1000-2000 μL/s, so that the flowing time at the same site can be achieved within 20-30 s, $-O-CH_2-N_3$ and/or $-O-CH(N_3)-L_2-R_4$ in the nucleotides of structural formulas 1-1, 1-2, and 1-3 can be rapidly reduced, and the reduction efficiency is relatively high.

As one possible embodiment of the sequencing method in the present application, an oligonucleotide is immobilized on the surface of the solid carrier, the sequencing method further includes, before performing step (a): flowing a solution comprising the nucleic acid template through the surface of the solid carrier, so that a part of the sequence of the nucleic acid template complementarily binds to the oligonucleotide.

In one possible implementation of this embodiment, the sequencing method further includes: after step (c), cleaning the sequencing system after the cleavage reaction with a cleaning reagent. Through this step, the palladium compound and the organic phosphine in the sequencing system can be removed, so as to avoid the residue of the palladium compound and the organic phosphine. If the palladium compound remains in the sequencing system, the following effects may be brought: in the nucleotides of structural formulas 1-1 and 1-2 added in a new cycle of sequencing, under the action of the palladium compound and the organic phosphine, $-O-CH_2-N_3$ in a part of the nucleotides is reduced into hydroxy, so that a plurality of nucleotides are simultaneously introduced into a part of complementary strands in the new cycle of sequencing, resulting in prephasing (or prephase, which refers to the case that during sequencing, the nucleotides or analogs thereof introduced in the next cycle or next consecutive cycles of sequencing are subjected to a polymerization reaction in advance in the current cycle of sequencing and bind to the complementary strand of the nucleic acid template), and thus nucleic acid sequences of the introduced nucleotide regions cannot be accurately identified; or when a first imaging treatment for the introduced nucleotides is not available in the process of a new cycle of derivatization reaction, under the action of the palladium compound and the organic phosphine, $-O-CH(N_3)-L_2-R_4$ in a part of the nucleotides of structural formulas 1-2 and 1-3 has been reduced, so that signals generated by detectable groups cannot be acquired by the first imaging treatment, and thus the type of the introduced nucleotides in this cycle of sequencing cannot be identified.

In some implementations, the cleaning reagent includes a divalent palladium removal reagent, and the divalent palladium removal reagent comprises at least one of allyl, oxygen azidoalkyl, and disulfide bonds. The organic phosphine can be washed away with the cleaning reagent during the cleaning process with the cleaning reagent, but the palladium compound is easy to form a complex to be adsorbed or to remain, for example, to remain in the bases of a nucleotide, and therefore, it is necessary to carry out a cleaning treatment of the palladium compound with the use of a cleaning reagent capable of efficiently removing the palladium compound. Allyl, oxygen azidoalkyl, and disulfide bond groups can react with the palladium compound to form a complex, and residual divalent palladium in the reaction system can be effectively cleaned by removing the complex. Particularly, when the sequenced nucleic acid template is immobilized on the surface of the solid carrier, a cleaning reagent solvent is applied to the surface of the solid carrier, and allyl, oxygen azidoalkyl, and disulfide bond groups can react with divalent palladium to form a complex; further removing the cleaning reagent on the surface of the solid carrier can achieve the purpose of removing the residual palladium ions.

In some implementations, the divalent palladium removal reagent is selected from at least one of ethyl isocyanoacetate, cysteine or a salt thereof, glutathione, potassium isocyanoacetate, and cystamine. The removal reagent provided in the implementation comprises a reactive group capable of forming a complex structure with divalent palladium and capable of removing the palladium compound from the system through a complex reaction.

In some implementations, the divalent palladium removal reagent has a concentration of 1-40 mmol/L. When the concentration of the divalent palladium removal reagent is within this range, the divalent palladium can exert the effect of metal complexation.

In a third aspect, the present application provides a kit comprising a cleavage reagent.

The cleavage reagent is used for cleaving a compound III containing an azidomethoxy group so as to reduce the azidomethoxy group in the compound III into hydroxy, the azidomethoxy group has a chemical formula of $-O-A_1(N_3)-A_2$, and $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

The cleavage reagent comprises a palladium compound and an organic phosphine.

The cleavage reagent provided by the present application comprises a palladium compound and an organic phosphine, and under the catalytic action of the palladium compound, the efficiency of reducing the azidomethoxy group with the chemical formula of - $O-A_1(N_3)-A_2$ into hydroxy by the organic phosphine is improved, that is, when the cleavage reagent provided by the present application acts on the compound III containing the azidomethoxy group, the cleavage efficiency of the compound III can be improved.

As one possible embodiment of the cleavage reagent in the present application, the palladium compound and the organic phosphine are in a molar ratio of 2:1 to 100:1. When the palladium compound and the organic phosphine act on the compound III containing the azidomethoxy group in the molar ratio described above, it is conducive to carrying out a synergistic reaction between the palladium compound and the organic phosphine, and the reaction efficiency of reducing the azidomethoxy group into hydroxy is improved through the synergistic action of the two. Specifically, when the organic phosphine and the palladium compound are in a molar ratio of 2:1 to 100:1, the compound III containing the azidomethoxy group, like the compound I described above, contacts with a mixed solution of the palladium compound and the organic phosphine for 20-30 s, and the reduction efficiency of reducing the azidomethoxy group in the compound into hydroxy can reach 90%-100%.

As one possible embodiment of the cleavage reagent in the present application, the palladium compound is selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound.

As one possible embodiment of the cleavage reagent in the present application, the palladium compound is selected from at least one of a palladium halate, a palladium halide, an organic palladium carboxylate, and an allylpalladium halide dimer. The palladium compound described above can act together with the organic phosphine on the azidomethoxy group in the compound, and the three may form a complex structure with a conjugate system to accelerate the reduction process of the azidomethoxy group by the organic phosphine and finally improve the reduction efficiency of the azidomethoxy group in the compound.

In some embodiments, the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate. These divalent palladium compounds are used as a catalyst to accelerate an oxidation-reduction reaction between the organic phosphine and the azidomethoxy group.

As one possible embodiment of the cleavage reagent in the present application, the organic phosphine is selected from at least one of trivalent organic phosphines. The trivalent organic phosphine, the compound containing the azidomethoxy group, and the palladium compound may form a conjugate-complex system, and under the combined action of the palladium compound and the trivalent organic phosphine, the azidomethoxy group rapidly removes one molecule of $N_2$ and is reduced into a hydroxyl structure. In some embodiments, the organic phosphine is selected from at least one of tris(3-hydroxypropyl)phosphine, tris(2-carboxyethyl)phosphine, tris(hydroxymethyl)phosphine, triphenylphosphine, and tris(2-hydroxyethyl)phosphine. Compared with using the organic phosphine described above as a catalyst alone, using the reaction system of the organic phosphine and the palladium compound described above can improve the reaction efficiency of reducing the azidomethoxy group into hydroxy.

As one possible embodiment of the cleavage reagent in the present application, the cleavage reagent further comprises metal ions selected from at least one of sodium ions and potassium ions. Maintaining a suitable concentration of sodium ions and/or potassium ions in the reaction system provides a suitable chemical reaction environment for the reduction of the azidomethoxy group by the organic phosphine, which is conducive to the improvement of the cleavage efficiency.

In one possible implementation of this embodiment, the metal ions are derived from at least one of halide salts, carbonates, acetates, phosphates, and borates of the metal ions. The salts described above for providing the metal ions can provide sodium ions and potassium ions, and can improve the reduction efficiency of the organic phosphine and the palladium compound on the azidomethoxy group by regulating the concentration of metal ions; moreover, anions of these salts have reaction inertia in the reduction reaction system, which does not affect the reduction reaction of the azidomethoxy group in the compound by adopting the organic phosphine and the palladium compound.

In one possible implementation of this embodiment, the mixed system comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate. In one possible implementation of this embodiment, the metal ions in the cleavage reagent have a concentration of 0.5-2.5 mol/L. When the concentration of the metal ions, i.e., sodium ions and/or potassium ions, is within the range described above, the reaction efficiency of the organic phosphine and the palladium compound for reducing the azidomethoxy group into hydroxy is improved, within this range, the reaction efficiency of the

palladium compound for catalyzing the organic phosphine to reduce the azidomethoxy group into hydroxy is improved; moreover, within this range, the higher the concentration of the metal ions, the stronger the reduction ability of the organic phosphine to the azidomethoxy group, and the higher the efficiency of the corresponding reduction reaction.

As one possible embodiment of the cleavage reagent in the present application, the cleavage reagent further comprises a buffer. The buffer can provide a stable pH environment, is conducive to a stable reaction of reducing the azidomethoxy group into hydroxy, and finally improves the uniformity of the reduction reaction.

In one possible implementation of this embodiment, the buffer is selected from at least one of a primary amine buffer and a tertiary amine buffer. Compared with the primary amine buffer, the tertiary amine buffer can provide a better and stable reaction environment for the reduction of the azidomethoxy group into hydroxy.

In one possible implementation of this embodiment, the buffer is selected from at least one of tris(hydroxymethyl) aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

As one possible embodiment of the cleavage reagent in the present application, the compound III is selected from at least one of the following structural formulas 1-1, 1-2, and 1-3:

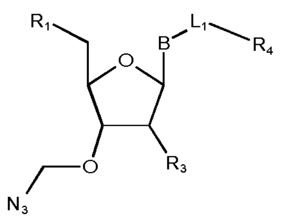

Formula 1-1

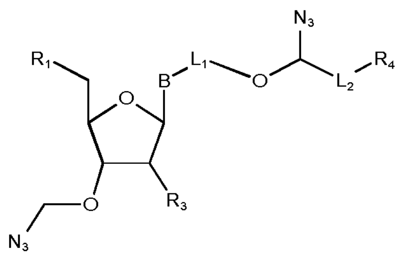

Formula 1-2

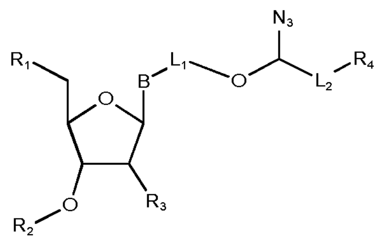

Formula 1-3 ,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains a detectable group.

In this embodiment, the chemical structures of three types of nucleotides are represented by structural formulas 1-1, 1-2, and 1-3. The cleavage reagent is used for reducing azidomethoxy groups in the chemical structures of the three types of nucleotides, that is: the cleavage reagent is used for reducing $-O-CH_2-N_3$ and/or $-O-CH(N_3)-L_2-R_4$ in structural formulas 1-1, 1-2, and 1-3 into -OH.

Among them, when the cleavage reagent contacts and reacts with the compound containing formula 1-1, the palladium compound and the organic phosphine act on the compound of formula 1-1 synergistically to reduce $-O-CH_2-N_3$ in the compound of formula 1-1 into -OH; when the cleavage reagent is mixed and reacts with the compound containing formula 1-2, the palladium compound and the organic phosphine act on the compound of formula 1-2 synergistically to reduce $-O-CH_2-N_3$ and/or $-O-CH(N_3)-L_2-R_4$ in the compound of formula 1-2 into -OH, respectively; when the cleavage reagent is mixed and reacts with the compound containing formula 1-3, the palladium compound and the organic phosphine act on the compound of formula 1-3 synergistically to reduce $-O-CH(N_3)-L_2-R_4$ in the compound of formula 1-3 into -OH.

It should be understood that when two or more of structural formulas 1-1, 1-2, and 1-3 are contained in the reaction system at the same time, the palladium compound and the organic phosphine in the cleavage reagent can act on $-O-CH_2-N_3$ and $-O-CH(N_3)-L_2-R_4$ of the two or more types of compounds, and reduce $-O-CH_2-N_3$ and $-O-CH(N_3)-L_2-R_4$ into -OH, respectively.

In one possible implementation of this embodiment, the palladium compound and the organic phosphine in the cleavage reagent are in a molar ratio of 5:1 to 50:1. When the molar ratio of the two is within the range described above, the palladium compound and the organic phosphine in the cleavage reagent react with the compound containing the azidomethoxy group with a structural general formula of $-O-CH(N_3)-A_1$ for 20-30 s, so that the reduction efficiency of reducing the azidomethoxy group in the compound into hydroxy is up to 95%-100%.

In some implementations, the palladium compound and the organic phosphine in the cleavage reagent are in a molar ratio of 5:1 to 20:1. When the molar ratio of the two is within the range described above, the palladium compound and the organic phosphine in the cleavage reagent react with the compound containing the azidomethoxy group with the structural general formula of $-O-CH(N_3)-A_1$ for 20-30 s, so that the reduction efficiency of reducing the azidomethoxy group in the compound into hydroxy is up to 98%-100%.

As one possible embodiment of the cleavage reagent in the present application, the cleavage reagent comprises a buffer and metal ions selected from at least one of sodium ions and potassium ions. The metal ions and the buffer provide

a good chemical reaction environment for sequencing, and simultaneously provide a good reaction medium for the reduction of the azidomethoxy groups, particularly $-O-CH_2-N_3$ and/or $-O-CH(N_3)-L_2-R_4$ in structural formulas 1-1, 1-2, and 1-3. In one possible implementation of this embodiment, the metal ions in the mixed system have a concentration of 1.0-2.5 mol/L. High-concentration metal ions are conducive to improving the reduction process of the organic phosphine on the azidomethoxy groups, particularly $-O-CH_2-N_3$ and $-O-CH(N_3)-L_2-R_4$ in structural formulas 1-1, 1-2, and 1-3, catalyzed by the palladium compound. In some implementations, the metal ions have a concentration of 1.2-2.0 mol/L.

In one embodiment, the mixed system comprises at least sodium chloride at a concentration of 1.0-2.0 mol/L. Moreover, the mixed system may further comprise at least one of other metal salts such as potassium chloride, sodium carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

In one possible implementation of this embodiment, the buffer has a concentration of 50-180 mmol/L. When the concentration of the buffer is within the range described above, the reduction reaction of the azidomethoxy groups, particularly $-O-CH_2-N_3$ and/or - $O-CH(N_3)-L_2-R_4$ in structural formulas 1-1, 1-2, and 1-3, by the palladium compound and the organic phosphine is in a stable pH environment, thereby being conducive to the improvement of the uniformity and stability of the reaction. Particularly, when the cleavage reagent is used for sequencing and flows through the surface of the compounds containing $-O-CH_2-N_3$ and $-O-CH(N_3)-L_2-R_4$ in structural formulas 1-1, 1-2, and 1-3, which are immobilized on the solid carrier, for reaction, the buffer system is conducive to improving the stability and uniformity of the reaction on the surface of the solid carrier. In some implementations, the buffer has a concentration of 100-150 mmol/L.

As one possible embodiment of the cleavage reagent in the present application, the kit further comprises a cleaning reagent, wherein the cleaning reagent includes a divalent palladium removal reagent, and the divalent palladium removal reagent comprises at least one of allyl, oxygen azidoalkyl, and disulfide bonds. Allyl, oxygen azidoalkyl, and disulfide bond groups can react with the palladium compound to form a complex, and residual divalent palladium in the reaction system can be effectively cleaned by removing the complex. Particularly, in the method provided in the first aspect or the third aspect, when the reduction reaction occurs on the surface of the solid carrier, a cleaning reagent solvent is applied to the surface of the solid carrier, and allyl, oxygen azidoalkyl, and disulfide bond groups can react with divalent palladium to form a complex; further removing the cleaning reagent on the surface of the solid carrier can achieve the purpose of removing the residual palladium ions.

In one possible implementation of this embodiment, the divalent palladium removal reagent is selected from at least one of ethyl isocyanoacetate, cysteine or a salt thereof, glutathione, potassium isocyanoacetate, and cystamine. The removal reagent provided in the implementation comprises a reactive group capable of forming a complex structure with divalent palladium and capable of removing the divalent palladium from the system through a complex reaction.

As one possible embodiment of the cleaning reagent in the present application, the divalent palladium removal reagent has a concentration of 1-40 mmol/L. When the concentration of the divalent palladium removal reagent is within this range, the divalent palladium can exert the effect of palladium ion complexation.

## BRIEF DESCRIPTION OF THE DRAWINGS

In order to more clearly illustrate the technical solutions in the embodiments of the present application, the drawings required for use in the description of the embodiments or the prior art will be briefly described below. It is obvious that the drawings in the description below are only some embodiments of the present application, and other drawings can be derived from these drawings by those of ordinary skill in the art without making creative efforts.

FIG. 1 shows an HPLC comparison provided in Example 1 and Comparative Example 1 of the present application,
FIG. 2 is a statistical graph showing the cleavage efficiencies in Examples 2-4 and Comparative Examples 2-4 provided in the present application;
FIG. 3 is a fluorescence image acquired by a fluorescence microscope in step c and step e provided in Example 41 of the present application;
FIG. 4 is a fluorescence image acquired by a fluorescence microscope in step c and step e provided in Example 47 of the present application;
FIG. 5 is a fluorescence image acquired by a fluorescence microscope in step c and step e provided in Comparative Example 6; and
FIG. 6 is a fluorescence image acquired by a fluorescence microscope in step c and step e provided in Comparative Example 7.

## DETAILED DESCRIPTION

In order to make the technical problems, technical solutions, and beneficial effects to be solved by the present

application more apparent, the present application will be further described in detail below with reference to the embodiments. It should be understood that the specific embodiments described herein are intended to explain the present application only rather than limit the present application.

In the present application, the terms used in the embodiments of the present application are for the purpose of describing particular embodiments only and are not intended to be limiting to the present application. The term "and/or" describes an associative relationship between associated objects, and means that there may be three relationships, for example, A and/or B may represent that: A is present alone, A and B are present simultaneously, and B is present alone. A and B can be singular or plural. The character "/" generally indicates a "and" relationship between the associated objects.

The term "at least one" means one or more, and "a plurality of" means two or more. "At least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, wherein a, b, and c may be a single item or a plurality of items, respectively.

As used in the embodiments and the appended claims of the present application, the singular forms "a", "an", and "the" are intended to include the plural forms as well, unless the context clearly indicates otherwise.

The terms "first" and "second" are used for description purposes only, are used for distinguishing purposes such as substance, orientation, interface, message, request, and terminal from one another, and should not be construed as indicating or implying relative importance or implicitly indicating the number of technical features indicated. For example, without departing from the scope of the embodiments in the present application, a first imaging treatment may also be referred to as a second imaging treatment, and similarly, a second imaging treatment may also be referred to as a first imaging treatment. Therefore, features defined with "first" and "second" may explicitly or implicitly include one or more of the features.

In the description of the present application, the concentration of the related components mentioned herein may not only refer to the specific content of each component, but also refer to the proportional relationship of the content among components. Therefore, as long as the content of the related components is scaled up or down according to the embodiments of the specification in the present application, it is within the scope disclosed in the embodiments of the specification in the present application.

It should be understood that in various embodiments of the present application, the sequence numbers of the processes described above do not imply an execution sequence, some or all of the steps may be executed in parallel or executed sequentially, and the execution sequence of each process should be determined by its function and inherent logic and should not constitute any limitation to the implementation process of the embodiments of the present application.

The abbreviations used herein have their conventional meanings in the chemical and biological fields. The chemical structures and chemical formulas herein are constructed according to standard rules of chemical valence known in the chemical field.

In the case that substituents are specifically indicated by their conventional formulas, the formula written from left to right likewise encompasses chemically identical substituents resulting from writing the structure from right to left, for example, $-O-CH(N_3)-$ is equivalent to $-(N_3)CH-O-$, and $-O-CA_1(N_3)-A_2$ is equivalent to $-O-CA_2(N_3)-A_1$.

Unless otherwise indicated, the term "hydrocarbyl", by itself or as part of a substituent, refers to a linear (i.e., unbranched) carbon chain, or a branched carbon chain (or carbon), or a combination thereof. It should be understood that "hydrocarbyl" is an acyclic chain. "Hydrocarbyl" may be a fully saturated group, or may be a monounsaturated group or a polyunsaturated group. When "hydrocarbyl" is a fully saturated group (i.e., the degree of unsaturation of hydrocarbyl is 0), "hydrocarbyl" is alkyl, i.e., a group formed by removing one hydrogen atom from an alkane molecule; when "hydrocarbyl" is a monounsaturated group (i.e., the degree of unsaturation of hydrocarbyl is 1), "hydrocarbyl" is an alkenyl containing one carbon-carbon double bond, i.e., one carbon-carbon single bond is substituted with one carbon-carbon double bond in alkyl; when "hydrocarbyl" is a polyunsaturated group (i.e., the degree of unsaturation of hydrocarbyl is greater than or equal to 2), "hydrocarbyl" is alkenyl or alkynyl, and the alkenyl contains two or more double bonds and/or contains one or more triple bonds in addition to two or more double bonds; the alkynyl may contain at least one triple bond and/or contain one or more double bonds in addition to at least one triple bond. "Alkenyl" may contain monovalent, divalent, and polyvalent groups having the indicated number of carbon atoms (e.g., C1-C100 represents 1-100 carbons).

The term "alkyl", by itself or as part of a substituent, refers to a linear (i.e., unbranched) carbon chain or a branched carbon chain (or carbon) free of unsaturated bonds, or a combination thereof, and it should be understood that "alkyl" is an acyclic chain; the term "alkenyl", by itself or as part of a substituent, refers to a linear (i.e., unbranched) carbon chain or a branched carbon chain (or carbon) containing a carbon-carbon double bond, or a combination thereof; the term "alkynyl", by itself or as part of a substituent, refers to a linear (i.e., unbranched) carbon chain or a branched carbon chain (or carbon) containing a carbon-carbon triple bond, or a combination thereof.

In the embodiments of the present application, "unsubstituted hydrocarbyl" refers to "hydrocarbyl" itself. "Unsubstituted hydrocarbyl" includes, but is not limited to, alkyl of homologs and isomers such as $-CH_3$, $-C_2H_5$, $-C_3H_7$, $-C_4H_9$, $-C_5H_{11}$, $-C_6H_{13}$, $- C_7H_{15}$, $-C_8H_{17}$, $-C_9H_{19}$, $-C_{10}H_{21}$, $-C_{15}H_{31}$, $-C_{20}H_{41}$, $-C_{25}H_{51}$, $-C_{30}H_{61}$, $-C_{35}H_{71}$, $-C_{40}H_{81}$, $-C_{45}H_{91}$, $-C_{50}H_{101}$, $-C_{55}H_{111}$, $-C_{60}H_{121}$, and the like, includes, but is not limited to, monounsaturated alkenyl

of homologs and isomers such as $-C_2H_3$, $-C_3H_5$, $-C_4H_7$, $-C_5H_9$, $-C_6H_{11}$, $-C_7H_{13}$, $-C_8H_{15}$, $-C_9H_{17}$, $-C_{10}H_{19}$, $-C_{15}H_{29}$, $-C_{20}H_{39}$, $-C_{25}H_{49}$, $-C_{30}H_{59}$, $-C_{35}H_{69}$, $-C_{40}H_{79}$, $-C_{45}H_{89}$, $-C_{50}H_{99}$, $-C_{55}H_{109}$, $-C_{60}H_{119}$, and the like, and includes, but is not limited to, polyunsaturated alkenyl or alkynyl of homologs and isomers such as $-C_2H$, $-C_3H_3$, $-C_4H_5$, $- C_4H_3$, $-C_5H_7$, $-C_5H_5$, $-C_5H_3$, $-C_6H_{11}$, $-C_6H_9$, $-C_6H_9$, $-C_6H_7$, $-C_7H_{13}$, $-C_7H_{11}$, $-C_7H_9$, $-C_7H_7$, $-C_7H_5$, $-C_7H_3$, $-C_8H_{15}$, $-C_8H_{13}$, $-C_8H_{11}$, $- C_8H_9$, $-C_8H_7$, $-C_8H_5$, $-C_9H_{17}$, $-C_9H_{15}$, $-C_9H_{13}$, $-C_9H_{11}$, $-C_9H_9$, $-C_9H_7$, $-C_{10}H_{17}$, $-C_{10}H_{15}$, $-C_{10}H_{13}$, $-C_{10}H_{11}$, $-C_{10}H_9$, $-C_{15}H_{27}$, $-C_{15}H_{25}$, $-C_{15}H_{23}$, $-C_{15}H_{21}$, $-C_{15}H_{19}$, $-C_{15}H_{17}$, $-C_{15}H_{15}$, $-C_{20}H_{37}$, $-C_{20}H_{35}$, $-C_{20}H_{33}$, $-C_{20}H_{31}$, $-C_{20}H_{29}$, $-C_{20}H_{27}$, $-C_{20}H_{25}$, $-C_{20}H_{23}$, $-C_{20}H_{21}$, $-C_{20}H_{19}$, $-C_{25}H_{47}$, $-C_{25}H_{45}$, $-C_{25}H_{43}$, $-C_{25}H_{41}$, $-C_{25}H_{39}$, $-C_{25}H_{37}$, $-C_{25}H_{35}$, $-C_{25}H_{33}$, $-C_{25}H_{31}$, $-C_{30}H_{57}$, $-C_{30}H_{55}$, $-C_{30}H_{53}$, $-C_{30}H_{51}$, $-C_{30}H_{49}$, $-C_{30}H_{47}$, $-C_{30}H_{45}$, $-C_{30}H_{43}$, $-C_{30}H_{41}$, $-C_{30}H_{39}$, $-C_{35}H_{67}$, $-C_{35}H_{65}$, $-C_{35}H_{63}$, $-C_{35}H_{61}$, $-C_{35}H_{59}$, $-C_{35}H_{57}$, $-C_{35}H_{55}$, $-C_{35}H_{53}$, $-C_{35}H_{51}$, $-C_{35}H_{49}$, $-C_{35}H_{47}$, $-C_{35}H_{45}$, $-C_{35}H_{43}$, $-C_{35}H_{41}$, $-C_{40}H_{77}$, $-C_{40}H_{75}$, $-C_{40}H_{73}$, $-C_{40}H_{71}$, $-C_{40}H_{69}$, $-C_{40}H_{67}$, $-C_{40}H_{65}$, $-C_{40}H_{63}$, $-C_{40}H_{61}$, $-C_{40}H_{59}$, $-C_{40}H_{57}$, $-C_{40}H_{55}$, $-C_{40}H_{53}$, $-C_{40}H_{51}$, $-C_{40}H_{49}$, $-C_{40}H_{47}$, $-C_{40}H_{45}$, $-C_{40}H_{43}$, $-C_{40}H_{41}$, $-C_{45}H_{89}$, $-C_{45}H_{87}$, $-C_{45}H_{85}$, $-C_{45}H_{83}$, $-C_{45}H_{81}$, $-C_{45}H_{79}$, $-C_{45}H_{77}$, $-C_{45}H_{75}$, $-C_{45}H_{73}$, $-C_{45}H_{71}$, $-C_{45}H_{69}$, $-C_{45}H_{67}$, $-C_{45}H_{65}$, $-C_{50}H_{97}$, $-C_{50}H_{95}$, $-C_{50}H_{93}$, $-C_{50}H_{91}$, $-C_{50}H_{89}$, $-C_{50}H_{87}$, $-C_{50}H_{85}$, $-C_{50}H_{83}$, $-C_{50}H_{81}$, $-C_{50}H_{79}$, $-C_{50}H_{77}$, $-C_{50}H_{75}$, and the like. Among them, the term "isomer" refers to compounds having the same number and type of atoms, and thus the same molecular weight, but differing in the structural arrangement or configuration of the atoms.

"Substituted hydrocarbyl" refers to a group formed by substituting one or more hydrogen atoms in the "hydrocarbyl" itself with a group other than "hydrocarbyl" itself. "Substituted hydrocarbyl" includes, but is not limited to, a group obtained by introducing one or more of the following atoms or substituents on the basis of unsubstituted hydrocarbyl: halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, heteroaryl, monophosphate group, diphosphate group, triphosphate group, and the like. Among them, the term "alkoxy" is alkyl linked to the rest of the molecule via an oxygen linker (-O-), and the alkyl moiety may contain a substitution or may be unsubstituted. The term "acyl" refers to - C(O)R, wherein R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl. Illustratively, the substituents for hydrocarbyl may be selected from, but not limited to, one or more of the following various groups: -OR', =O, =NR', =N-OR', -NR'R", -SR', -halogen, - SiR'R"R"', -OC(O)R', -C(O)R', $-CO_2R'$, -CONR'R", -OC(O)NR'R", -NR"C(O)R', -NR'-C(O)NR"R"', $-NR'C(O)_2R"$, -NR-C(NR'R"R"')=NR"", -NR-C(NR'R")=NR"', -S(O)R', $-S(O)_2R'$, $-S(O)_2NR'R"$, $-NRSO_2R'$, -NR'NR"R"', -ONR'R", - NR'C(O)NR"NR"'R"", -CN, $-NO_2$, $-NR'SO_2R"$, -NR'C(O)R", -NR'C(O)-OR", and -NR'OR", in amounts ranging from 0 to (2m'+1), where m' is the total number of carbon atoms in such group. R, R', R", R"', and R"" each independently represent hydrogen, substituted or unsubstituted heteroatom-containing hydrocarbyl, substituted or unsubstituted cyclic hydrocarbyl, substituted or unsubstituted heteroatom-containing cyclic hydrocarbyl, substituted or unsubstituted aryl (e.g., aryl substituted with 1-3 halogens), substituted or unsubstituted heteroaryl, substituted or unsubstituted hydrocarbyl, alkoxy or thioalkoxy, or arylalkyl.

Unless otherwise indicated, the term "heteroatom" is intended to include boron (B), oxygen (O), nitrogen (N), sulfur (S), phosphorus (P), and silicon (Si).

Unless otherwise indicated, the term "heteroatom-containing hydrocarbyl" refers to a stable linear or branched chain or a combination thereof containing at least one carbon atom and at least one heteroatom (e.g., B, O, N, P, Si, and S), wherein the nitrogen and sulfur atoms may optionally be oxidized and the nitrogen heteroatom may optionally be quaternized. The heteroatom (e.g., B, O, N, S, Si, or P) may be located at any internal position of heteroalkyl or at the position where alkyl is linked to the rest of the molecule. It should be understood that heteroalkyl is an acyclic chain. Examples of heteroalkyl include, but are not limited to: $-CH_2-CH_2-O-CH_3$, $-CH_2-CH_2-NH-CH_3$, $-CH_2-CH_2-N(CH_3)-CH_3$, $-CH_2-SCH_2-CH_3$, $-CH_2-S(O)-CH_3$, $-CH_2-CH_2-S(O)_2-CH_3$, $-CH=CH-O-CH_3$, $-CH_2-Si(CH_3)_3$, $-CH_2-O-Si(CH_3)_3$, $-CH_2-CH=NOCH_3$, $-CH=CH-N(CH_3)-CH_3$, $-CH_2-O-CH_3$, $-CH_2-O-CH_2-CH_3$, $-CH_2-CN$, $- CH_2-O-CH_2-N_3$, and the like. Up to two or three heteroatoms may be consecutive, e.g., $-CH_2-NH-OCH_3$ and $-CH_2-O-Si(CH_3)_3$. The "heteroatom-containing hydrocarbyl" moiety may contain one heteroatom (e.g., B, O, N, S, Si, or P), may also contain two optionally different heteroatoms (e.g., B, O, N, S, Si, or P), may also contain three optionally different heteroatoms (e.g., B, O, N, S, Si, or P), and even four, five, or more optionally different heteroatoms (e.g., B, O, N, S, Si, or P).

In the embodiments of the present application, when a group other than the heteroatom in the "heteroatom-containing hydrocarbyl" is alkyl, the "heteroatom-containing hydrocarbyl" is heteroalkyl. When a group other than the heteroatom in the "heteroatom-containing hydrocarbyl" contains a carbon-carbon double bond, the "heteroatom-containing hydrocarbyl" may be referred to as heteroalkenyl. The heteroalkenyl may optionally contain more than one double bond and/or contain one or more triple bonds in addition to one or more double bonds. When a group other than the heteroatom in the "heteroatom-containing hydrocarbyl" contains a carbon-carbon triple bond, the "heteroatom-containing hydrocarbyl" may be referred to as heteroalkynyl. The heteroalkynyl may optionally contain more than one triple bond and/or contain one or more double bonds in addition to one or more triple bonds.

Unless otherwise indicated, the terms "cyclic hydrocarbyl" and "heteroatom-containing cyclic hydrocarbyl", by themselves or in combination with other terms, mean a cyclic form of "hydrocarbyl" and "heteroatom-containing hydrocarbyl", respectively. It should be understood that "cyclic hydrocarbyl" and "heteroatom-containing cyclic hydrocarbyl" are not aromatic. In addition, with respect to the "heteroatom-containing cyclic hydrocarbyl", a heteroatom may occupy the position at which a heterocyclic ring is linked to the rest of the molecule. Examples of "cyclic hydrocarbyl" include, but are not limited to, cyclopropyl, cyclobutyl, cyclopentyl, cyclohexyl, 1-cyclohexenyl, 3-cyclohexenyl, cycloheptyl, and the like. Examples of "heteroatom-containing cyclic hydrocarbyl" include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, D-2-deoxyribose, D-ribose, and the like.

The terms "substituted cyclic hydrocarbyl" and "substituted heteroatom-containing cyclic hydrocarbyl" refer to a group formed by substituting one or more hydrogen atoms in the "cyclic hydrocarbyl" and "heteroatom-containing cyclic hydrocarbyl" themselves with a substituent, respectively. The substituent may substitute any hydrogen atom on the cyclic hydrocarbyl. Examples of "substituted cyclic hydrocarbyl" and "substituted heteroatom-containing cyclic hydrocarbyl" include, but are not limited to, a group obtained by introducing one or more of the following atoms or substituents on the basis of unsubstituted "cyclic hydrocarbyl" and "heteroatom-containing cyclic hydrocarbyl": halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, monophosphate group, diphosphate group, triphosphate group, and the like. Among them, the term "alkoxy" is alkyl linked to the rest of the molecule via an oxygen linker (-O-), and the alkyl moiety may contain a substitution or may be unsubstituted. The term "acyl" refers to -C(O)R, wherein R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

Unless otherwise indicated, the term "aryl" refers to a polyunsaturated aromatic hydrocarbon substituent, which may be a single ring or multiple rings (preferably 1-3 rings) fused together or linked covalently, i.e., a fused ring aryl. The fused ring aryl refers to multiple rings fused together, where at least one fused ring is an aryl ring.

Unless otherwise indicated, the term "heteroaryl" refers to aryl (or ring) containing at least one heteroatom (e.g., N, O, or S), wherein the nitrogen and sulfur atoms are optionally oxidized and the nitrogen atom is optionally quaternized. Thus, the term "heteroaryl" includes fused ring heteroaryl (i.e., multiple rings fused together, wherein at least one fused ring is a heteroaromatic ring). The heteroaryl may be linked to the rest of the molecule via a carbon atom or a heteroatom.

Non-limiting examples of aryl and heteroaryl include phenyl, naphthyl, pyrrolyl, pyrazolyl, pyridazinyl, triazinyl, pyrimidinyl, imidazolyl, pyrazinyl, purinyl, hypoxanthinyl, oxazolyl, isoxazolyl, thiazolyl, furyl, thienyl, pyridyl, pyrimidinyl, benzothiazolyl, benzoxazolyl, benzimidazolyl, benzofuryl, isobenzofuryl, indolyl, isoindolyl, benzothienyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidinyl, 4-pyrimidinyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. The substituents for each of the aryl and heteroaryl ring systems described above are selected from acceptable substituents described below. "Arylene" and "heteroarylene", alone or as part of another substituent, represent a divalent group derived from aryl and heteroaryl, respectively. A heteroaryl substituent may be bonded to a ring heteroatom nitrogen-O-.

The terms "substituted aryl" and "substituted heteroaryl" refer to a group formed by substituting one or more hydrogen atoms in the "aryl" and "heteroaryl" themselves with a substituent, respectively. Examples of "substituted aryl" and "substituted heteroaryl" include, but are not limited to, a group obtained by introducing one or more of the following atoms or substituents on the basis of unsubstituted "aryl" and "heteroaryl": halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, monophosphate group, diphosphate group, triphosphate group, and the like. Among them, the term "alkoxy" is alkyl linked to the rest of the molecule via an oxygen linker (-O-), and the alkyl moiety may contain a substitution or may be unsubstituted. The term "acyl" refers to -C(O)R, wherein R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

Illustratively, the substituents for aryl and heteroaryl are each independently selected from, but not limited to, one or more of the following various groups: -OR', -NR'R", -SR', -halogen, -SiR'R"R'", -OC(O)R', -C(O)R', $-CO_2R'$, -CONR'R", -OC(O)NR'R", - NR"C(O)R', -NR'-C(O)NR"R"', -NR"C(O)2R', -NR-C(NR'R"R‴)=NR⁗, -NR'-C(NR"R‴)=NR⁗, -S(O)R', $-S(O)_2R'$, $-S(O)_2NR'R"$, $-NRSO_2R'$, -NR'NR"R‴, -ONR'R", -NR'C(O)NR"NR‴R⁗, -CN, $-NO_2$, -R', $-N_3$, $-CH(Ph)_2$, (C1-C4) haloalkoxy and (C1-C4) haloalkyl, $-NR'SO_2R"$, -NR'C(O)R", -NR'C(O)-OR", and -NR'OR", in amounts ranging from 0 to the total number of open valences on the aromatic ring system. R, R', R", R"', and R⁗ each independently represent hydrogen, substituted or unsubstituted heteroatom-containing hydrocarbyl, substituted or unsubstituted cyclic hydrocarbyl, substituted or unsubstituted heteroatom-containing cyclic hydrocarbyl, substituted or unsubstituted aryl (e.g., aryl substituted with 1-3 halogens), substituted or unsubstituted heteroaryl, substituted or unsubstituted hydrocarbyl, alkoxy or thioalkoxy, or arylalkyl.

The symbol " 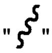 " represents the point of attachment to the rest of a molecule or chemical formula in the chemical structure.

The term "palladium compound" refers to a compound containing a palladium element in the structure; the term "Pd(0) compound" refers to a palladium-containing compound in which the valence of palladium is 0; the monovalent palladium compound refers to a palladium-containing compound in which the valence of palladium is +1 (i.e., $Pd^{+}$); similarly, the divalent palladium compound, the trivalent palladium compound, the tetravalent palladium compound, or the hexavalent palladium compound refers to a palladium-containing compound in which the valence of palladium is +2 (i.e., $Pd^{2+}$), +3 (i.e., $Pd^{3+}$), +4 (i.e., $Pd^{4+}$), or +6 (i.e., $Pd^{6+}$), respectively. The palladium compound may be an ionic compound, such as sodium tetrachloropalladate, palladium acetate, palladium dichloride, tetraamminepalladium dichloride, sodium tetranitropalladate, palladium dibromide, palladium diiodide, palladium trifluoroacetate, palladium pivalate, and the like, and may also be a nonionic compound, such as allylpalladium(II) chloride dimer (APC, with the chemical formula of ($\eta$3-$C_3H_5)_2Pd_2Cl_2$). The palladium compound may also be a colloid formed by a plurality of palladium complexes.

The term "organic phosphine" refers to an organic compound containing a carbon-phosphorus bond. The term "trivalent organic phosphine" refers to an organic phosphine in which the valence of phosphorus in the compound is +3. Illustratively, the trivalent organic phosphine includes, but is not limited to, tris(3-hydroxypropyl)phosphine, tris(2-carboxyethyl)phosphine, tris(hydroxymethyl)phosphine, triphenylphosphine, and tris(2-hydroxyethyl)phosphine.

In the embodiments of the present application, the term "sequencing" may also be referred to as "nucleic acid sequencing" or "gene sequencing", that is, the three expressions are interchangeable, and refers to the determination of the type and order of bases in a nucleic acid sequence, including sequencing by synthesis (SBS) and/or sequencing by ligation (SBL), including DNA sequencing and/or RNA sequencing, including long fragment sequencing and/or short fragment sequencing (the long fragment and short fragment are defined relatively; for example, nucleic acid molecules longer than 1 Kb, 2 Kb, 5 Kb, or 10 Kb may be referred to as long fragments, and nucleic acid molecules shorter than 1 Kb or 800 bp may be referred to as short fragments), and including double-end sequencing, single-end sequencing, paired-end sequencing, and/or the like (the double-end sequencing or paired-end sequencing may refer to the reading of any two segments or portions of the same nucleic acid molecule that are not completely overlapping); the sequencing involves the process of binding nucleotides (including nucleotide analogs) to the template and acquiring the corresponding signals emitted on the nucleotides (including analogs).

Sequencing generally involves multiple cycles of processes to achieve the determination of the order of multiple nucleotides/bases on the template, and each cycle of "a process to achieve the determination of the order of multiple nucleotides/bases on the template" may be referred to as "one cycle of sequencing" in the embodiments of the present application. "One cycle of sequencing", also referred to as "sequencing cycle", may be defined as completion of the base extensions of four types of nucleotides/bases once, and in other words, "one cycle of sequencing" may be defined as the determination of the base type at any given position on the template. For sequencing platforms that realize sequencing based on polymerization or ligation reactions, one cycle of sequencing comprises the process of binding four types of nucleotides (including nucleotide analogs) to the template at a time and acquiring the corresponding signals emitted; for platforms that realize sequencing based on polymerization reaction, a reaction system comprises reaction substrate nucleotide, polymerase and a template, a sequence fragment (a sequencing primer) is bound to the template, and on the basis of a base pairing principle and a polymerization reaction principle, the added reaction substrate nucleotide is connected to the sequencing primer under the catalysis of the polymerase to realize the binding of the nucleotide to a specific position of the template. Generally, one cycle of sequencing may comprise one or more base extensions (repeats). For example, four types of nucleotides are sequentially added to the reaction system to each perform base extension and corresponding acquisition of reaction signals, and one cycle of sequencing comprises four base extensions; for another example, four types of nucleotides are added into the reaction system in any combinations (such as in pairs or in one-three combinations), the two combinations each perform base extension and corresponding acquisition of reaction signals, and one cycle of sequencing comprises two base extensions; for yet another example, four types of nucleotides are added simultaneously to the reaction system for base extension and reaction signal acquisition, and one cycle of sequencing comprises one base extension.

The term "nucleic acid molecule" refers to a polymeric form of nucleotides of any length, and may include ribonucleotides, deoxyribonucleotides, analogs thereof, or mixtures thereof. The term may refer to a single-stranded or double-stranded polynucleotide. Nucleotides in a nucleic acid molecule may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a backbone containing a phosphodiester bond. Analog structures may have alternative backbone linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or

guanine. Any non-natural base or base analog may also be used for a nucleotide, such as a locked nucleic acid (LNA) and a bridged nucleic acid (BNA).

The term "primer", also known as "probe", refers to an oligonucleotide or a nucleic acid molecule that can hybridize to a target sequence of interest. In an embodiment, the primer functions as a substrate onto which nucleotides may be polymerized by a polymerase. For example, the primer may be used as a starting point for DNA or RNA synthesis. For example, a sequencing primer can hybridize to the synthesized nucleic acid template strand so as to trigger the synthesis of a new strand complementary to the synthesized nucleic acid template strand. The primer may include any combination of nucleotides or analogs thereof. In some examples, the primer is a single-stranded oligonucleotide or polynucleotide.

The term "target" or "template" nucleic acid molecule may refer to a sequence to be analyzed.

The term "ribosyl" refers to a group formed by removing one or more hydrogen atoms from ribose. The chemical formula of ribose is $C_5H_{10}O_5$, the ribose has two configurations of L-ribose and D-ribose, and the chemical structure of L-ribose is shown as follows:

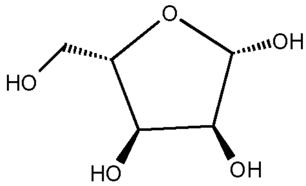

;

the chemical structure of D-ribose is shown as follows:

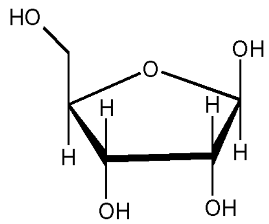

.

In the embodiments of the present application, the term "deoxyribosyl" refers to a group formed by removing one or more hydrogen atoms from deoxyribose. Deoxyribose is also referred to as D-deoxyribose, 2-deoxy-D-ribose, and thyminose. The chemical formula of deoxyribose is $C_4H_9O_3CHO$ ($C_5H_{10}O_4$), and the chemical structure is shown as follows:

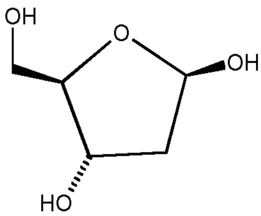

.

Unless otherwise indicated, the term "base," also referred to as nucleobase and nitrogenous base, includes natural bases, non-natural bases, and base analogs. Among them, the natural bases include adenine (A), guanine (G), cytosine (C), thymine (T), and uracil (U); the non-natural bases include, for example, locked nucleic acids (LNAs) and bridged nucleic acids (BNAs); the base analogs include, for example, hypoxanthine, deazaadenine, deazaguanine, deazahypoxanthine, 7-methylguanine, 5,6-dihydrouracil, 5-methylcytosine, and 5-hydroxymethylcytosine.

The term "phosphate group" refers to a phosphoryl group, i.e., a group formed by removing one or two hydrogen atoms from monophosphoric acid or polyphosphoric acid, and has the following chemical structure, wherein n is an integer selected from 1 to 3:

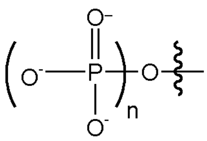

.

The term "analog" or "functional analog" refers to a chemical compound that is structurally similar to another compound (i.e., a so-called "reference" compound) but has a different composition, for example, one atom is replaced by an atom of a different element, or a particular functional group is present differently, or one functional group is replaced by another functional group.

In the embodiments of the present application, it should be understood that "XX or an analog thereof" includes "XX" and "XX analog". Illustratively, a nucleotide or an analog thereof includes a nucleotide and a nucleotide analog, a base or an analog thereof includes a base and a base analog, ribosyl or an analog thereof includes ribose and a ribose analog, and deoxyribosyl or an analog thereof includes deoxyribose and a deoxyribose analog.

In the embodiments of the present application, the term "hydroxy" and the chemical group "-OH" or "OH" have the same meaning and are interchangeable.

In the embodiments of the present application, the azidomethoxy group is a chemical group having a chemical formula of $-O-CA_1(N_3)-A_2$, wherein $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

The azidomethoxy group in the compound may be obtained by a chemical reaction of hydroxy in the compound. Moreover, the azidomethoxy group may be converted into hydroxy by reduction. Reversible conversion between the azidomethoxy group and the hydroxy provides a wider application scenario for the azidomethoxy group.

Illustratively, in one application scenario, since hydroxy has active chemical reactivity and can be converted into other atoms or groups through various chemical reactions such as oxidation, acylation, halogenation, and the like, when a compound containing hydroxy is subjected to a chemical reaction and the hydroxy is not expected to participate in the chemical reaction, the hydroxy is usually subjected to a blocking treatment before the chemical reaction, and is converted into other groups which have lower reactivity than the hydroxy but can be converted into the hydroxy through other ways, so as to avoid the effect of the chemical environment on the hydroxy, which is also called hydroxy protection. The azidomethoxy group ($-O-CH(N_3)-A_1$) can be used as a blocking group for hydroxy, and the hydroxy is converted into the azidomethoxy group to protect the hydroxy in a chemical reaction; after the chemical reaction, the azidomethoxy group can be reduced into hydroxy through a reduction reaction, and the activity of the hydroxy can be restored.

In another application scenario, due to the difference of reactivity between an azidomethoxy group ($-O-CH(N_3)-A_1$) and hydroxy, a group at a certain position in a compound structure can be regulated and controlled to be converted between the azidomethoxy group ($-O-CH(N_3)-A_1$) and the hydroxy, so that the position is used as a "switch" of the compound participating in a certain type of chemical reaction, thereby regulating and controlling the reactivity of the compound participating in the certain type of chemical reaction, and finally controlling the process of the expected reaction or the reaction flow. Specifically, when a group at a certain structure of the compound is an azidomethoxy group ($-O-CH(N_3)-A_1$), one or more types of chemical reactivity at the position is closed. In this case, the azidomethoxy group ($-O-CH(N_3)-A_1$) fails to undergo the chemical reaction under a condition suitable for hydroxy to undergo this type of chemical reaction. When a group at a certain structure of the compound is hydroxy, the corresponding chemical reactivity at the position is restored, and the hydroxy participates in and undergoes the chemical reaction under a condition suitable for the hydroxy to undergo this type of chemical reaction. Thus, by regulating and controlling the type of group at a certain structure of the compound, the reactivity at the position can be regulated and controlled.

For example, in the process of performing sequencing by synthesis on a nucleic acid molecule, when a nucleotide or an analog thereof (dNTP) with an azidomethoxy group ($-O-CH(N_3)-A_1$) at the 3'-position of a pentose sugar is used as a polymerization reaction raw material (also referred to as a reaction substrate) in each cycle of sequencing, the reactivity of 3'-OH of the nucleotide or the analog thereof (dNTP) is blocked, so that the activity of the nucleotide or the analog thereof for performing a polymerization reaction with a phosphate group of the next nucleotide or an analog thereof (dNTP) through 3'-OH is lost, and thus only one nucleotide or an analog thereof (dNTP) is introduced into a synthesized strand of each nucleic acid template in each cycle of sequencing. After the introduced nucleotide or the analog thereof (dNTP) is identified or determined, the azidomethoxy group ($-O-CH(N_3)-A_1$) is converted into hydroxy, and the activity of the nucleotide or the analog thereof (dNTP) for performing the polymerization reaction with the phosphate group of other nucleotide or the analog thereof at the 3'-OH position is restored, so that the extension between the nucleotides or the analogs thereof (dNTPs) is continued. Thus, the progress of sequencing can be controlled by the group conversion of the 3'-OH site of the nucleotide or the analog thereof (dNTP), so as to achieve the effect of synthesizing a nucleic acid strand while sequencing.

In still another application scenario, the azidomethoxy group ($-O-CH(N_3)-A_1$) may carry a target group having a certain function (such as luminescence property under the action of excitation light, a property of generating electrochemical signal difference before and after a reaction, and the like) through $A_1$, so that a compound containing the azidomethoxy group ($-O-CH(N_3)-A_1$) exerts the function of the target group in a specific case suitable for exerting the function, thereby achieving a specific effect. Then, the azidomethoxy group ($-O-CH(N_3)-A_1$) is reduced into hydroxy by an oxidation-reduction reaction to remove the target group in the compound, and the effect brought by the target group is

accordingly eliminated. For example, $A_1$ containing a fluorophore is introduced into a nucleotide or an analog thereof (dNTP) through an azidomethoxy group (-O-CH($N_3$)-$A_1$), and the fluorophore emits fluorescence under the excitation of a specific wavelength, so that a compound containing the azidomethoxy group (-O-CH($N_3$)-$A_1$) generates a recognizable fluorescence signal. After the fluorescence signal is acquired or utilized, the azidomethoxy group (-O-CH($N_3$)-$A_1$) in the compound is reduced into hydroxy, the fluorophore in $A_1$ is removed, and the fluorescence signal of the compound correspondingly disappears. The type of nucleotides or analogs thereof (dNTPs) participating in a certain type of reaction are identified or determined by labeling different types of nucleotides or analogs thereof (dNTPs) with different types of fluorophores (including different luminescent colors, different excitation wavelengths of excitation light, and the like), for example, the type of nucleotides or analogs thereof (dNTPs) introduced in one cycle of sequencing are identified or determined during nucleic acid sequencing, and after identification or determination, the corresponding fluorophores are removed and the next cycle of sequencing is continued.

The above are only several application scenarios of the azidomethoxy group, but are not limited thereto. In the application process of the azidomethoxy group, the reduction of the azidomethoxy group into hydroxy is a very important part. In the embodiments of the present application, the reaction of reducing the azidomethoxy group into hydroxy can be understood as a reaction of removing the azidomethoxy group (-CH($N_3$)-$A_1$) moiety in a compound from the compound, and thus the reduction of the azidomethoxy group in the embodiments of the present application may also be referred to as a cleavage reaction. At present, the azidomethoxy group can be reduced into hydroxy by using tris(3-hydroxypropyl) phosphine (THP or THPP), tris(2-carboxyethyl)phosphine (TCEP), tris(hydroxymethyl)phosphine (THMP) or tris(2-hydroxyethyl)phosphine (THEP), dithiothreitol (DTT), and the like as a cleavage reagent. These methods of reducing the azidomethoxy group into hydroxy have been widely used. In practical application, how to improve the reaction efficiency of reducing the azidomethoxy group into hydroxy has naturally become a focus of attention of researchers.

Palladium (Pd) is a platinum group element of group VIII in the fifth period, and its valence in an oxidation state includes +2, +3, and +4. Palladium metal and palladium-containing compounds are chemically inactive, but they can form complexes and are often used as catalysts in chemical reactions. A palladium complex refers to a compound formed by binding palladium metal or a compound containing palladium metal with a ligand, wherein the palladium metal or the compound containing palladium metal forms a coordination bond with one or more other compounds (also referred to as ligands) through an empty valence orbital in a palladium atom (a valence shell of an element, i.e., an orbital of the valence shell, has no electron, and the corresponding orbital is referred to as an empty valence orbital or an empty orbital). There are many chemical reaction methods catalyzed by palladium metal or palladium-containing compounds as catalysts, and the more common methods are as follows: Heck reaction (which refers to a coupling reaction of an unsaturated halogenated hydrocarbon and olefin under the catalysis of a strong base and palladium to produce substituted olefin), Stille reaction (a cross-coupling reaction of an organic tin compound and a halogenated hydrocarbon without β-hydrogen under the catalysis of palladium), Suzuki reaction (which refers to cross-coupling of aryl or alkenyl boric acid or borate with chloro-, bromo-, and iodoarene or olefin under the catalysis of a palladium complex), Hiyama coupling reaction (which refers to coupling of a halogenated hydrocarbon with organosilicon, that is, a cross-coupling reaction of palladium- or nickel-catalyzed aryl, alkenyl, and alkyl halide or a pseudohalogen (such as trifluoromethanesulfonate) with organosilane), Negishi coupling reaction (which is a reaction of coupling an organic zinc reagent and a halogenated hydrocarbon under the catalysis of a nickel or palladium complex to produce a new C-C bond), and the like.

In the embodiments of the present application, the technical research shows that a palladium compound can effectively accelerate the reaction of reducing an azidomethoxy group into hydroxy catalyzed by an organic phosphine. Hereinafter, the method and use of the palladium compound for effectively accelerating the reduction of the azidomethoxy group into hydroxy catalyzed by the organic phosphine will be described in detail in the embodiments of the present application.

The embodiment of the present application provides a method for reducing an azidomethoxy group, wherein the azidomethoxy group has a structural general formula of -O-C$A_1$($N_3$)-$A_2$, and $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

Specifically, the method includes: mixing a compound I containing the azidomethoxy group, a palladium compound, and an organic phosphine to give a mixed system, and placing the mixed system under a condition suitable for a chemical reaction to reduce the azidomethoxy group of the compound I into hydroxy, that is, reducing -O-CH($N_3$)-$A_1$ in the compound I into -OH after the mixing reaction.

In view of the reactivity of the palladium compound and the organic phosphine with the compound I during the cleavage reaction, in some embodiments, at least one of $A_1$ and $A_2$ is hydrogen, so that the steric hindrance of the palladium compound and the organic phosphine contacting the compound I can be decreased, and the direct contact probability of the reactants can be increased.

For example, when $A_2$ is hydrogen, the azidomethoxy group can be divided into two cases according to whether $A_1$ is hydrogen or not. In one case, $A_1$ is hydrogen, in which case the azidomethoxy group is azidomethoxy, corresponding

to the chemical formula of $-O-CH_2-N_3$. In another case, $A_1$ is a group other than hydrogen, i.e., $A_1$ is selected from one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl, in which case the azidomethoxy group is equivalent to a group in which one hydrogen atom on methylene $-CH_2-$ in azidomethoxy $-O-CH_2-N_3$ is substituted with $A_1$ that is not a hydrogen atom, i.e., the azidomethoxy group in this case has an azidomethoxy-like structure.

In the embodiments of the present application, the cleavage reaction refers to a process of reacting a compound I containing an azidomethoxy group with a palladium compound and an organic phosphine to cleave the compound I. In this embodiment, an oxidation-reduction reaction is performed between the compound I containing the azidomethoxy group and the organic phosphine, where the azidomethoxy group is reduced into hydroxy, and the organic phosphine is oxidized into other phosphine-containing compounds. The palladium compound is used as a catalyst to participate in the reaction during the chemical reaction, thereby promoting the oxidation-reduction reaction between the azidomethoxy group and the organic phosphine, and accelerating the reaction efficiency of reducing the azidomethoxy group into hydroxy. However, after the chemical reaction, the palladium compound returns to the structure before the chemical reaction, i.e., the chemical structure of the palladium compound is not changed before and after the chemical reaction.

In the embodiments of the present application, "mixing the compound I containing the azidomethoxy group, the palladium compound, and the organic phosphine" included in the mixing reaction may be achieved in various ways. In one embodiment, the palladium compound and the organic phosphine are mixed to form a homogeneous mixed solution, and then the mixed solution is mixed with the compound I containing the azidomethoxy group. In this embodiment, the compound I may be added to the mixed solution of the palladium compound and the organic phosphine; the mixed solution of the palladium compound and the organic phosphine may also be added to a reactor in which the compound I is present. In a second embodiment, the compound I containing the azidomethoxy group, the palladium compound, and the organic phosphine may be added separately to a reactor, the three substances undergoing molecular motion in the reactor and tending to mix. In this embodiment, the adding order of the three substances is not strictly limited. The compound I may be added first, then the palladium compound and the organic phosphine are added separately, and the adding order of the palladium compound and the organic phosphine is not strictly limited; the palladium compound may also be added first, then the compound I and the organic phosphine are added separately, and the adding order of the compound I and the organic phosphine is not strictly limited; the organic phosphine may also be added first, then the palladium compound and the organic phosphine are added separately, and the adding order of the palladium compound and the organic phosphine is not strictly limited. In the embodiments of the present application, the reactor may be a container capable of containing reaction raw materials such as the compound I containing the azidomethoxy group, the palladium compound, and the organic phosphine, and providing a place where the three react, illustratively, for example, a round-bottom flask. The reactor may also be other carriers capable of providing a place where the compound I, the palladium compound, and the organic phosphine react, such as a solid carrier and the like. It should be understood that when the reactor is a solid carrier, the contacting and/or reaction of the compound I containing the azidomethoxy group with the palladium compound and the organic phosphine may occur at the surface of the solid carrier.

In the mixed system according to the embodiments of the present application, the organic phosphine is used as a reducing agent to reduce the azidomethoxy group into hydroxy. The organic phosphine used for reducing the azidomethoxy group is selected from at least one of trivalent organic phosphines. In this case, the azidomethoxy group may form a complex system with the palladium compound, and bind to the trivalent organic phosphine at the same time to form a conjugate system, so that the formed conjugate-complex system may accelerate the rapid removal of one molecule of $N_2$ from the azidomethoxy group and further reduce the azidomethoxy group into a hydroxyl structure. In some embodiments, the organic phosphine is selected from at least one of tris(3-hydroxypropyl)phosphine, tris(2-carboxyethyl)phosphine, tris(hydroxymethyl)phosphine, triphenylphosphine, and tris(2-hydroxyethyl)phosphine. Compared with using the organic phosphine described above as a catalyst alone, using the reaction system of the organic phosphine and the palladium compound described above can improve the reaction efficiency of reducing the azidomethoxy group into hydroxy.

In the mixed system according to the embodiments of the present application, the palladium compound is used as a catalyst for accelerating an oxidation-reduction reaction between the organic phosphine and the compound I, especially between the organic phosphine and the azidomethoxy group. The palladium compound used as a catalyst in the embodiments of the present application may be one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound. The type of palladium compound is not strictly limited, and the palladium compound may be an organic palladium compound, such as an allylpalladium halide dimer and the like; the divalent palladium compound may also be an inorganic palladium compound, such as palladium dichloride and the like. The mechanism by which the palladium compound catalyzes the oxidation-reduction reaction in the embodiments of the present application is not clear. One possible mechanism is that the azidomethoxy group of the compound and the palladium compound are first subjected to a

complex reaction (high-valence palladium may first be reduced into low-valence palladium during the reaction and then participate in the complex reaction), and meanwhile, the electron cloud density on the azido group is changed to form a similar structure of -NH-N⊕≡N (a palladium atom in the palladium compound forms coordination bonds with an oxygen atom and N⊕≡N in the azidomethoxy group, respectively). -NH-N⊕≡N is conducive to accelerating the binding of the organic phosphine and the compound I, thereby improving the binding efficiency of the two. In addition, the divalent palladium improves the charge transfer efficiency during the reaction, and accelerates the breaking of the covalent bond between the oxygen atom in the azidomethoxy group and the rest of the azidomethoxy group.

In some embodiments of the present application, the compound is at least one of a palladium halate, a palladium halide, an organic palladium carboxylate, and an allylpalladium halide dimer. The palladium compound described above can accelerate the reduction process of the azidomethoxy group by the organic phosphine, and finally improve the reduction efficiency of the azidomethoxy group in the compound I. Illustratively, taking the divalent palladium compound as an example, it may be selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate. In some embodiments of the present application, when the compound I containing the azidomethoxy group is mixed with the palladium compound and the organic phosphine according to the molar ratio of the organic phosphine to the palladium compound of 2:1 to 2000:1, the organic phosphine and the palladium compound in the obtained mixed system are in a molar ratio of 2:1 to 2000:1 in this case. When the molar ratio of the two is within the range described above, the palladium compound can effectively improve the reaction efficiency of the organic phosphine for reducing the azidomethoxy group into hydroxy. Moreover, within this range, the reaction efficiency of reducing the azidomethoxy group in the compound I into hydroxy, i.e., the cleavage efficiency of the compound I, increases first and then decreases with the increase of the content of the palladium compound. When the content of the palladium compound is further added, and the molar ratio of the organic phosphine to the palladium compound is higher than 2:1, the effect of the palladium compound on the reduction of the azidomethoxy group by the organic phosphine is not significant compared to a reaction system without the palladium compound. When the content of the palladium compound is too low, and the molar ratio of the palladium compound to the organic phosphine is lower than 1:2000, the effect of the palladium compound on the reduction of the azidomethoxy group by the organic phosphine is also not significant compared to the reaction system without the palladium compound. In specific embodiments, the organic phosphine and the palladium compound may be in a molar ratio of 2:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1, 100:1, 200:1, 500:1, 800:1, 1000:1, 1200:1, 1500:1, 1800:1, 2000:1, and the like. In some embodiments, the mixed system reacts on the surface of the solid carrier, and the organic phosphine and the palladium compound are in a molar ratio of 2:1 to 100:1. At this time, the compound I, the palladium compound, and the organic phosphine are mixed for 20-30 s, and the reduction efficiency of reducing the azidomethoxy group in the compound I into hydroxy at different reaction temperatures is between 70% and 100%. In the embodiments of the present application, the chemistry reaction is carried out under an alkaline condition, i.e., under the alkaline condition, the palladium compound is used as a catalyst to accelerate the reaction of reducing the azidomethoxy group into hydroxy by the organic phosphine. In the embodiments of the present application, in the solution system in which the palladium compound, the organic phosphine, and the compound I are subjected to a chemical reaction under an alkaline condition, the pH of the solution is more than 7. The embodiments of the present application may be obtained by adjusting the pH of the solution system. In some embodiments, the pH value of the solution system may be decreased by adding, for example, a hydrogen-containing ion ($H^+$) or a Lewis acid capable of increasing acidity, so that the pH value of the solution system is in a suitable range; the pH value of the solution system may also be increased by adding a substance such as hydroxyl ions ($OH^-$) or other Lewis bases capable of increasing basicity, so that the pH value of the solution system is in a suitable range. A buffer is used as a reaction medium for the mixing reaction, and plays a role in stabilizing the pH value of the reaction system.

The pH environment of the mixed system is stabilized by the buffer, which is conducive to the stable reduction of the azidomethoxy group into hydroxy by the organic phosphine, and improves the uniformity of the oxidation-reduction reaction between the organic phosphine and the compound I.

Theoretically, the type of the buffer used in the embodiments of the present application is not strictly limited, as long as the reaction system comprising the palladium compound, the organic phosphine, and the compound I may be maintained under an alkaline condition. In some embodiments, the buffer is selected from at least one of a primary amine buffer and a tertiary amine buffer. The selection of such buffers is mainly considered that these buffers can have good solubility in the palladium compound, the organic phosphine, and the compound I, i.e., they can be used as good solvents for the reaction raw materials in the mixing reaction. Particularly, when the reaction raw material comprises a protein or a nucleic acid, the buffer can dissolve the protein or the nucleic acid well. Moreover, the buffer can provide a relatively stable pH environment. Particularly, when nucleic acid molecules are contained in the reactor in which the cleavage reaction occurs or in the chemical environment in which the cleavage reaction is present according to the embodiments of the present application, such buffers are advantageous for improving the structural stability of the nucleic acid molecules. Compared with the primary amine buffer, the tertiary amine buffer can provide a more stable reaction environment for the

reduction of the azidomethoxy group into hydroxy. Illustratively, the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and N,N-dihydroxyethylglycine. Among them, tris(hydroxymethyl) aminomethane buffer is also called tris(hydroxymethyl)aminomethane hydrochloride or Tris buffer, and is abbreviated as Tris-HCl; Hepes buffer is a non-ionic amphoteric buffer, whose major ingredient is 2-[4-(2-hydroxyethyl)-1-piperazinyl] ethanesulfonic acid.

It should be understood that the reaction system referred to in the embodiments of the present application comprises a palladium compound, an organic phosphine, and a compound I, as well as a solvent and even necessary additives; the reaction system may further comprise a place where the chemical reaction occurs, such as a reactor including but not limited to a container bottle with a certain accommodating space, a solid carrier capable of immobilizing a reaction substrate, and the like.

In the mixed system comprising a palladium compound, an organic phosphine, and a compound I, the buffer has a concentration of 50-200 mmol/L. The concentration of the buffer within the range described above is conducive to providing a stable pH environment for the reaction of reducing the azidomethoxy group into hydroxy. Particularly, when nucleic acid molecules are contained in the reactor or in the chemical environment where the reduction reaction is carried out, the pH condition provided by this concentration is conducive to maintaining the structural stability of the nucleic acid molecules. In some specific embodiments, the buffer may have a concentration of 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, 165 mmol/L, 170 mmol/L, 175 mmol/L, 180 mmol/L, 185 mmol/L, 190 mmol/L, 195 mmol/L, 200 mmol/L, and the like.

In some embodiments of the present application, the mixed system further comprises metal ions selected from at least one of sodium ions and potassium ions. It has been found by technical personnel that maintaining a suitable concentration of sodium ions and/or potassium ions in the reaction system can improve the reaction efficiency of reducing the azidomethoxy group into hydroxy by the organic phosphine. The metal ions in the embodiments of the present application are derived from at least one of halide salts, carbonates, acetates, phosphates, and borates of the sodium ion and the potassium ion. The salts described above can provide sodium ions and potassium ions through ionization, and can improve the reduction efficiency of the organic phosphine and the palladium compound on the azidomethoxy group by regulating the concentration of metal ions. Moreover, anions of these salts have reaction inertia in the reaction system, which does not affect the reduction reaction between the azidomethoxy group in the compound I and the organic phosphine catalyzed by the palladium compound. Illustratively, the mixed system comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

In some embodiments of the present application, the metal ions in the mixed system have a concentration of 0.1-2.5 mol/L. Illustratively, the metal ions in the mixed system have a concentration of 0.1 mol/L, 0.2 mol/L, 0.3 mol/L, 0.4 mol/L, 0.5 mol/L, 0.6 mol/L, 0.7 mol/L, 0.8 mol/L, 0.9 mol/L, 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, or 2.5 mol/L. In some specific embodiments, the metal ions, i.e., sodium ions and/or potassium ions, have a concentration of 0.5-2.5 mol/L. In this case, compared with a reaction system without metal ions or with fewer metal ions, the reaction efficiency of reducing the azidomethoxy group into hydroxy by the organic phosphine under the catalysis of the palladium compound is improved; moreover, within this range, the higher the concentration of the metal ions, the stronger the reduction ability of the organic phosphine and the palladium compound to the azidomethoxy group, and the higher the efficiency of the corresponding reduction reaction.

In the step of the mixing reaction in the embodiments of the present application, the too low reaction temperature is not only unfavorable for the palladium compound to exert the function as a catalyst, but also can reduce the reduction effect of the organic phosphine itself on the azidomethoxy group. However, when the reaction temperature is higher than a certain temperature value, the catalytic and synergistic effect of the palladium compound on the reduction of the azidomethoxy group by the organic phosphine tends to be gentle, and the high-temperature condition may cause reaction substrates to generate other miscellaneous side reactions, such as cleaving at other structures of the compound I. It should be understood that the temperature referred to in the embodiments of the present application refers to a temperature at which the organic phosphine and the compound I are subjected to an oxidation-reduction reaction under the catalysis of the palladium compound, i.e., a temperature at which the compound I is subjected to a cleavage reaction at the azidomethoxy group. The control of the cleavage reaction temperature can be achieved by controlling the reaction temperature of the mixed solution, or by regulating and controlling the temperature of the reactor such as a solid carrier, such that the temperature at which the organic phosphine and the compound I are subjected to the oxidation-reduction reaction under the catalysis of the palladium compound reaches the expected temperature for reaction.

The mixing reaction provided by the embodiments of the present application may be carried out at a temperature

of 30 °C to 100 °C. Illustratively, the mixing reaction may be carried out at a temperature of 30 °C, 35 °C, 40 °C, 45 °C, 50 °C, 55 °C, 60 °C, 65 °C, 70 °C, 75 °C, 80 °C, 85 °C, 90 °C, 95 °C, 100 °C, and the like.

In the embodiments described above, in the compound I containing the azidomethoxy group, structures other than the azidomethoxy group are not strictly required. If the structural general formula of the compound is represented by $A_3$-O-$CA_1(N_3)$-$A_2$, $A_1$ and/or $A_2$ are functional groups that exert a certain specific effect in the compound I, and $A_3$ represents the main structure of the compound I. Since $A_3$ does not change before and after the mixing reaction, there is no strict requirement for the selection of the chemical structure. For example, $A_3$ is selected from one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

As one implementation, in the compound I, $A_3$ is selected from C1-C100 groups. It should be understood that the C1-C100 groups as referred to herein include substituted or unsubstituted C1-C100 hydrocarbyl, substituted or unsubstituted C1-C100 cyclic hydrocarbyl, substituted or unsubstituted heteroatom-containing C1-C100 hydrocarbyl, substituted or unsubstituted heteroatom-containing C1-C100 cyclic hydrocarbyl, substituted or unsubstituted C1-C100 aryl, and substituted or unsubstituted C1-C100 heteroaryl. This is because the compound I in which the azidomethoxy group needs to be reduced is mostly concentrated in the compounds having 1 to 100 carbon atoms in $A_3$. Of course, it should be understood that when the compound I is a polymer or contains a bulky main structure $A_3$, and $A_3$ contains one or more types of bulky groups such as fluorophores, the number of carbon atoms in $A_3$ is not limited to 1 to 100.

In some embodiments, $A_3$ contains at least one of ribosyl or an analog thereof, deoxyribosyl or an analog thereof, a base or an analog thereof, a phosphate group, and a detectable group.

Since the difference in structure between ribose and deoxyribose is manifested in a substitution of one hydroxy on ribose with a hydrogen atom, in the embodiments of the present application, the structural general formula of ribosyl or an analog thereof and deoxyribosyl or an analog thereof can be represented as follows:

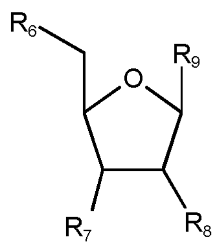

wherein $R_6$ is -OH or an optionally substituted group; $R_7$ is -OH or an optionally substituted group; $R_8$ is H, -OH, or an optionally substituted group; $R_9$ is -OH or an optionally substituted group. When $R_8$ is H, the corresponding structure is that of deoxyribose; when $R_8$ is -OH, the corresponding structure is that of ribose; when $R_8$ is an optionally substituted group, the corresponding structure may be a ribose analog or a deoxyribose analog.

Illustratively, the base or the analog thereof may be cytosine or an analog thereof, thymine or an analog thereof, adenine or an analog thereof, guanine or an analog thereof, hypoxanthine or an analog thereof, deazaadenine or an analog thereof, deazaguanine or an analog thereof, deazahypoxanthine or an analog thereof, 7-methylguanine or an analog thereof, 5,6-dihydrouracil or an analog thereof, 5-methylcytosine or an analog thereof, or 5-hydroxymethylcytosine or an analog thereof.

In the embodiments of the present application, the structure of the phosphate group may be represented as follows:

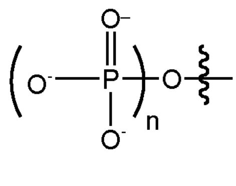

wherein n is an integer from 1 to 3. Illustratively, the phosphate group may be a monophosphate group (n = 1), a diphosphate group (n = 2), or a triphosphate group (n = 3).

In the embodiments of the present application, the detectable group refers to a group capable of generating a detectable signal under suitable conditions, such as a fluorophore capable of generating a detectable fluorescence signal under excitation of excitation light. Of course, the types of detectable groups are not limited thereto.

In one embodiment, the compound I is a nucleotide or an analog thereof, in which case $A_3$ contains not only a base or an analog thereof and a phosphate group, but also ribosyl or an analog thereof or deoxyribosyl or an analog

thereof.

Illustratively, $A_3$ includes at least one of the following structures:

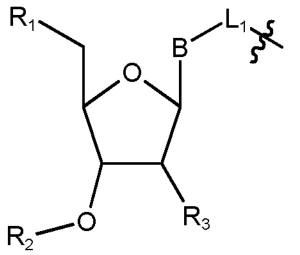

and

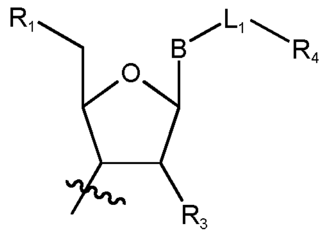

,

wherein B is a base or an analog thereof, $L_1$ is a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains a detectable group.

As one implementation, at least one of $A_1$ and $A_2$ in the compound I is hydrogen. As one implementation, in the compound I, $A_1$ is selected from hydrogen or C1-C50 groups. It should be understood that the C1-C50 groups as referred to herein include substituted or unsubstituted C1-C50 hydrocarbyl, substituted or unsubstituted C1-C50 cyclic hydrocarbyl, substituted or unsubstituted heteroatom-containing C1-C50 hydrocarbyl, substituted or unsubstituted heteroatom-containing C1-C50 cyclic hydrocarbyl, substituted or unsubstituted C1-C50 aryl, and substituted or unsubstituted C1-C50 heteroaryl. When the number of carbon atoms in $A_1$ is selected to be 1-50, it is beneficial for the azidomethoxy group to have a certain reaction space in the compound I. It should be understood that when the number of carbon atoms in $A_1$ is lower, correspondingly, the steric hindrance for reducing the azidomethoxy group into hydroxy is less, which is more conducive to the reaction.

In some embodiments, $A_2$ is selected from hydrogen, or $A_2$ contains at least one of a group capable of inhibiting a polymerization reaction of the compound I and a detectable group. In some embodiments, $A_2$ contains at least one of a group capable of inhibiting a polymerization reaction of the compound I and a detectable group. In the embodiments of the present application, the group capable of inhibiting the polymerization reaction of the compound I refers to a group capable of inhibiting a polymerase from exerting a catalytic effect at the position of the group of the compound I, and may also be a polymerase reaction inhibiting group. The polymerase reaction inhibiting group has no strict limitation on the structural type of the group. A group suitable for inhibiting the polymerase activity can be selected according to the chemical reactivity, and a group structure can also be set according to other structural levels, such as steric hindrance, that affect the chemical reaction, for example, a large steric hindrance is formed at the position to inhibit the polymerase reactivity. In some embodiments, $A_2$ contains a group capable of inhibiting a polymerization reaction of the compound I and a detectable group.

The method for reducing the azidomethoxy group in the compound I is described below with reference to a class of structures. Specifically, the compound I containing the azidomethoxy group is selected from at least one of the following structural formulas 1-1, 1-2, and 1-3:

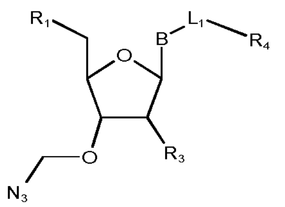

Formula 1-1

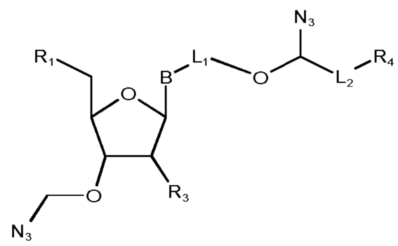

Formula 1-2

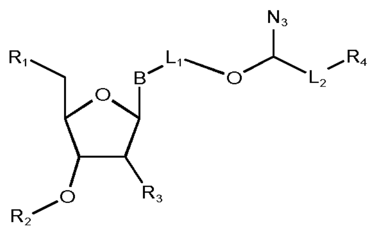

Formula 1-3

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains a detectable group.

In this case, the method for reducing the azidomethoxy group is as follows: mixing at least one of structural formulas 1-1, 1-2, and 1-3, a palladium compound, and an organic phosphine to give a mixed system, and placing the mixed system under a condition suitable for a chemical reaction to reduce the azidomethoxy group in structural formulas 1-1, 1-2, and 1-3 participating in the reaction into hydroxy.

In this embodiment, structural formulas 1-1, 1-2, and 1-3 correspond to chemical structures of three types of nucleotides, respectively. In the structure of structural formula 1-1, the azidomethoxy group is azidomethoxy, which is located at the 3' site of ribose or deoxyribose in the nucleotide structure of structural formula 1-1, and can be used for inhibiting the reactivity of the site to achieve a certain specific function, for example, to regulate and control a polymerization reaction process in the nucleotide polymerization reaction. Specifically, for example, the polymerization reaction of another nucleotide with the nucleotide at the 3' site is inhibited through azidomethoxy at the 3' site. After azidomethoxy at the site is reduced, azidomethoxy is changed back to hydroxy, and the activity of the nucleotide polymerization reaction at the site is restored, such that the next cycle of polymerization reaction can be further carried out. In the embodiments of the present application, the palladium compound, the organic phosphine, and the compound I containing formula 1-1 are mixed and reacted, and the organic phosphine and the compound I of formula 1-1 are subjected to an oxidation-reduction reaction under the catalysis of the palladium compound, so that $-O-CH_2-N_3$ in the compound I of formula 1-1 is rapidly reduced into -OH.

In the structure of structural formula 1-2, the 3' site of ribose or deoxyribose in the nucleotide structure is azidomethoxy, which is used for inhibiting the reactivity of the site to achieve a certain specific function, for example, only one nucleotide or nucleotide analog is introduced into a nucleic acid strand in each polymerization reaction through azidomethoxy at the 3' site, thereby achieving sequencing by sequentially reading nucleic acid base sequences. Specifically, for example, the polymerization reaction of the next nucleotide with the nucleotide at the 3' site is inhibited through azidomethoxy at the 3' site. After azidomethoxy at the site is reduced, azidomethoxy is changed back to hydroxy, and the activity of the nucleotide polymerization reaction at the site is restored, such that the next cycle of polymerization reaction can be further carried out. Moreover, in the nucleotide structure of structural formula 1-2, $-O-CH(N_3)-L_2-R_4$ linked to B also contains an azidomethoxy group. The azidomethoxy group at the site also has chemical cleavability (the group can be removed by a chemical reaction), and can be used for controlling the presence or absence of $R_4$ or a detectable group carried by $R_4$ through the reaction characteristics of the azidomethoxy group, thereby changing the detectable signal of the azidomethoxy group of the nucleotide before and after the oxidation-reduction reaction. For example, when $R_4$ contains a fluorophore, the fluorophore generates fluorescence under excitation of excitation light with a proper wavelength, and the fluorescence can be captured or acquired by an optical system; when the azidomethoxy group is removed by the cleavage reaction in the embodiments of the present application, the fluorophore is removed along with $-CH(N_3)-L_2-R_4$, and at this time, under the excitation of excitation light with a proper wavelength, no fluorescence signal is generated in $-O-CH(N_3)-L_2-R_4$ of the nucleotide any more, that is, the fluorescence signal of $-O-CH(N_3)-L_2-R_4$ of the nucleotide can no longer be captured or acquired by the optical system.

In the embodiments of the present application, after the palladium compound, the organic phosphine, and the compound I containing formula 1-2 are mixed and reacted, the organic phosphine and the compound I of formula 1-2 are subjected to an oxidation-reduction reaction under the catalysis of the palladium compound, so that $-O-CH_2-N_3$ and $-O-CH(N_3)-L_2-R_4$ in the compound I of formula 1-2 are rapidly reduced into -OH.

In the structure of structural formula 1-3, $-O-CH(N_3)-L_2-R_4$ linked to B contains an azidomethoxy group, which is similar to $-O-CH(N_3)-L_2-R_4$ in the compound I of formula 1-2. The azidomethoxy group at the site also has chemical cleavability (the group can be removed by a chemical reaction), and can be used for controlling the presence or absence of $R_4$ or a detectable group carried by $R_4$ through the reaction characteristics of the azidomethoxy group, thereby changing the detectable signal of the azidomethoxy group of the nucleotide before and after the oxidation-reduction reaction. For example, when $R_4$ contains a fluorophore, the fluorophore generates fluorescence under excitation of excitation light with a proper wavelength, and the fluorescence can be captured or acquired by an optical system; when the azidomethoxy group is removed by the reduction reaction of the present application, the fluorophore is removed along with $-CH(N_3)-L_2-R_4$, and at this time, under the excitation of excitation light with a proper wavelength, no fluorescence signal is generated in $-B-O-CH(N_3)-L_2-R_4$ of the nucleotide any more, that is, the fluorescence signal of $-O-CH(N_3)-L_2-R_4$ of the nucleotide can no longer be captured or acquired by the optical system.

In the embodiments of the present application, after the palladium compound, the organic phosphine, and the compound I containing formula 1-3 are mixed and reacted, the organic phosphine and the compound I of formula 1-3 are subjected to an oxidation-reduction reaction under the catalysis of the palladium compound, so that $-O-CH(N_3)-L_2-R_4$ in the compound I of formula 1-3 is rapidly reduced into -OH.

It should be understood that when two or more of structural formulas 1-1, 1-2, and 1-3 are contained in the reaction system at the same time, the palladium compound and the organic phosphine can theoretically act on $-O-CH_2-N_3$ and $-O-CH(N_3)-L_2-R_4$ of the two or more types of compounds I, and reduce $-O-CH_2-N_3$ and $-O-CH(N_3)-L_2-R_4$ into -OH, respectively. Of course, when the palladium compound and the organic phosphine are reacted with $-O-CH_2-N_3$ and $-O-CH(N_3)-L_2-R_4$, the reaction may be interfered by chemical reaction factors such as steric hindrance, resulting in different reaction efficiencies or reaction rates. When at least one of structural formulas 1-1, 1-2, and 1-3 provided by the embodiment is used as dNTPs for gene sequencing, $-O-CH_2-N_3$ and $-O-CH(N_3)-L_2-R_4$ can be used as inhibiting groups to prevent the introduction of the next dNTP on a nucleic acid strand, such that only one dNTP can be introduced into each nucleic acid molecule in each cycle of sequencing; after the type of the introduced dNTPs is identified or determined, the

inhibiting groups $-O-CH_2-N_3$ and $-O-CH(N_3)-L_2-R_4$ in the introduced dNTPs can be removed by adding a palladium compound and an organic phosphine into the sequencing system for mixing and reacting. Removing the inhibiting groups in the introduced dNTPs in this way can effectively improve the removal efficiency, which is further conducive to improving the sequencing rate. Particularly, when the number of sequencing cycles is increased, the effect of improving the sequencing rate is more significant.

In this embodiment, the organic phosphine and palladium compound are selected as described above and will not be described in detail herein.

In this embodiment, when at least one of structural formulas 1-1, 1-2, and 1-3 is mixed with a palladium compound and an organic phosphine, the palladium compound and the organic phosphine are added according to the molar ratio of the palladium compound to the organic phosphine of 5:1 to 20:1. Of course, the organic phosphine and the palladium compound in the resulting mixed system are in a molar ratio of 5:1 to 50:1. When the molar ratio of the two is within the range described above, the compound with the structure described above is mixed with the palladium compound and the organic phosphine for 20-30 s, and the reduction efficiency of reducing the azidomethoxy group in the compound I into hydroxy can be up to 95%-100% under suitable reaction conditions. Illustratively, the palladium compound and the organic phosphine in the mixed system may be in a molar ratio of 5:1, 8:1, 10: 1, 12:1, 15:1, 18:1, 20:1, 22:1, 25:1, 28:1, 30:1, 32:1, 35:1, 38:1, 40:1, 42:1, 45:1, 48:1, 50:1, and the like. In some embodiments, in order to further improve the reduction efficiency of reducing the azidomethoxy group into hydroxy, the molar ratio of the palladium compound to the organic phosphine in the mixed system can be adjusted to be 5:1 to 20:1. In this case, the compound I is mixed with the palladium compound and the organic phosphine for 20-30 s, and the reduction efficiency of the azidomethoxy group in the compound I can be up to 98%-100% under suitable reaction conditions. In the embodiments of the present application, the suitable reaction conditions include temperature, pH, and the like.

In this embodiment, when at least one of structural formulas 1-1, 1-2, and 1-3 is mixed with a palladium compound and an organic phosphine, the pH value of the reaction system is regulated and controlled to be 8.0-10.0. The pH condition is conducive to the palladium compound to catalyze an oxidation-reduction reaction between the organic phosphine and the compound, and to improving the reduction of $-O-CH_2-N_3$ and $-O-CH(N_3)-L_2-R_4$ by the palladium compound and the organic phosphine. That is, under the condition that the pH value is 8.0-10.0, the oxidation-reduction reaction rate between the organic phosphine and the compound is improved, and the cleavage efficiency of the compound at the azidomethoxy group is improved. When at least one of structural formulas 1-1, 1-2, and 1-3 is used as dNTPs for sequencing, the pH condition is conducive to the sequencing. Specifically, when the pH of the reaction is greater than 10, a higher pH may hinder the binding of hydrogen bonds during base complementary, which is thus not conducive to the complementary pairing between dNTPs and a nucleic acid template during the sequencing; when the pH is less than 8, the polymerization reactivity of the dNTPs is decreased, thereby decreasing the sequencing efficiency. When the pH is further decreased, the stability of the nucleic acid strand is decreased and even curling occurs, which is not conducive to the sequencing. Illustratively, the pH of the reaction system may be 8.0, 8.3, 8.5, 8.8, 9.0, 9.1, 9.2, 9.3, 9.4, 9.5, 9.8, 10.0, and the like. In some embodiments, at least one of structural formulas 1-1, 1-2, and 1-3 is used as dNTPs for sequencing, and in order to better improve the stability of a nucleic acid strand and provide a good reduction reaction environment for the azidomethoxy group, the reaction is carried out at a pH value of 9.0-9.5. It should be understood that the pH of the reaction system may be decreased by adding, for example, a hydrogen-containing ion ($H^+$, including but not limited to hydrochloric acid) or a Lewis acid capable of increasing acidity, so that the pH of the mixed solution in the reaction system is in a suitable range; the pH of the reaction system may also be increased by adding a substance such as hydroxyl ions ($OH^-$) or other Lewis bases capable of increasing basicity, so that the pH of the mixed solution in the reaction system is in a suitable range.

In this embodiment, the temperature of the mixing reaction is also a factor that affects the reaction rate of reducing the azidomethoxy group into hydroxy. However, it should be understood that although the temperature of the mixing reaction affects the reduction rate of the azidomethoxy group, the reduction rate of the azidomethoxy group in the reaction system comprising the palladium compound is still higher than that in the reaction system without the palladium compound under the same temperature condition.

In some embodiments, at least one of structural formulas 1-1, 1-2, and 1-3 is used as dNTPs for sequencing. At least one of structural formulas 1-1, 1-2, and 1-3, the palladium compound, and the organic phosphine are subjected to a chemical reaction at a temperature of 40-75 °C. Under this temperature condition, the structure of the nucleic acid strand is stable, and this temperature can also effectively improve the reduction efficiency of $-O-CH_2-N_3$ and $-O-CH(N_3)-L_2-R_4$ by the palladium compound and the organic phosphine. Illustratively, at least one of structural formulas 1-1, 1-2, and 1-3, the palladium compound, and the organic phosphine may be mixed and reacted at a temperature of 40 °C, 42 °C, 45 °C, 48 °C, 50 °C, 53 °C, 55 °C, 57 °C, 60 °C, 62 °C, 65 °C, 67 °C, 68 °C, 70 °C, or the like. In some embodiments, the mixing reaction may be carried out at a temperature of 45-70 °C.

In this embodiment, considering the nucleotide structures of structural formulas 1-1, 1-2, and 1-3, a buffer is used as a solvent system in the reaction system, and at the same time, metal ions containing at least one selected from sodium ions and potassium ions are added, that is, the mixed system comprises metal ions selected from at least one of sodium

ions and potassium ions and a buffer. This is because the buffer and the metal ions selected from at least one of sodium ions and potassium ions may serve as good reaction media and provide a good reaction environment with respect to a nucleotide structure, a nucleic acid molecule formed by a polymerization reaction of nucleotides, and a double-helix structure formed by base complementary pairing. In this embodiment, the type of buffer and metal ions is selected as described above and will not be described in detail herein in order to save space.

Under this condition, the metal ions selected from at least one of sodium ions and potassium ions and the buffer in the mixed system are analyzed, and it is found that:

(1) The pH environment provided when the concentration of the buffer in the mixed system is 50-180 mmol/L is favorable for the palladium compound to catalyze the reduction reaction of $-O-CH_2-N_3$ and $-O-CH(N_3)-L_2-R_4$ by the organic phosphine, and for improving the uniformity and stability of the reduction reaction. Illustratively, the buffer in the mixed system has a concentration of 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, 165 mmol/L, 170 mmol/L, 175 mmol/L, or 180 mmol/L. In some embodiments, the buffer has a concentration of 100-150 mmol/L.

(2) In the mixed system, when the concentration of the metal ions is 1.0-2.5 mol/L, it is beneficial to improving the reduction efficiency of the azidomethoxy group such as $-O-CH_2-N_3$ and $-O-CH(N_3)-L_2-R_4$ by the organic phosphine catalyzed by the palladium compound. Illustratively, the metal ions in the mixed system have a concentration of 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or the like. In some embodiments, the metal ions have a concentration of 1.2-2.0 mol/L.

In one embodiment, the mixed system comprises at least sodium chloride at a concentration of 1.0-2.0 mol/L. Moreover, the mixed system may further comprise at least one of other metal salts such as potassium chloride, sodium carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

In combination with the method for reducing the azidomethoxy group provided above, the present application provides a specific application method as an example.

Specifically, the embodiment of the present application further provides a sequencing method. This embodiment uses dNTPs (also referred to as "reversible terminators") with a reversible terminating group and a detectable group as substrates, and a nucleotide or an analog thereof incorporated into a complementary strand is sequenced under the action of a polymerase during synthesis of the complementary strand according to the base complementary pairing principle, to identify the type of the nucleotide or the nucleotide analog.

Taking one cycle of sequencing as an example, the sequencing method provided in this embodiment includes the following steps: S1, under a condition suitable for a polymerization reaction, a compound II is incorporated into a nucleic acid template to obtain an extension product.

In step S1, the compound II is a nucleotide analog containing an azidomethyl group and a detectable group, and the compound II has a structural formula of formula 1-1, formula 1-2, or formula 1-3:

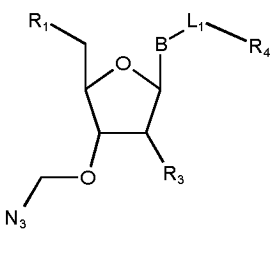

Formula 1-1

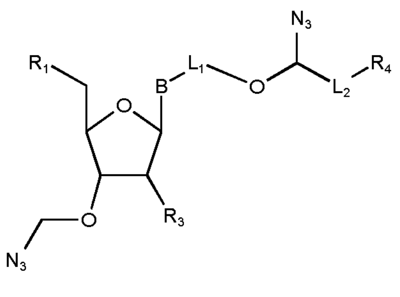

Formula 1-2

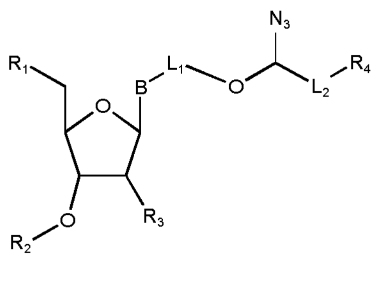

Formula 1-3

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains the detectable group.

In this step, $-CH_2-N_3$ in formula 1-1, $-CH_2-N_3$ and $-O-CH(N_3)-L_2-R_4$ in formula 1-2, and $-O-CH(N_3)-L_2-R_4$ in formula 1-3 serve as reversible terminating groups, and can inhibit the polymerization reactivity of next dNTPs or analogs

thereof, so that only one dNTP can be introduced into the nucleic acid strand in each polymerization reaction. Moreover, the reversible terminating groups can be reversibly removed by a chemical reaction such as a cleavage reaction or the like under suitable conditions. The reversible terminating groups in the embodiments of the present application have at least one of the following functions: (1) The reversible terminating group binds to 3'-hydroxyl site of ribose or deoxyribose as a blocking group to inhibit the polymerization reactivity of the site with other nucleotides or nucleotide analogs, such as $-CH_2-N_3$. The reversible terminating group can be removed by a chemical reaction under certain reaction conditions to restore the state of 3'-hydroxyl of ribose or deoxyribose. (2) The reversible terminating group binds to other sites of the nucleotide, such as a base or an analog thereof, and generates steric hindrance on the 3'-hydroxyl site of ribose or deoxyribose by forming a certain spatial conformation, thereby blocking the polymerization reactivity of the site with other nucleotides or nucleotide analogs, such as $-O-CH(N_3)-L_2-R_4$. Likewise, the reversible terminating group can be removed (reduced into hydroxy) by a chemical reaction under suitable conditions. Among them, the suitable conditions may be chemical reaction conditions capable of initiating the reduction of the reversible terminating group into hydroxy.

In formulas 1-1, 1-2, and 1-3 provided in this step, $R_4$ contains the detectable group. The detectable group refers to a group that is capable of generating a detectable signal under a certain condition, and the detectable signal may be, but is not limited to, an optical signal, an electrochemical signal, and the like, wherein the optical signal includes a fluorescence signal, and the electrochemical signal includes a current signal, a resistance signal, a voltage signal, and the like. Illustratively, in one example, the detectable group is a fluorescent dye, and in this case, a reversible terminator containing the fluorescent dye generates a detectable fluorescence signal under the action of excitation light; in other examples, the detectable group is a click chemistry reactive group, a targeting group, or the like.

Theoretically, the reversible terminator only needs to contain a reversible terminating group and a detectable group. The positions of the reversible terminating group and the detectable group (or the targeting group) in the reversible terminator and the types of the reversible terminating group and the detectable group (or the targeting group) may be selected variously, which are not strictly limited in the embodiments of the present application, and various reversible terminators capable of being used for sequencing at present may be selected.

In the structures of formulas 1-1, 1-2, and 1-3, $L_1$ and $L_2$ are each independently a covalent bond or a covalent linker group, and the selection thereof is not strictly limited.

In the embodiments of the present application, the reversible terminator is selected from at least one of the following: dATP or a dATP analog, dCTP or a dCTP analog, dGTP or a dGTP analog, and dTTP or a dTTP analog.

In the step of "a compound II is incorporated into a nucleic acid template", at least one of the structures of formulas 1-1, 1-2, and 1-3 described above may be added as a compound II. Illustratively, the compound II may contain one of four different types of nucleotides (dATP or an analog thereof, dTTP or an analog thereof, dGTP or an analog thereof, and dCTP or an analog thereof), may also contain two or three of four different types of nucleotides (dATP or an analog thereof, dTTP or an analog thereof, dGTP or an analog thereof, and dCTP or an analog thereof), and may further contain four different types of nucleotides (dATP or an analog thereof, dTTP or an analog thereof, dGTP or an analog thereof, and dCTP or an analog thereof) simultaneously. When the compound II contacted with the nucleic acid template contains two or three of four different types of nucleotides (dATP or an analog thereof, dTTP or an analog thereof, dGTP or an analog thereof, and dCTP or an analog thereof), the combination manner of the two or three types of nucleotides is not strictly limited, for example, when the compound II contacted with the nucleic acid template contains two of four different types of nucleotides (dATP or an analog thereof, dTTP or an analog thereof, dGTP or an analog thereof, and dCTP or an analog thereof), the two types of nucleotides may be a combination of dATP or an analog thereof and dTTP or an analog thereof, and may also be a combination of dATP or an analog thereof and dGTP or an analog thereof, a combination of dATP or an analog thereof and dCTP or an analog thereof, a combination of dTTP or an analog thereof and dGTP or an analog thereof, a combination of dTTP or an analog thereof and dCTP or an analog thereof, a combination of dGTP or an analog thereof and dCTP or an analog thereof, or the like. For another example, when the compound II contacted with the nucleic acid template contains three of four different types of nucleotides (dATP or an analog thereof, dTTP or an analog thereof, dGTP or an analog thereof, and dCTP or an analog thereof), the three types of nucleotides may be a combination of dATP or an analog thereof, dTTP or an analog thereof, and dGTP or an analog thereof, and may also be a combination of dATP or an analog thereof, dGTP or an analog thereof, and dCTP or an analog thereof, a combination of dATP or an analog thereof, dCTP or an analog thereof, and dTTP or an analog thereof, or the like. It should be understood that expressions, such as a combination of dGTP or an analog thereof and dCTP or an analog thereof, a combination of dATP or an analog thereof, dTTP or an analog thereof, and dGTP or an analog thereof, and the like, merely indicate that the combination includes these types of nucleotides at the same time and do not imply anything else. Illustratively, a combination of dGTP or an analog thereof and dCTP or an analog thereof means that the compound II contacted with the nucleic acid template contains dGTP or an analog thereof and dCTP or an analog thereof, and a combination of dATP or an analog thereof, dTTP or an analog thereof, and dGTP or an analog thereof means that the compound II contacted with the nucleic acid template contains dATP or an analog thereof, dTTP or an analog thereof, and dGTP or an analog thereof. When the compound II contacted with the nucleic acid template contains two or three of four different types of nucleotides (dATP or an analog thereof, dTTP or an analog thereof, dGTP or an analog thereof, and dCTP or an analog thereof), the multiple types of nucleotides may be

treated simultaneously or in multiple times, for example, compounds II corresponding to four different types of nucleotides (dATP or an analog thereof, dTTP or an analog thereof, dGTP or an analog thereof, and dCTP or an analog thereof) are added to the reaction system sequentially or simultaneously.

In the embodiments of the present application, the step of "a compound II is incorporated into a nucleic acid template" is carried out in the presence of a polymerase, which is used to initiate a polymerization reaction of the compound II with a terminal nucleotide or a nucleotide analog of a complementary strand of the nucleic acid template. The polymerase may be a DNA polymerase or an RNA polymerase. In the embodiments of the present application, the polymerase may be added during the mixing of a solution comprising the compound II and the polymerase with the nucleic acid template. Illustratively, a solution comprising the compound II and the polymerase are added to a reactor containing the nucleic acid template. Alternatively, the solution comprising the compound II and the polymerase are placed in different sample tubes of a kit, respectively, and each sample is extracted from the kit to the reactor where the nucleic acid template is placed, so that each component in the reaction system is contacted and tends to be mixed.

When the solution comprising the compound II and the polymerase is mixed with the nucleic acid template, the solution comprises a solvent used for dissolving the compound II and the polymerase, and the solvent may be a buffer such as tris(hydroxymethyl)aminomethane buffer, glycine, ethanolamine, tetraethylethylenediamine, tetramethylethylenediamine, *N*-butyldiethanolamine, diethylaminoethanol, Hepes buffer, and N,N-dihydroxyethylglycine.

In the embodiments of the present application, the step of "a compound II is incorporated into a nucleic acid template" is carried out in a reactor, which may be a reaction bottle with a certain accommodating space, or may be other carriers providing a reaction site. In some embodiments, the solution comprising the compound II and the polymerase and the nucleic acid template are placed in a reaction flask with a certain accommodating space, such as a triangular flask, a round-bottom flask, or the like, the substances are mixed, and the compound II is polymerized with a terminal nucleotide or a nucleotide analog of a complementary strand of the nucleic acid template under suitable conditions.

In some embodiments, the step of "a compound II is incorporated into a nucleic acid template" is carried out on the surface of a solid carrier. Specifically, a primer is immobilized on the surface of the solid carrier, and at least a part of the nucleotide template complementarily binds to the primer. Correspondingly, before carrying out step S1, the embodiment of the present application further includes: flowing a solution comprising the nucleic acid template through the surface of the solid carrier, so that a part of the sequence of the nucleic acid template complementarily binds to an oligonucleotide. At this time, a solution comprising the compound II flows through the surface of the solid carrier, and the compound II in the solution is complementarily paired with a base in the nucleic acid template, polymerized at the end of the primer away from the solid carrier, and immobilized on the surface of the solid carrier. In this implementation, the solid carrier may also be referred to as a biochip. In this implementation, the solution comprising the compound II may be transported to the surface of the solid carrier by a fluid path system to react with the nucleic acid template on the surface of the solid carrier, so that the integration and continuity of the sequencing can be achieved by the solid carrier and the fluid path system.

In one embodiment, the solid carrier comprises a channel structure capable of communicating one end of the solid carrier to the other end, in which case the solution comprising the compound II and the polymerase, the polymerase, and other solutions subsequently contacted with the solid carrier can be contacted by flowing through the surface of the channel structure. In the embodiment of the present application, the channel structure on the surface of the solid carrier may be formed on the surface of the solid carrier by, for example, etching. Materials are removed from a part of the surface of the solid carrier by, for example, etching, so that the part of the surface forms a groove structure. The groove structure leads from one end of the solid carrier to the other end of the solid carrier. The channel structure on the surface of the solid carrier can also be formed by covering a mask material layer on the surface of a solid substrate with a certain thickness, and the pattern of the mask material layer corresponds to the expected channel structure arranged on the surface of the solid carrier. For example, the mask material layer masks a region where the corresponding groove is located, a hollow treatment is performed on the other regions except the groove region, and then the material is deposited on the hollowed region of the mask material layer by using, for example, a chemical deposition method, so as to form a material layer with the channel structure on the surface of the solid substrate, and the solid substrate and the material layer formed on the surface of the solid substrate jointly form the solid carrier. It should be understood that the surface of the solid carrier may have one channel structure, or two or more, such as 4, 8, or 16, channel structures. Of course, the number of channel structures is not limited thereto. In some embodiments, the channel structure on the surface of the solid carrier is also referred to as Lane.

Illustratively, the channel structure has a width of 5-15 mm and a height of 50-200 μm. It should be understood that the width and height of the solid carrier may be arbitrarily combined within the ranges described above without strict limitations. Illustratively, when the width of the solid carrier is 5-10 mm, the height of the solid carrier may be in the range of 50-100 μm, 80-120 μm, 100-150 μm, 120-180 μm, 180-200 μm, or the like; of course, when the width of the solid carrier is 10-15 mm, the height of the solid carrier may be in the range of 50-100 μm, 80-120 μm, 100-150 μm, 120-180 μm, 180-200 μm, or the like.

S2, the detectable group is detected, and a detectable signal generated by the detectable group is acquired.

In this step, a signal generated by the detectable group in the reaction system after the polymerization reaction is

detected and acquired. The type of the compound II participating in the polymerization reaction is determined through the acquired signal. According to the type of the detectable group, the signal may be a fluorescence signal, an electrochemical signal, or the like. When the detectable signal is a fluorophore, the detectable signal corresponds to a fluorescence signal; when the detectable signal is a group that can undergo an electrochemical reaction, the detectable signal corresponds to an electrochemical signal, illustratively, a resistance signal.

The detectable groups may be different, and the acquired detectable signals generated by the detectable groups may also be different. In one embodiment, the detectable group is a fluorophore, in which case an imaging treatment is performed after the polymerization reaction to acquire a fluorescence signal generated by the nucleotide incorporated into the complementary strand of the nucleic acid template. When the detectable group is a group that can generate an electrochemical signal, signal acquisition is performed during the polymerization reaction.

S3, a mixed solution comprising a palladium compound and an organic phosphine is added and placed under a condition suitable for a cleavage reaction to remove the azidomethyl group on the extension product or the azidomethyl group and the detectable group on the extension product.

In this step, the mixed solution comprising the palladium compound and the organic phosphine is added to remove the azidomethyl group on the extension product or the azidomethoxy group on the extension product, including $-CH_2-N_3$ and $-O-CH(N_3)-L_2-R_4$ in the structures of formulas 1-1, 1-2, and 1-3. When the compound II is at least one of the structures of formulas 1-2 and 1-3, the mixed solution comprising the palladium compound and the organic phosphine is added, $R_4$ on the extension product can also be removed, and the detectable group on the extension product is removed, in which case the detectable signal generated by the detectable group is correspondingly eliminated.

According to the embodiments of the present application, the palladium compound is adopted to catalyze an oxidation-reduction reaction between the organic phosphine and the compound containing the azidomethoxy group, so that the reaction efficiency of reducing the azidomethoxy group into hydroxy may be accelerated, the sequencing may be accelerated, and the sequencing rate is improved. Particularly, as the number of sequencing cycles in the sequencing is increased, the improvement of the sequencing efficiency is more remarkable.

In the method, the step of "a mixed solution comprising a palladium compound and an organic phosphine is added" may be: mixing a mixed solution which is prepared in advance and comprises a palladium compound and an organic phosphine with a nucleic acid template, or may also be: adding a mixed solution comprising a palladium compound and an organic phosphine to or on the surface of a reactor containing a nucleic acid template or with a nucleic acid template immobilized thereon, and enabling the palladium compound and the organic phosphine to tend to be mixed in the reactor or on the surface of the reactor. For example, the palladium compound and the organic phosphine are added to the reactor or on the surface of the reactor, respectively, and the added palladium compound and the organic phosphine are gradually mixed through molecular diffusion, wherein the molecular diffusion may be completed under a standing condition or achieved through other methods, such as vibration, ultrasound, and the like. The palladium compound and the organic phosphine may even be contacted and mixed before entering the reactor or the surface of the reactor in the process of adding the palladium compound and the organic phosphine to the reactor.

In the embodiments of the present application, the organic phosphine is used as a reducing agent to reduce the azidomethoxy group into hydroxy. The organic phosphine used for reducing the azidomethoxy group is selected from at least one of trivalent organic phosphines. In this case, the azidomethoxy group forms a complex system with the palladium compound in one aspect, and in another aspect, the azidomethoxy group binds to the trivalent organic phosphine to form a conjugate system, and under the combined action of the palladium compound and the trivalent organic phosphine, the azidomethoxy group rapidly removes one molecule of $N_2$ and is further reduced into a hydroxyl structure. In some embodiments, the organic phosphine is selected from at least one of tris(3-hydroxypropyl)phosphine, tris(2-carboxyethyl) phosphine, tris(hydroxymethyl)phosphine, triphenylphosphine, and tris(2-hydroxyethyl)phosphine. Compared with using the organic phosphine described above as a catalyst alone, using the reaction system of the organic phosphine and the palladium compound described above can improve the reaction efficiency of reducing the azidomethoxy group into hydroxy.

In the embodiments of the present application, the palladium compound is used as a catalyst for accelerating an oxidation-reduction reaction between the organic phosphine and a compound containing at least one of structural formulas 1-1, 1-2, and 1-3, particularly between the organic phosphine and a azidomethoxy group in the compound, but the mechanism of the oxidation-reduction reaction catalyzed by the palladium compound is not clear. The palladium compound used as a catalyst in the embodiments of the present application may be one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound. The type of palladium compound is not strictly limited, and the palladium compound may be an organic palladium compound, such as an allylpalladium halide dimer and the like; the divalent palladium compound may also be an inorganic palladium compound, such as palladium chloride and the like.

In some embodiments, the palladium compound is at least one of a palladium halate, a palladium halide, an organic palladium carboxylate, and an allylpalladium halide dimer. The palladium compound described above can accelerate the reduction process of the azidomethoxy group by the organic phosphine, and finally improve the reduction efficiency of the azidomethoxy group in the compound II. Illustratively, taking the divalent palladium compound as an example, it may be selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate.

In some embodiments, when at least one of structural formulas 1-1, 1-2, and 1-3 is mixed with the palladium compound and the organic phosphine, the contents of the palladium compound and the organic phosphine in the mixed solution satisfy: the palladium compound and the organic phosphine in a molar ratio of 2.5:1 to 100:1. Compared with the case that the organic phosphine is adopted as a catalyst alone, when the palladium compound and the organic phosphine are in a molar ratio of 2.5:1 to 100:1, the reduction efficiency and the reduction rate of $-O-CH_2-N_3$ and/or $-O-CH(N_3)-L_2-R_4$ in structural formulas 1-1, 1-2, and 1-3 can be significantly improved. Specifically, the contact time of the compound with a mixed solution of the palladium compound and the organic phosphine is 20-30 s, and the reduction efficiency of reducing $-O-CH_2-N_3$ and/or $-O-CH(N_3)-L_2-R_4$ in formulas 1-1, 1-2, and 1-3 into hydroxy can reach 90%-100%. Illustratively, the palladium compound and the organic phosphine in the mixed solution may be in a molar ratio of 2.5:1, 3:1, 4:1, 5:1, 8:1, 10:1, 12:1, 15:1, 18:1, 20:1, 22:1, 25:1, 28:1, 30:1, 32:1, 35:1, 38:1, 40:1, 42:1, 45:1, 48:1, 50:1, 55:1, 60:1, 65:1, 70:1, 75:1, 80:1, 85:1, 90:1, 95:1, 100:1, or the like.

In some embodiments, the palladium compound and the organic phosphine are in a molar ratio of 5:1 to 50:1. When the molar ratio of the two is within the range described above, at least one of structural formulas 1-1, 1-2, and 1-3 is mixed with the palladium compound and the organic phosphine for 20-30 s, and the reduction efficiency of reducing the azidomethoxy group in $-O-CH_2-N_3$ and/or $-O-CH(N_3)-L_2-R_4$ in structural formulas 1-1, 1-2, and 1-3 into hydroxy can be up to 95%-100%.

In some embodiments, in order to further improve the reduction efficiency of reducing the azidomethoxy group into hydroxy, the molar ratio of the palladium compound to the organic phosphine in the mixed solution can be adjusted to be 5:1 to 20:1. In this case, the compound II is mixed with the palladium compound and the organic phosphine for 20-30 s, and the reduction efficiency of the azidomethoxy group in the compound II can be up to 98%-100%.

In this embodiment, the cleavage reaction is carried out at a pH value of 8.0-10.0. The pH condition is conducive to improving the reduction of $-O-CH_2-N_3$ and $-O-CH(N_3)-L_1-L_2(L_3)-L_4-R_5$ by the palladium compound and the organic phosphine, and is favorable for maintaining the stability of a nucleic acid molecular structure during sequencing, thereby maintaining the orderly progress of the sequencing. Illustratively, the pH of the reaction system may be 8.0, 8.3, 8.5, 8.8, 9.0, 9.1, 9.2, 9.3, 9.4, 9.5, 9.8, 10.0, and the like. In some embodiments, at least one of structural formulas 1-1, 1-2, and 1-3 is used as a compound II for sequencing, and in order to better improve the stability of the nucleic acid molecular structure and provide a good reduction reaction environment for the azidomethoxy group, the cleavage reaction is carried out at a pH value of 9.0-9.5.

In this embodiment, the temperature of the cleavage reaction is also a factor that affects the reaction rate of reducing the azidomethoxy group into hydroxy. However, it should be understood that although the temperature of the cleavage reaction affects the reduction rate of the azidomethoxy group, the reduction rate of the azidomethoxy group in the reaction system comprising the palladium compound is still higher than that in the reaction system without the palladium compound under the same temperature condition.

In some embodiments, the cleavage reaction is carried out at a temperature of 40-75 °C. Under this temperature condition, the structure of the nucleic acid strand is stable, and this temperature can also effectively improve the reduction efficiency of $-O-CH_2-N_3$ and $-O-CH(N_3)-L_2-R_4$ by the palladium compound and the organic phosphine. Illustratively, at least one of structural formulas 1-1, 1-2, and 1-3, the palladium compound, and the organic phosphine may be mixed and reacted at a temperature of 40 °C, 42 °C, 45 °C, 48 °C, 50 °C, 53 °C, 55 °C, 57 °C, 60 °C, 62 °C, 65 °C, 67 °C, 68 °C, 70 °C, or the like. In some embodiments, the cleavage reaction may be carried out at a temperature of 45-70 °C.

In this embodiment, considering the nucleotide structures of structural formulas 1-1, 1-2, and 1-3, a buffer is used as a solvent system in the reaction system, and at the same time, metal ions containing at least one selected from sodium ions and potassium ions are added, that is, the mixed system comprises metal ions selected from at least one of sodium ions and potassium ions and a buffer. This is because the buffer and the metal ions selected from at least one of sodium ions and potassium ions may serve as good reaction media and provide a good reaction environment with respect to a nucleotide structure, a nucleic acid strand structure formed by a polymerization reaction of nucleotides, and a double-helix structure formed by base complementary pairing. In this embodiment, the type of buffer and metal ions is selected as described above and will not be described in detail herein in order to save space.

In some embodiments, the buffer is selected from at least one of a primary amine buffer and a tertiary amine buffer. These buffers can serve as solvents for raw materials in the mixing reaction, and can provide a stable pH environment. Compared with the primary amine buffer, the tertiary amine buffer can provide a better and stable reaction environment for the reduction of the azidomethoxy group into hydroxy. Illustratively, the buffer is selected from at least one

of tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

In some embodiments, the buffer in the mixed solution has a concentration of 50-180 mmol/L. The pH environment provided when the concentration of the buffer is 50-180 mmol/L is favorable for the palladium compound to catalyze the reduction reaction of -O-$CH_2$-$N_3$ and -O-CH($N_3$)-$L_2$-$R_4$ by the organic phosphine, and for improving the uniformity and stability of the reduction reaction. Illustratively, the buffer in the mixed solution has a concentration of 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, 165 mmol/L, 170 mmol/L, 175 mmol/L, or 180 mmol/L. In some embodiments, the buffer has a concentration of 100-150 mmol/L.

In some embodiments of the present application, the metal ions selected from at least one of sodium ions and potassium ions in the mixed solution may improve the reaction efficiency of reducing the azidomethoxy group into hydroxy by the organic phosphine. The metal ions in the embodiments of the present application are derived from at least one of halide salts, carbonates, acetates, phosphates, and borates of the sodium ion and the potassium ion. The salts provided above can provide sodium ions and potassium ions through ionization, and can improve the reduction efficiency of the organic phosphine and the palladium compound on the azidomethoxy group by regulating the concentration of metal ions. Moreover, anions of these salts have reaction inertia in the reaction system, which does not affect the reduction reaction between the azidomethoxy group in the compound II and the organic phosphine catalyzed by the palladium compound. Illustratively, in some embodiments, the mixed solution comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

In some embodiments of the present application, when the concentration of the metal ions is 1.0-2.5 mol/L, it is beneficial to improving the reduction efficiency of the azidomethoxy group such as -O-$CH_2$-$N_3$ and -O-CH($N_3$)-$L_2$-$R_4$ by the organic phosphine catalyzed by the palladium compound. Illustratively, the metal ions in the mixed solution have a concentration of 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or the like. In some embodiments, the metal ions have a concentration of 1.2-2.0 mol/L.

In one embodiment, the mixed solution comprises at least sodium chloride at a concentration of 1.0-2.0 mol/L. Moreover, the mixed solution may further comprise at least one of other metal salts such as potassium chloride, sodium carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

Illustratively, the nucleic acid template binds to the surface of the solid carrier, and the mixed solution flows through the surface of the solid carrier. In the current sequencing method, immobilizing the nucleic acid template to be sequenced on the surface of the solid carrier is a common method for sequencing. A solid carrier on which a nucleic acid template to be sequenced is immobilized is also referred to as a biochip. In the sequencing, after nucleotides or nucleotide analogs of structural formulas 1-1, 1-2, and 1-3 are introduced, the nucleotides or the nucleotide analogs are subjected to a polymerization reaction to extend on a complementary strand, and complementary pairing with a nucleic acid template is achieved through hydrogen bonds. Through these reactions, the nucleotides of structural formulas 1-1, 1-2, and 1-3 introduced in each cycle of sequencing are also substantially immobilized on the surface of the solid carrier. The embodiment achieves the reduction of -O-$CH_2$-$N_3$ and -O-CH($N_3$)-$L_2$-$R_4$ in the nucleotides of structural formulas 1-1, 1-2, and 1-3 and improves the reduction efficiency on the basis of not changing the basic principle and the conventional operations of sequencing by synthesis by flowing a mixed solution comprising a palladium compound and an organic phosphine through the surface of the solid carrier. That is, the method provided herein for reducing -O-$CH_2$-$N_3$ and -O-CH($N_3$)-$L_2$-$R_4$ in the nucleotides of structural formulas 1-1, 1-2, and 1-3 by the palladium compound and the organic phosphine can be used for the current sequencing method for immobilizing the nucleic acid template to be sequenced on the surface of the solid carrier, and can improve the reduction efficiency of -O-$CH_2$-$N_3$ and -O-CH($N_3$)-$L_2$-$R_4$ in the nucleotides of structural formulas 1-1, 1-2, and 1-3. Moreover, the method can remove the compound II which does not participate in the reaction on the surface of the solid carrier in a fluid manner.

In one possible implementation of this embodiment, the solid carrier comprises a channel structure capable of communicating one end of the solid carrier to the other end, and the mixed solution flows through the surface of the channel structure. In this implementation, by introducing the mixed solution into one end of the solid carrier, the mixed solution contacts and reacts with the nucleotides of structural formulas 1-1, 1-2, and 1-3 immobilized on the surface of the solid carrier in the channel when the mixed solution flows through the channel structure, and -O-$CH_2$-$N_3$ and -O-CH($N_3$)-$L_2$-$R_4$ are reduced into hydroxy. The method can meet the requirements of automatic sample introduction and continuous sequencing when the current sequencer carries out automatic sequencing.

In some implementations, the channel structure has a width of 5-15 mm and a height of 50-200 $\mu$m, the flow

velocity of the mixed solution is 1000-2000 μL/s, and the volume of flow of the mixed solution is 50-500 μL. When the channel structure has a width of 5-15 mm and a height of 50-200 μm, the volume of flow of the mixed solution flowing through each site is 50-500 μL by regulating the flow velocity of the mixed solution to be 1000-2000 μL/s, so that $-O-CH_2-N_3$ and $-O-CH(N_3)-L_2-R_4$ in the nucleotides of structural formulas 1-1, 1-2, and 1-3 can be rapidly reduced within 20-30 s, and the reduction efficiency is relatively high. It should be understood that the dimensions of the channel structure (including the width and height of the channel structure, and the like) on the surface of the solid carrier may be adjusted within the ranges described above, and the flow velocity and volume of flow of the mixed solution may also be adjusted. Illustratively, when the channel structure has a width of 5-15 mm and a height of 50-200 μm, the flow velocity of the mixed solution may be 1000 μL/s, 1050 μL/s, 1100 μL/s, 1250 μL/s, 1200 μL/s, 1300 μL/s, 1350 μL/s, 1400 μL/s, 1450 μL/s, 1500 μL/s, 1550 μL/s, 1600 μL/s, 1650 μL/s, 1700 μL/s, 1850 μL/s, 1800 μL/s, 1950 μL/s, 2000 μL/s, and the like. In order to contact and react the mixed solution with the nucleotides of structural formulas 1-1, 1-2, and 1-3 in the channel structure, when the channel structure has a width of 5-15 mm and a height of 50-200 μm, the volume of flow of the mixed solution is 50-500 μL under the condition that the flow velocity of the mixed solution is 1000-2000 μL/s. Illustratively, the volume of flow of the mixed solution may be 50 μL, 100 μL, 150 μL, 200 μL, 250 μL, 300 μL, 350 μL, 400 μL, 450 μL, 500 μL, and the like.

After step S3, the embodiment of the present application further includes: S4, cleaning the sequencing system after the cleavage reaction with a cleaning reagent.

In this step, the reagents used in step S3 and the products generated after cleavage are removed by cleaning to avoid interference with the next cycle of sequencing.

After step S3, the sequencing system comprises palladium compounds and organic phosphines. Residues of these palladium compounds and organic phosphines may affect the next cycle of sequencing, for example, when the compound II added in the next cycle of sequencing contains structural formula 1-1, the azidomethoxy group at the 3' site of ribose or deoxyribose of the compound is reduced and cleaved under the catalysis of the palladium compound, so that a plurality of nucleotide structures may be continuously introduced in one cycle of sequencing, which further leads to the failure of nucleotide sequence analysis. When the compound II added in the next cycle of sequencing contains structural formula 1-3, the azidomethoxy group with a reversible terminating group or a detectable group is reduced and cleaved under the catalysis of the palladium compound. In one aspect, the removal of the reversible terminating group causes a plurality of nucleotide structures to be continuously introduced in the next cycle of sequencing, which further leads to the failure of nucleotide sequence analysis; in another aspect, the removal of the detectable group leads to the loss of the signal group carried by the compound II, and the type of the compound II cannot be determined by the signal of the introduced detectable group of the compound II. When the compound II added in the next cycle of sequencing contains structural formula 1-2, there are problems that the reversible terminator contains structural formula 1-1 and/or structural formula 1-3 at the same time. Therefore, a cleaning treatment is required to be carried out after step S3 to remove the palladium compound and the organic phosphine in the system described above.

Through this step, the palladium compound and the organic phosphine which are used as catalysts in the sequencing system can be removed, so as to avoid the residue of the palladium compound and the organic phosphine. Particularly, when a compound containing at least one of structural formulas 1-1, 1-2, and 1-3 is immobilized on the solid carrier, since the palladium compound is easily adsorbed on the surface of the solid carrier or remains in the nucleic acid immobilized on the solid carrier, the embodiment of the present application adopts a cleaning reagent comprising a divalent palladium removal reagent to carry out a surface cleaning treatment on the solid carrier. The "divalent palladium removal reagent" referred to in the embodiments of the present application refers to a reagent capable of removing the palladium compound. Through this step, the palladium compound and the organic phosphine which are used as catalysts in the sequencing system can be removed, so as to avoid the residue of the palladium compound and the organic phosphine. If the palladium compound remains in the sequencing system, the following effects may be brought: in the nucleotides of structural formulas 1-1 and 1-2 added in a new cycle of sequencing, under the action of the palladium compound and the organic phosphine, $-O-CH_2-N_3$ in a part of the nucleotides is reduced into hydroxy, so that a plurality of nucleotides are simultaneously introduced into a part of complementary strands in the new cycle of sequencing, resulting in prephasing (or prephase, which refers to the case that during sequencing, the nucleotides or analogs thereof introduced in the next cycle or next consecutive cycles of sequencing are subjected to a polymerization reaction in advance in the current cycle of sequencing and bind to the complementary strand of the nucleic acid template), and thus nucleic acid sequences of the introduced nucleotide regions cannot be accurately identified; or when a first imaging treatment for the introduced nucleotides is not available in the process of a new cycle of derivatization reaction, under the action of the palladium compound and the organic phosphine, $-O-CH(N_3)-L_2-R_4$ in a part of the nucleotides of structural formulas 1-2 and 1-3 has been reduced, so that signals generated by detectable groups cannot be acquired by the first imaging treatment, and thus the type of the introduced nucleotides in this cycle of extension reaction cannot be identified.

In some embodiments, the divalent palladium removal reagent comprises at least one of allyl, oxygen azidoalkyl, and disulfide bonds. The organic phosphine can be washed away with the cleaning reagent during the cleaning process with the cleaning reagent, but the palladium compound is easy to form a complex to be adsorbed or to remain, for example, to remain in the bases of a nucleotide, and therefore, it is necessary to carry out a cleaning treatment of the palladium

compound with the use of a cleaning reagent capable of efficiently removing the palladium compound. Allyl, oxygen azidoalkyl, and disulfide bond groups can react with the palladium compound to form a complex, and residual divalent palladium in the reaction system can be effectively cleaned by removing the complex. Particularly, when the sequenced nucleic acid template is immobilized on the surface of the solid carrier, a cleaning reagent is applied to the surface of the solid carrier, and allyl, oxygen azidoalkyl, and disulfide bond groups can react with divalent palladium to form a complex; further removing the cleaning reagent on the surface of the solid carrier can achieve the purpose of removing the residual palladium ions.

In one possible implementation of this embodiment, the divalent palladium removal reagent is selected from at least one of ethyl isocyanoacetate, cysteine or a salt thereof, glutathione, potassium isocyanoacetate, and cystamine. The removal reagent provided in the implementation comprises a reactive group capable of forming a complex structure with divalent palladium and capable of removing the palladium compound from the system through a complex reaction.

As one possible embodiment of the cleaning reagent in the present application, the divalent palladium removal reagent has a concentration of 1-40 mmol/L. When the concentration of the divalent palladium removal reagent is within this range, the palladium compound can exert the effect of metal complexation.

It should be understood that the cleaning reagent provided in the embodiments of the present application can be used not only in the cleaning step of step S4 in the sequencing method, but also in other situations where the palladium compound needs to be removed. The cleaning reagent provided in the embodiments of the present application can be directly added or used as a mixed solution. Different reagent components can also be aliquoted into different sample tubes, each sample tube is placed in the kit, and when in use, the reagent is mixed or input into a reactor or the surface of the reactor through a fluid pipeline as required.

The above is merely an example of one cycle of sequencing. In some embodiments, the sequencing further includes: carrying out data analysis on the detectable signal acquired in step S3 to determine the type of the introduced compound II. Illustratively, the detectable group is a fluorophore, and a fluorescence signal (including color, intensity, and the like) at each site of the solid carrier in one cycle of sequencing is detected, so as to obtain the type of compound II corresponding to the site. Thus, the type of compound II incorporated into the complementary strand is sequentially obtained by acquiring the fluorescence signal generated by the compound II in each cycle of sequencing, so as to obtain a nucleotide sequence of the complementary strand.

In the embodiments of the present application, the sequencing method further includes: carrying out signal acquisition on the sequencing system after cleaning treatment. By this step, the degree of the removal of the detectable group in the cleaned sequencing system can be checked. Similarly, the signal acquisition method for the cleaned sequencing system can be the same as that of step S2. Illustratively, when the detectable group is a fluorophore, the sequencing system after the cleaning treatment is subjected to an imaging treatment, and the removal or participation of the detectable group is analyzed by the acquired fluorescence signal.

Correspondingly, the embodiment of the present application provides a kit.

The cleavage reagent is used for cleaving a compound III containing an azidomethoxy group so as to reduce the azidomethoxy group in the compound III into hydroxy, the azidomethoxy group has a chemical formula of $-O-A_1(N_3)-A_2$, and $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

The cleavage reagent comprises a palladium compound and an organic phosphine.

Specifically, in this embodiment, the cleavage reagent comprises a palladium compound and an organic phosphine, and under the catalytic action of the palladium compound, the efficiency of reducing the azidomethoxy group with the chemical formula of $-O-A_1(N_3)-A_2$ into hydroxy by the organic phosphine is accelerated.

In the cleavage reagent, the organic phosphine is used as a reducing agent for the reduction reaction and is used for carrying out an oxidation-reduction reaction with the azidomethoxy group to reduce the azidomethoxy group into hydroxy. In this embodiment, when an organic phosphine having a trivalent valence (i.e., a trivalent organic phosphine) and a compound containing an azidomethoxy group are subjected to an oxidation-reduction reaction, the rate of the oxidation-reduction reaction is increased under the action of a palladium compound, which is probably because: the trivalent organic phosphine, the compound containing the azidomethoxy group, and the palladium compound form a conjugate-complex system, and under the combined action of the palladium compound and the trivalent organic phosphine, the azidomethoxy group rapidly removes one molecule of $N_2$ and is reduced into a hydroxyl structure. Illustratively, the organic phosphine is selected from at least one of tris(3-hydroxypropyl)phosphine, tris(2-carboxyethyl) phosphine, tris(hydroxymethyl)phosphine, triphenylphosphine, and tris(2-hydroxyethyl)phosphine.

In this embodiment, palladium used as a catalyst may be one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a

hexavalent palladium compound. The type of the palladium compound is not strictly limited, and may be an organic compound or an ionic compound of palladium. In some embodiments, the palladium compound may be selected from at least one of a palladium halate, a palladium halide, an organic palladium carboxylate, and an allylpalladium halide dimer of palladium. The palladium compound described above can act together with the organic phosphine on the azidomethoxy group in the compound, so as to accelerate the reduction process of the azidomethoxy group by the organic phosphine and finally improve the reduction efficiency of the azidomethoxy group in the compound. Illustratively, taking the divalent palladium compound as an example, it may be selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate. These divalent palladium compounds synergistically react with the organic phosphine to contribute to accelerating the reduction of the azidomethoxy group in the compound III.

According to the cleavage reagent provided in the embodiments of the present application, the organic phosphine and the palladium compound are in a molar ratio of 2:1 to 2000:1. Illustratively, the palladium compound and the organic phosphine in the cleavage reagent may be in a molar ratio of 2:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1, 100:1, 200:1, 500:1, 800:1, 1000:1, 1200:1, 1500:1, 1800:1, 2000:1, and the like.

In some embodiments, when the cleavage reagent is used for sequencing, the palladium compound can accelerate the reduction efficiency of the azidomethoxy group by the organic phosphine. However, when the palladium compound and the organic phosphine are in different contents, the synergistic effect is different. After repeated research by researchers, it is found that: when the palladium compound and the organic phosphine are in a molar ratio of 2:1 to 100:1, it is relatively significant that the palladium compound and the organic phosphine are subjected to a synergistic reaction to improve the reaction efficiency of reducing the azidomethoxy group into hydroxy. Specifically, when the organic phosphine and the palladium compound are in a molar ratio of 2:1 to 100:1, the compound (compound I described above) containing the azidomethoxy group is mixed with the palladium compound and the organic phosphine for 20-30 s, and the reduction efficiency of reducing the azidomethoxy group in the compound into hydroxy can be 70%-100%.

It should be understood that the cleavage reagent further comprises a solvent, which is selected taking into account, in one aspect, the solubility properties of the organic phosphine and the palladium compound, and in another aspect, the solvent properties of the azidomethoxy group to be reacted, and the reaction inertia with the azidomethoxy group. In some embodiments, the solvent in the cleavage reagent is a buffer. The buffer can help to stabilize the pH environment of the reaction system, and is conducive to a stable reaction of reducing the azidomethoxy group into hydroxy. In this embodiment, the buffer may be selected from at least one of a primary amine buffer and a tertiary amine buffer. Compared with the primary amine buffer, the tertiary amine buffer can provide a better and stable reaction environment for the reduction of the azidomethoxy group into hydroxy. In terms of the reaction effect, under otherwise identical conditions, compared with a reaction system adopting the primary amine buffer, the reaction efficiency of reducing the azidomethoxy group into hydroxy by the organic phosphine catalyzed by the palladium compound is higher when the tertiary amine buffer is adopted as a reaction system. Illustratively, the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and N,N-dihydroxyethylglycine.

In this embodiment, the cleavage reagent further comprises metal ions selected from at least one of sodium ions and potassium ions. The metal ions described above provide a suitable concentration of sodium ions and/or potassium ions for the reaction system, and the metal ions provide a suitable reaction environment for the organic phosphine to reduce the azidomethoxy group catalyzed by the palladium compound. It should be understood that the metal ions in this embodiment may be derived from at least one of halide salts, carbonates, acetates, phosphates, and borates containing the metal ions. Anions of these salts have reaction inertia in the reduction reaction system, which does not affect the reduction reaction of the azidomethoxy group in the compound by adopting the organic phosphine and the palladium compound. Illustratively, the cleavage reagent comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

Researches find that: when the concentration of the metal ions, i.e., sodium ions and/or potassium ions, in the cleavage reagent is 0.5-2.5 mol/L, the reaction efficiency of reducing the azidomethoxy group into hydroxy by the organic phosphine and the palladium compound is improved, and within this range, the reduction efficiency of the azidomethoxy group by the organic phosphine and the palladium compound is improved along with the increase of the concentration of the metal ions. Illustratively, in various applications, the metal ions in the cleavage reagent may have a concentration of 0.5 mol/L, 0.6 mol/L, 0.7 mol/L, 0.8 mol/L, 0.9 mol/L, 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, and the like.

In one embodiment, the cleavage reagent comprises at least sodium chloride at a concentration of 1.0-2.0 mol/L. Moreover, the cleavage reagent may further comprise at least one of other metal salts such as potassium chloride, sodium

carbonate, sodium borate, sodium bicarbonate, potassium carbonate, potassium bicarbonate, sodium acetate, sodium phosphate, sodium dihydrogen phosphate, disodium hydrogen phosphate, and sodium borate, at a concentration of 20-500 mmol/L.

In the cleavage reagent, the source of the azidomethoxy group is not limited, and theoretically, compounds which contain the azidomethoxy group and need to reduce the azidomethoxy group into hydroxy can be treated with the cleavage reagent. The cleavage reagent provided in the embodiments of the present application is described below with reference to a specific example.

In this example, compound III is selected from at least one of the following structural formulas 1-1, 1-2, and 1-3:

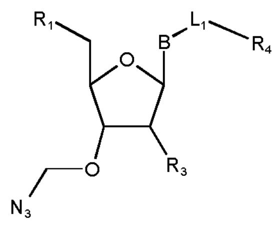

Formula 1-1

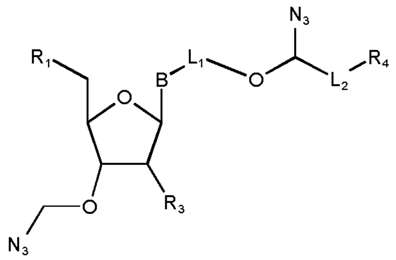

Formula 1-2

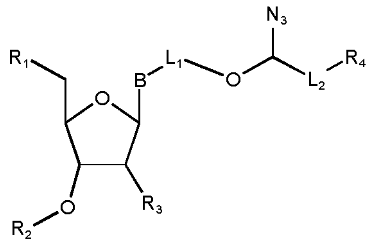

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ contains a detectable group.

The cleavage reagent provided in the embodiments of the present application may be used for reducing azidomethoxy groups in the chemical structures of the three types of nucleotides, that is: $-O-CH_2-N_3$ and/or $-O-CH(N_3)-L_2-R_4$ in structural formulas 1-1, 1-2, and 1-3 is reduced into -OH.

Among them, when the cleavage reagent contacts and reacts with the compound containing formula 1-1, the palladium compound and the organic phosphine act on the compound III of formula 1-1 synergistically to reduce $-O-CH_2-N_3$ in the compound of formula 1-1 into -OH; when the cleavage reagent is mixed and reacts with the compound III containing formula 1-2, the palladium compound and the organic phosphine act on the compound of formula 1-2 synergistically to reduce $-O-CH_2-N_3$ and/or $-O-CH(N_3)-L_2-R_4$ in the compound III of formula 1-2 into -OH, respectively, when the cleavage reagent is mixed and reacts with the compound III containing formula 1-3, the palladium compound and the organic phosphine act on the compound of formula 1-3 synergistically to reduce $-O-CH(N_3)-L_2-R_4$ in the compound of formula 1-3 into -OH. It should be understood that when two or more of structural formulas 1-1, 1-2, and 1-3 are contained in the reaction system at the same time, the cleavage reagent, particularly the palladium compound and the organic phosphine in the cleavage reagent, can act on $-O-CH_2-N_3$ and $-O-CH(N_3)-L_2-R_4$ of the two or more types of compounds, and reduce $-O-CH_2-N_3$ and $-O-CH(N_3)-L_2-R_4$ into -OH, respectively.

When at least one of structural formulas 1-1, 1-2, and 1-3 provided in this example is used as dNTPs for gene sequencing, $-O-CH_2-N_3$ and $-O-CH(N_3)-L_2-R_4$ are used as reversible terminating groups, such that only one dNTP can be introduced into a nucleic acid strand in each cycle of sequencing, after the type of the introduced dNTPs is identified or determined, the inhibiting groups $-O-CH_2-N_3$ and $-O-CH(N_3)-L_2-R_4$ in the introduced dNTPs for inhibiting the reaction of the next dNTP can be removed by adding a cleavage reagent comprising a palladium compound and an organic phosphine into the sequencing system for reaction. Removing the inhibiting groups in the introduced dNTPs in this way can effectively improve the removal efficiency, which is further conducive to improving the sequencing rate. Particularly, when the number of sequencing cycles is increased, the effect of improving the sequencing rate is more significant.

In the cleavage reagent used for the compound structure described above, the palladium compound and the organic phosphine are in a molar ratio of 5:1 to 50:1. When the molar ratio of the two is within the range described above, the palladium compound and the organic phosphine in the cleavage reagent are mixed and react with the compound containing the azidomethoxy group with a structural general formula of $-O-CH(N_3)-A_1$ for 20-30 s, so that the reduction efficiency of reducing the azidomethoxy group in the compound into hydroxy is up to 95%-100%. When the palladium compound and the organic phosphine in the cleavage reagent are in a molar ratio of 5:1 to 20:1, the palladium compound and the organic phosphine are mixed and react with the compound containing the azidomethoxy group with the structural general formula of $-O-CH(N_3)-A_1$ for 20-30 s, so that the reduction efficiency of reducing the azidomethoxy group in the compound into hydroxy is up to 98%-100%.

The cleavage reagent used for the compound structure (structural formulas 1-1, 1-2, and 1-3) described above comprises metal ions selected from at least one of sodium ions and potassium ions and a buffer at the same time. The metal ions and the buffer provide a good chemical reaction environment for sequencing, and simultaneously provide a

good reaction medium for the reduction of $-O-CH_2-N_3$ and/or $-O-CH(N_3)-L_2-R_4$ in structural formulas 1-1, 1-2, and 1-3. In one possible implementation of this embodiment, the metal ions in the mixed system have a concentration of 1.0-2.5 mol/L. High-concentration metal ions are conducive to improving the reduction efficiency of the azidomethoxy groups, particularly $-O-CH_2-N_3$ and $-O-CH(N_3)-L_2-R_4$ in structural formulas 1-1, 1-2, and 1-3, by the palladium compound and the organic phosphine. In some implementations, the metal ions have a concentration of 1.2-2.0 mol/L.

When the concentration of the buffer is 50-180 mmol/L, the reduction reaction of $-O-CH_2-N_3$ and $-O-CH(N_3)-L_2-R_4$ in structural formulas 1-1, 1-2, and 1-3 by the palladium compound and the organic phosphine is in a stable pH environment, thereby being conducive to the improvement of the uniformity and stability of the reaction. Particularly, when the cleavage reagent is used for sequencing, and the reagent in the cleavage reagent flows through the surface of the compounds containing $-O-CH_2-N_3$ and $-O-CH(N_3)-L_2-R_4$ in structural formulas 1-1, 1-2, and 1-3, which are immobilized on the substrate, for reaction, the buffer system is conducive to improving the stability and uniformity of the reaction on the surface of the substrate. In some implementations, the buffer has a concentration of 100-150 mmol/L.

It should be understood that in the embodiments of the present application, each component in the cleavage reagent can be directly added or used as a mixed solution. Different components can also be aliquoted into different sample tubes, each sample tube is placed in the kit, and when in use, the reagent is mixed or input into a reactor or the surface of the reactor through a fluid pipeline as required.

In some embodiments, the kit further comprises a cleaning reagent, which includes a divalent palladium removal reagent, and the divalent palladium removal reagent comprises at least one of allyl, oxygen azidoalkyl, and disulfide bonds. Allyl, oxygen azidoalkyl, and disulfide bond groups can react with the palladium compound to form a complex, and residual divalent palladium in the reaction system can be effectively cleaned by removing the complex. Particularly, in the method provided in the first aspect or the third aspect, when the reduction reaction occurs on the surface of the solid carrier, a cleaning reagent solvent is applied to the surface of the solid carrier, and allyl, oxygen azidoalkyl, and disulfide bond groups can react with divalent palladium to form a complex; further removing the cleaning reagent on the surface of the solid carrier can achieve the purpose of removing the residual palladium ions.

In one possible implementation of this embodiment, the divalent palladium removal reagent is selected from at least one of ethyl isocyanoacetate, cysteine or a salt thereof, glutathione, potassium isocyanoacetate, and cystamine. The removal reagent provided in the implementation comprises a reactive group capable of forming a complex structure with divalent palladium and capable of removing the divalent palladium from the system through a complex reaction.

As one possible embodiment of the cleaning reagent in the present application, the divalent palladium removal reagent has a concentration of 1-40 mmol/L. When the concentration of the divalent palladium removal reagent is within this range, the divalent palladium can exert the effect of palladium ion complexation.

It should be understood that in the embodiments of the present application, the cleaning reagent and the cleavage reagent are aliquoted into different sample tubes, each sample tube is placed in the kit, and when in use, the reagent is input into a reactor or the surface of the reactor through a fluid pipeline as required, respectively.

The present application will be illustrated with reference to the following specific examples.

**Example 1**

A method for reducing azidomethoxy is as follows:

THPP and sodium tetrachloropalladate were placed in Tris buffer containing NaCl to form a mixed solution S1, the concentration of each component in the mixed solution S1 was regulated as follows: 12.5 mmol/L of THPP, 0.025 mmol/L of sodium tetrachloropalladate, 500 mmol/L of NaCl, and 25 mmol/L of Tris, and hydrochloric acid was added to adjust the pH value of the mixed solution S1 to be 9.2.

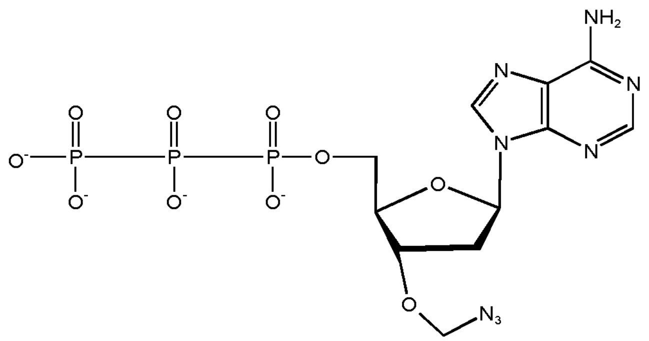

Formula 2

5'-Triphosphate-3'-O-azidomethyladenine nucleotide of the above formula 2 was mixed and reacted with the mixed solution S1 at a temperature of 60 °C for 5 s and then quenched, and -O-azidomethyl in the 5'-triphosphate-3'-O-azidomethyladenine nucleotide was reduced into 5'-triphosphate-3'-hydroxyadenine nucleotide of the following formula 2'

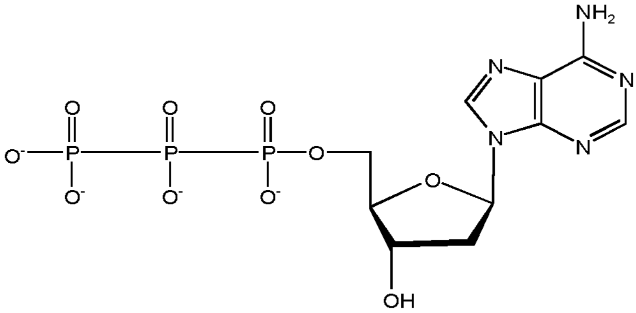

.

**Comparative Example 1**

A method for reducing azidomethoxy is as follows:
THPP and sodium tetrachloropalladate were placed in Tris buffer containing NaCl to form a mixed solution D1, the concentration of each component in the mixed solution D1 was regulated as follows: 12.5 mol/L of THPP, 500 mmol/L of NaCl, and 25 mmol/L of Tris, and hydrochloric acid was added to adjust the pH value of the mixed solution D1 to be 9.2.

5'-Triphosphate-3'-O-azidomethylthymine nucleotide of the above formula 2 was mixed and reacted with the mixed solution D1 at a temperature of 60 °C for 5 s and then quenched, and -O-azidomethyl in the 5'-triphosphate-3'-O-azidomethylthymine nucleotide was reduced into -OH.

The quenched liquid phase systems in Example 1 and Comparative Example 1 were subjected to high-performance liquid chromatography (HPLC) using a 4.6 × 250 mm phenomenex gemini NX-C18 110A chromatographic column under the following analysis conditions:

flow velocity: 1 mL/min,
column temperature: 25 °C, and
mobile phase: TEAB and acetonitrile.

Before HPLC analysis, a sample was 10-fold diluted with an initial mobile phase, such that the loading concentration was 100 μmol/L.

The HPLC patterns of the quenched liquid phase systems in Example 1 and Comparative Example 1 are shown in FIG. 1 (the horizontal axis represents the retention time in min; the vertical axis represents the absorbance value in mAU). The upper pattern is the HPLC pattern of the quenched liquid phase system in Example 1, and the lower pattern is the HPLC pattern of the quenched liquid phase system in Comparative Example 1. In the figure, the absorption peak (left peak) corresponding to the retention time of 8.587 min is the compound of formula 2', and the absorption peak (right peak) corresponding to the retention time of 9.420 min is the compound of formula 2. In FIG. 1, compared with Comparative Example 1, the peak area of the compound of formula 2' in the liquid phase system after the reaction in Example 1 was increased, that is, the content of the compound of formula 2' was increased, meanwhile, the peak area of the compound of formula 2 was decreased, that is, the content of the compound of formula 2 was decreased.

The peak area of the 5'-triphosphate-3'-O-azidomethyladenine nucleotide in the solution system before the reaction was labeled as $S_1$, the peak area of the 5'-triphosphate-3'-O-azidomethyladenine nucleotide in the solution system after the reaction was labeled as $S_2$, and the cleavage efficiency was calculated according to the following method.

$$\text{Cleavage efficiency (\%)} = \frac{S_1 - S_2}{S_1} \times 100\%$$

The statistical calculation showed that compared with the reaction system without palladium in Comparative Example 1, the cleavage efficiency was increased by 61.42% after the palladium was added for the reaction in Example 1.

As can be seen, under the same liquid phase reaction condition, the cleavage efficiency of reducing -O-azidomethyl in the 5'-triphosphate-3'-O-azidomethyladenine nucleotide into -OH can be significantly improved by adding the organic palladium.

**Example 2**

A method for reducing azidomethoxy is as follows:

THPP and sodium tetrachloropalladate were placed in Tris buffer containing NaCl to form a mixed solution S2, the concentration of each component in the mixed solution S2 was regulated as follows: 100 mmol/L of THPP, 10 mmol/L of sodium tetrachloropalladate, 2.0 mol/L of NaCl, and 100 mmol/L of Tris, and hydrochloric acid was added to adjust the pH value of the mixed solution S2 to be 9.2.

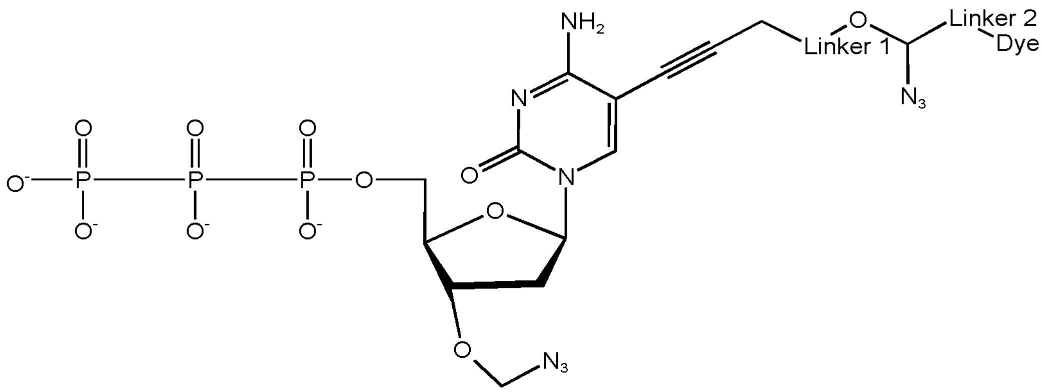

Formula 3

5'-Triphosphate-3'-O-azidomethylthymine nucleotide of the above formula 3 containing a fluorophore was immobilized on the surface of a solid carrier. The mixed solution S2 was added to the surface of the solid carrier, and the mixed solution S2 was allowed to flow through the 5'-triphosphate-3'-O-azidomethylthymine nucleotide via a channel structure. The time for adding the mixed solution S2 was 23 s (taking a certain site on the surface of the solid carrier as an example, the time for the mixed solution S2 to flow through the site was 23 s). During the addition, the mixed solution S2 was heated to 60 °C and left to stand at this temperature for 0 s, 2 s, and 5 s to reduce -O-azidomethyl in the 5'-triphosphate-3'-O-azidomethylthymine nucleotide into -OH.

**Example 3**

A method for reducing azidomethoxy is as follows:
THPP and sodium tetrachloropalladate were placed in ethanolamine containing NaCl to form a mixed solution S3, the concentration of each component in the mixed solution S3 was regulated as follows: 100 mmol/L of THPP, 10 mmol/L of sodium tetrachloropalladate, 2.0 mol/L of NaCl, and 100 mmol/L of ethanolamine, and hydrochloric acid was added to adjust the pH value of the mixed solution S3 to be 9.2.

5'-Triphosphate-3'-O-azidomethylthymine nucleotide of formula 3 containing a fluorophore was immobilized on the surface of a solid carrier. The mixed solution S3 was added to the surface of the solid carrier, and the mixed solution S3 was allowed to flow through the 5'-triphosphate-3'-O-azidomethylthymine nucleotide via a channel structure. The time for adding the mixed solution S3 was 23 s (taking a certain site on the surface of the solid carrier as an example, the time for the mixed solution S3 to flow through the site was 23 s). During the addition, the mixed solution S3 was heated to 60 °C and left to stand at this temperature for 0 s, 2 s, and 5 s to reduce -O-azidomethyl in the 5'-triphosphate-3'-O-azidomethylthymine nucleotide into -OH.

**Example 4**

A method for reducing an azidomethoxy group is substantially the same as that of Example 2, except that a mixed solution S4 was used in place of the mixed solution S2 in Example 2, and the mixed solution S4 was prepared by a method including the following steps:
THPP and sodium tetrachloropalladate were placed in ethanolamine containing NaCl to form the mixed solution S4, wherein the buffer was a mixed solution of ethanolamine and tetraethylethylenediamine, the concentration of each component in the mixed solution S4 was regulated as follows: 100 mmol/L of THPP, 10 mmol/L of sodium tetrachloropalladate, 2.0 mol/L of NaCl, 50 mmol/L of ethanolamine, and 50 mmol/L of tetraethylethylenediamine, and hydrochloric acid was added to adjust the pH value of the mixed solution S4 to be 9.2.

**Comparative Example 2**

A method for reducing an azidomethoxy group is substantially the same as that of Example 2, except that a mixed solution D2 was used in place of the mixed solution S2 in Example 2, and the mixed solution D2 was prepared by a method including the following steps:
THPP was placed in Tris buffer containing NaCl to form the mixed solution D2, the concentration of each component in the mixed solution D2 was regulated as follows: 100 mmol/L of THPP, 2.0 mol/L of NaCl, and 100 mmol/L of Tris, and hydrochloric acid was added to adjust the pH value of the mixed solution D2 to be 9.0.

**Comparative Example 3**

A method for reducing an azidomethoxy group is substantially the same as that of Example 2, except that a mixed solution D3 was used in place of the mixed solution S2 in Example 2, and the mixed solution D3 was prepared by a method including the following steps:
THPP was placed in ethanolamine containing NaCl to form the mixed solution D3, the concentration of each component in the mixed solution D3 was regulated as follows: 100 mmol/L of THPP, 2.0 mol/L of NaCl, and 100 mmol/L of ethanolamine, and hydrochloric acid was added to adjust the pH value of the mixed solution D3 to be 9.0.

**Comparative Example 4**

A method for reducing an azidomethoxy group is substantially the same as that of Example 2, except that a mixed solution D4 was used in place of the mixed solution S2 in Example 2, and the mixed solution D4 was prepared by a method including the following steps:
THPP was placed in a buffer containing NaCl to form the mixed solution D4, wherein the buffer was a mixed solution of ethanolamine and tetraethylethylenediamine, the concentration of each component in the mixed solution D4 was regulated as follows: 100 mmol/L of THPP, 2.0 mol/L of NaCl, 50 mmol/L of ethanolamine, and 50 mmol/L of tetraethylethylenediamine, and hydrochloric acid was added to adjust the pH value of the mixed solution D4 to be 9.0.

In the methods for reducing azidomethoxy of Examples 2-4 and Comparative Examples 2-4, after the reaction was carried out according to the reaction conditions in the examples or comparative examples, a cleaning reagent was used to wash away the reaction reagent on the surface of the solid carrier, such that the reaction of reducing -O-azidomethyl in the 5'-triphosphate-3'-O-azidomethylthymine nucleotide into -OH was terminated. Fluorescence images before and after the reaction were acquired by a microscope, and the spot density in the fluorescence images was counted. A method for determining the spot density was as follows: extracting data in the fluorescence images, carrying out binarization treatment, carrying out Gaussian fitting, calculating the coordinate in each image, and then carrying out chromatic aberration construction; combining the point coordinate in each image, taking a union set of the point coordinates as a template reference coordinate, and determining the spot density.

The spot density in the fluorescence images acquired before the reaction (the fluorescence image acquired from the surface of the solid carrier before the mixed solution was added) was labeled as M1, the spot density in the fluorescence image acquired after the reaction (the cleaning reagent was used to wash away the reaction reagent on the surface of the solid carrier, such that the reaction of reducing -O-azidomethyl in the 5'-triphosphate-3'-O-azidomethylthymine nucleotide into -OH was terminated) was labeled as M2, and the cleavage efficiency was calculated according to the following method. It should be understood that in all examples (Examples 2-40) and comparative examples (Comparative Examples 2-5) for the reaction on the surface of the solid carrier in the present application, the cleavage efficiencies were characterized by this method (the method and conditions for acquiring fluorescence images before and after the reaction by the microscope were identical) and calculated by the following formula.

$$\text{Cleavage efficiency (\%)} = \frac{M1-M2}{M1} \times 100\%$$

The results of the cleavage efficiencies in Examples 2-4 and Comparative Examples 2-4 are shown in Table 1 below, and the statistical graph is shown in FIG. 2:

Table 1

| Cleavage efficiency (%) | Example 2 | Example 3 | Example 4 | Comparative Example 2 | Comparative Example 3 | Comparative Example 4 |
|---|---|---|---|---|---|---|
| Standing for 0 s | 76 | 73 | 50 | / | 23 | 21 |

(continued)

| Cleavage efficiency (%) | Example 2 | Example 3 | Example 4 | Comparative Example 2 | Comparative Example 3 | Comparative Example 4 |
|---|---|---|---|---|---|---|
| Standing for 2 s | 91 | 88 | 70 | 62 | 42 | 42 |
| Standing for 5 s | 98 | 96 | 77 | 81 | 61 | 56 |

With reference to Table 1 and FIG. 2, comparing Example 2 with Comparative Example 2, Example 3 with Comparative Example 3, and Example 4 with Comparative Example 4, respectively, under otherwise identical conditions, the cleavage efficiencies of reducing -O-azidomethyl in the 5'-triphosphate-3'-O-azidomethylthymine nucleotide into -OH after the addition of the palladium compound were significantly improved in Examples 2-4 of the present application, compared with Comparative Examples 2-4 without the palladium compound. Comparing Examples 2-4 and Comparative Examples 2-4, the cleavage efficiencies of reducing -O-azidomethyl in the 5'-triphosphate-3'-O-azidomethylthymine nucleotide into -OH in different buffer systems were different, but the cleavage efficiency was still significantly increased compared with the case where no palladium compound was added.

**Examples 5-8**

A method for reducing azidomethoxy is as follows:
THPP and sodium tetrachloropalladate were placed in Tris buffer containing NaCl to form mixed solutions S5-S8 (the mixed solution in Example 5 was labeled as S5, the mixed solution in Example 6 was labeled as S6, the mixed solution in Example 7 was labeled as S7, and the mixed solution in Example 8 was labeled as S8), and the molar ratio of THPP to sodium tetrachloropalladate in each mixed solution was regulated to 10:1. The concentrations of THPP in S5-S8 were as follows: the concentration of THPP in Example 5 was 10 mmol/L, the concentration of THPP in Example 6 was 25 mmol/L, the concentration of THPP in Example 7 was 50 mmol/L, and the concentration of THPP in Example 8 was 100 mmol/L. Sodium tetrachloropalladate was 10 mmol/L, NaCl was 2.0 mol/L, and Tris was 100 mmol/L. The pH value of each mixed solution was adjusted to 9.5 with hydrochloric acid.

5'-Triphosphate-3'-O-azidomethylthymine nucleotide of the above formula 3 containing a fluorophore was immobilized on the surface of a solid carrier. The mixed solutions S5-S8 were added to the surface of the solid carrier, respectively (the mixed solution S5 was added in Example 5, the mixed solution S6 was added in Example 6, the mixed solution S7 was added in Example 7, and the mixed solution S8 was added in Example 8), and the mixed solutions S5-S8 were allowed to flow through the 5'-triphosphate-3'-O-azidomethylthymine nucleotide via a channel structure. The time for adding the mixed solutions S5-S8 was 23 s (taking a certain site on the surface of the solid carrier as an example, the time for the mixed solutions S5-S8 to flow through the site was 23 s). During the addition, the mixed solutions S5-S8 were heated to 60 °C and left to stand at this temperature for 0 s, 2 s, and 5 s to reduce -O-azidomethyl in the 5'-triphosphate-3'-O-azidomethylthymine nucleotide into -OH.

The cleavage efficiencies of Examples 5-8 were calculated. The results are shown in Table 2:

Table 2

| Cleavage efficiency (%) | Example 5 | Example 6 | Example 7 | Example 8 |
|---|---|---|---|---|
| Standing for 0 s | 46 | 68 | 70 | 71 |
| Standing for 2 s | 52 | 74 | 86 | 88 |
| Standing for 5 s | 63 | 92 | 97 | 99 |

With reference to Table 2, it is found by comparing Examples 5-8 that: under otherwise identical conditions, when the concentration of THPP is in the range of 25-100 mmol/L, the cleavage efficiency of reducing -O-azidomethyl in the 5'-triphosphate-3'-O-azidomethylthymine nucleotide into -OH is improved along with the improvement of the concentration of THPP, and the cleavage efficiency of reducing -O-azidomethyl in the 5'-triphosphate-3'-O-azidomethylthymine nucleotide into -OH substantially tends to be stable after the concentration of THPP is higher than 50 mmol/L.

It is found by comparing Examples 2 and 8 that, under otherwise identical conditions, the cleavage efficiency of reducing -O-azidomethyl in the 5'-triphosphate-3'-O-azidomethylthymine nucleotide into -OH is improved when the pH value of the mixed solution is 9.5, compared with the case where the pH value of the mixed solution is 9.2.

**Examples 9-12**

A method for reducing azidomethoxy is as follows:
THPP and sodium tetrachloropalladate were placed in Tris buffer containing NaCl to form mixed solutions S9-S12 (the mixed solution in Example 9 was labeled as S9, the mixed solution in Example 10 was labeled as S10, the mixed solution in Example 11 was labeled as S11, and the mixed solution in Example 12 was labeled as S12), and the molar ratio of THPP to sodium tetrachloropalladate in each mixed solution was regulated to 10:1. The concentrations of THPP in S9-S12 were as follows: the concentration of THPP in Example 9 was 10 mmol/L, the concentration of THPP in Example 10 was 25 mmol/L, the concentration of THPP in Example 11 was 50 mmol/L, and the concentration of THPP in Example 12 was 100 mmol/L. Sodium tetrachloropalladate was 10 mmol/L, NaCl was 2.0 mol/L, and Tris was 100 mmol/L. The pH value of each mixed solution was adjusted to 9.2 with hydrochloric acid.

5'-Triphosphate-3'-O-azidomethylthymine nucleotide of the above formula 3 containing a fluorophore was immobilized on the surface of a solid carrier. The mixed solutions S9-S12 were added to the surface of the solid carrier, respectively (the mixed solution S9 was added in Example 9, the mixed solution S10 was added in Example 10, the mixed solution S11 was added in Example 11, and the mixed solution S12 was added in Example 12), and the mixed solutions S9-S12 were allowed to flow through the 5'-triphosphate-3'-O-azidomethylthymine nucleotide via a channel structure. The time for adding the mixed solutions S9-S12 was 23 s (taking a certain site on the surface of the solid carrier as an example, the time for the mixed solutions S9-S12 to flow through the site was 23 s). During the addition, the mixed solutions S9-S12 were heated to 70 °C and left to stand at this temperature for 0 s to reduce -O-azidomethyl in the 5'-triphosphate-3'-O-azidomethylthymine nucleotide into -OH.

The cleavage efficiencies of Examples 9-12 were calculated, and the cleavage efficiencies of Examples 9-12 were 81%, 95%, 97%, and 98%, respectively.

Comparing the cleavage efficiencies of Examples 9-12 with those of Examples 5-8 when the mixed solutions were left to stand for 0 s, it is found that: the reduction efficiency of -O-azidomethyl in the 5'-triphosphate-3'-O-azidomethylthymine nucleotide into hydroxy is improved when the temperature is increased to 70 °C compared with the reaction temperature of 60 °C.

**Examples 13-16**

A method for reducing azidomethoxy is as follows:
THPP and sodium tetrachloropalladate were placed in ethanolamine containing NaCl to form mixed solutions S13-S16 (the mixed solution in Example 13 was labeled as S13, the mixed solution in Example 14 was labeled as S14, the mixed solution in Example 15 was labeled as S15, and the mixed solution in Example 16 was labeled as S16). The concentrations of ethanolamine in the mixed S13-S16 were regulated as follows: the concentration of ethanolamine in Example 13 was 50 mmol/L, the concentration of ethanolamine in Example 14 was 100 mmol/L, the concentration of ethanolamine in Example 15 was 150 mmol/L, and the concentration of ethanolamine in Example 16 was 200 mmol/L. THPP was 100 mmol/L, sodium tetrachloropalladate was 10 mmol/L, and NaCl was 2.0 mol/L. The pH value of each mixed solution was adjusted to 9.0 with hydrochloric acid.

5'-Triphosphate-3'-O-azidomethylthymine nucleotide of the above formula 3 containing a fluorophore was immobilized on the surface of a solid carrier. The mixed solutions S13-S16 were added to the surface of the solid carrier, respectively (the mixed solution S13 was added in Example 13, the mixed solution S14 was added in Example 14, the mixed solution S15 was added in Example 15, and the mixed solution S16 was added in Example 16), and the mixed solutions S13-S16 were allowed to flow through the 5'-triphosphate-3'-O-azidomethylthymine nucleotide via a channel structure. The time for adding the mixed solutions S13-S16 was 23 s (taking a certain site on the surface of the solid carrier as an example, the time for the mixed solutions S13-S16 to flow through the site was 23 s). During the addition, the mixed solutions S13-S16 were heated to 60 °C and left to stand at this temperature for 0 s, 2 s, and 5 s to reduce -O-azidomethyl in the 5'-triphosphate-3'-O-azidomethylthymine nucleotide into -OH.

The cleavage efficiencies of Examples 13-16 were calculated. The results are shown in Table 3:

Table 3

| Cleavage efficiency (%) | Example 13 | Example 14 | Example 15 | Example 16 |
|---|---|---|---|---|
| Standing for 0 s | 60 | 61 | 65 | 61 |
| Standing for 2 s | 63 | 63 | 84 | 78 |
| Standing for 5 s | 81 | 86 | 93 | 91 |

With reference to Table 3, it is found by comparing Examples 13-16 that, under otherwise identical conditions,

when the concentration of ethanolamine is 100-200 mmol/L, the cleavage efficiency of reducing -O-azidomethyl in the 5'-triphosphate-3'-O-azidomethylthymine nucleotide into -OH is relatively high.

**Examples 17-20**

A method for reducing azidomethoxy is as follows:
THPP and sodium tetrachloropalladate were placed in ethanolamine containing NaCl to form mixed solutions S17-S20 (the mixed solution in Example 17 was labeled as S17, the mixed solution in Example 18 was labeled as S18, the mixed solution in Example 19 was labeled as S19, and the mixed solution in Example 20 was labeled as S20). The concentrations of ethanolamine in the mixed solutions S17-S20 were regulated as follows: the concentration of ethanolamine in Example 17 was 50 mmol/L, the concentration of ethanolamine in Example 18 was 100 mmol/L, the concentration of ethanolamine in Example 19 was 150 mmol/L, and the concentration of ethanolamine in Example 20 was 100 mmol/L. THPP was 100 mmol/L, sodium tetrachloropalladate was 10 mmol/L, and NaCl was 2.0 mol/L. The pH value of each mixed solution was adjusted to 9.0 with hydrochloric acid.

5'-Triphosphate-3'-O-azidomethylthymine nucleotide of the above formula 3 containing a fluorophore was immobilized on the surface of a solid carrier. The mixed solutions S17-S20 were added to the surface of the solid carrier, respectively (the mixed solution S17 was added in Example 17, the mixed solution S18 was added in Example 18, the mixed solution S19 was added in Example 19, and the mixed solution S20 was added in Example 20), and the mixed solutions S17-S20 were allowed to flow through the 5'-triphosphate-3'-O-azidomethylthymine nucleotide via a channel structure. The time for adding the mixed solutions S17-S20 was 23 s (taking a certain site on the surface of the solid carrier as an example, the time for the mixed solutions S17-S20 to flow through the site was 23 s). During the addition, the mixed solutions S17-S20 were heated to 70 °C and left to stand at this temperature for 0 s to reduce -O-azidomethyl in the 5'-triphosphate-3'-O-azidomethylthymine nucleotide into -OH.

The cleavage efficiencies of Examples 17-20 were calculated, and the cleavage efficiencies of Examples 17-20 were calculated to be 91%, 91%, 95%, and 96%, respectively.

Comparing the cleavage efficiencies of Examples 17-20 with those of Examples 13-16 when the mixed solutions were left to stand for 0 s, it is found that: the reduction efficiency of azidomethoxy is improved when the temperature is increased to 70 °C compared with the reaction temperature of 60 °C.

**Example 21**

A method for reducing azidomethoxy is as follows:
THPP and sodium tetrachloropalladate were placed in ethanolamine containing NaCl to form a mixed solution S21, the concentration of each component in the mixed solution S21 was regulated as follows: 100 mmol/L of THPP, 10 mmol/L of sodium tetrachloropalladate, and 150 mmol/L of ethanolamine, the concentrations of NaCl were divided into four groups of 0.5 mol/L, 1.0 mol/L, 1.5 mol/L, and 2.0 mol/L in turn, and the pH value of the mixed solution S21 was adjusted to 9.2 with hydrochloric acid. 5'-Triphosphate-3'-O-azidomethylthymine nucleotide of the above formula 3 containing a fluorophore was immobilized on the surface of a solid carrier. The mixed solution S21 was added to the surface of the solid carrier, and the mixed solution S21 was allowed to flow through the 5'-triphosphate-3'-O-azidomethylthymine nucleotide via a channel structure. The time for adding the mixed solution S21 was 23 s (taking a certain site on the surface of the solid carrier as an example, the time for the mixed solution S21 to flow through the site was 23 s). During the addition, the mixed solution S21 was heated to 60 °C and left to stand at this temperature for 0 s and 5 s to reduce -O-azidomethyl in the 5'-triphosphate-3'-O-azidomethylthymine nucleotide into -OH.

The cleavage efficiencies were calculated at different NaCl concentrations in Example 21. The results are shown in Table 4:

Table 4

| Cleavage efficiency (%) | 0.5 mol/L | 1.0 mol/L | 1.5 mol/L | 2.0 mol/L |
|---|---|---|---|---|
| Standing for 0 s | 54 | 47 | 60 | 85 |
| Standing for 5 s | 87 | 75 | 83 | 91 |

Table 4 shows that the cleavage efficiency is decreased first and then increased along with the increase of the concentration of sodium chloride in the mixed solution, and the cleavage efficiency is the highest when the concentration of the sodium chloride is 2.0 mol/L.

### Example 22

A method for reducing an azidomethoxy group is substantially the same as that of Example 21, except that THPP and sodium tetrachloropalladate were placed in ethanolamine containing NaCl to form a mixed solution S22. The mixed solution S22 was heated to 70 °C and left to stand at this temperature for 0 s. The cleavage efficiencies were calculated at different NaCl concentrations, and the results showed that when the concentrations of sodium chloride in the mixed solution were 0.5 mol/L, 1.0 mol/L, 1.5 mol/L, and 2.0 mol/L, respectively, the cleavage efficiency was 100%. Comparing the cleavage efficiency of Example 22 with that of Example 20 when the mixed solution was left to stand for 0 s, it is found that: the reaction temperature is increased, which is beneficial to improving the reduction efficiency of reducing azidomethoxy into hydroxy.

### Example 23

THPP and sodium tetrachloropalladate were placed in ethanolamine containing sodium tetraborate to form a mixed solution S23, the concentration of each component in the mixed solution S23 was regulated as follows: 100 mmol/L of THPP, 10 mmol/L of sodium tetrachloropalladate, 150 mol/L of ethanolamine, and 2 mol/L of NaCl, the concentrations of sodium tetraborate were divided into three groups of 150 mmol/L, 200 mmol/L, and 300 mmol/L in turn, and the pH value of the mixed solution S23 was adjusted to 9.2 with hydrochloric acid.

5'-Triphosphate-3'-O-azidomethylthymine nucleotide of the above formula 3 containing a fluorophore was immobilized on the surface of a solid carrier. The mixed solution S23 was added to the surface of the solid carrier, and the mixed solution S23 was allowed to flow through the 5'-triphosphate-3'-O-azidomethylthymine nucleotide via a channel structure. The time for adding the mixed solution S23 was 23 s (taking a certain site on the surface of the solid carrier as an example, the time for the mixed solution S23 to flow through the site was 23 s). During the addition, the mixed solution S23 was heated to 60 °C and left to stand at this temperature for 0 s and 5 s to reduce -O-azidomethyl in the 5'-triphosphate-3'-O-azidomethylthymine nucleotide into -OH.

The cleavage efficiencies were calculated at different sodium tetraborate concentrations in Example 23. The results are shown in Table 5:

Table 5

| Cleavage efficiency (%) | 150 mmol/L | 200 mmol/L | 300 mmol/L |
|---|---|---|---|
| Standing for 0 s | 89 | 95 | 97 |
| Standing for 5 s | 95 | 98 | 98 |

Table 5 shows that the cleavage efficiency is increased along with the increase of the concentration of carbonic acid in the mixed solution, and the cleavage efficiency tends to be stable after the concentration is 200 mmol/L.

### Example 24

A method for reducing an azidomethoxy group is substantially the same as that of Example 23, except that THPP and sodium tetrachloropalladate were placed in ethanolamine containing sodium tetraborate to form a mixed solution S24. During the addition, the mixed solution S24 was heated to 70 °C and left to stand at this temperature for 0 s. The cleavage efficiencies were calculated at different sodium tetraborate concentrations. The results showed that when the concentrations of sodium tetraborate in the mixed solution were 150 mmol/L, 200 mmol/L, and 300 mmol/L, the cleavage efficiencies were 97%, 99%, and 99%, respectively. Comparing the cases in Example 23 and Example 24 when the mixed solutions were left to stand for 0 s, it can be seen that: the reaction temperature is increased, which is beneficial to improving the reduction efficiency of reducing azidomethoxy into hydroxy.

### Examples 25-27

A method for reducing azidomethoxy is as follows:
THPP and sodium tetrachloropalladate were placed in Tris buffer containing NaCl to form mixed solutions S25-S27 (the mixed solution in Example 25 was labeled as S25, the mixed solution in Example 26 was labeled as S26, and the mixed solution in Example 27 was labeled as S27), and the concentration of each component in S25-S27 was as follows: the concentration of THPP was 100 mmol/L, NaCl was 2.0 mol/L, and Tris was 100 mmol/L. The concentrations of sodium tetrachloropalladate were as follows: the concentration of sodium tetrachloropalladate in Example 25 was 1.0 mmol/L, the concentration of sodium tetrachloropalladate in Example 26 was 10 mmol/L, and the concentration of sodium tetrachloropalladate in Example 27 was 20 mmol/L. The pH values of the mixed solution S25-S27 were adjusted to 9.2 with hydrochloric acid.

5'-Triphosphate-3'-O-azidomethylthymine nucleotide of the above formula 3 containing a fluorophore was immobilized on the surface of a solid carrier. The mixed solutions S25-S27 were added to the surface of the solid carrier, respectively (the mixed solution S25 was added in Example 25, the mixed solution S26 was added in Example 26, and the mixed solution S27 was added in Example 27), and the mixed solutions S25-S27 were allowed to flow through the 5'-triphosphate-3'-O-azidomethylthymine nucleotide via a channel structure. The time for adding the mixed solutions S25-S27 was 23 s (taking a certain site on the surface of the solid carrier as an example, the time for the mixed solutions S25-S27 to flow through the site was 23 s). During the addition, the mixed solutions S25-S27 were heated to 60 °C and left to stand at this temperature for 0 s and 2 s to reduce -O-azidomethyl in the 5'-triphosphate-3'-O-azidomethylthymine nucleotide into -OH.

**Comparative Example 5**

A method for reducing an azidomethoxy group is substantially the same as that of Examples 25-27, except that a mixed solution D5 did not contain sodium tetrachloropalladate, that is, THPP was placed in Tris buffer containing NaCl to form the mixed solution D5. The cleavage efficiencies of Examples 25-27 at different sodium tetrachloropalladate concentrations and that of Comparative Example 5 were calculated. The results are shown in Table 6:

Table 6

| Cleavage efficiency (%) | Example 25 | Example 26 | Example 27 | Comparative Example 5 |
|---|---|---|---|---|
| Standing for 0 s | 72 | 98 | 100 | 50 |
| Standing for 2 s | 82 | 100 | 100 | 65 |

Table 6 shows that on the premise that the THPP concentration is unchanged, the cleavage efficiency is increased along with the increase of the concentration of sodium tetrachloropalladate in the mixed solution.

**Examples 28-31**

A method for reducing azidomethoxy is as follows:

THPP and sodium tetrachloropalladate were placed in Tris buffer containing NaCl to form mixed solutions S28-S31 (the mixed solution in Example 28 was labeled as S28, the mixed solution in Example 29 was labeled as S29, the mixed solution in Example 30 was labeled as S30, and the mixed solution in Example 31 was labeled as S31), and the content of each component in the mixed solutions S28-S31 was regulated as follows: 100 mmol/L of THPP, 10 mmol/L of sodium tetrachloropalladate, 2.0 mol/L of NaCl, and 200 mmol/L of $Na_2CO_3$. The concentrations of Tris were as follows: the concentration of Tris buffer in Example 28 was 50 mmol/L, the concentration of Tris buffer in Example 29 was 100 mmol/L, the concentration of Tris buffer in Example 30 was 150 mmol/L, and the concentration of Tris buffer in Example 31 was 200 mmol/L. The pH value of each mixed solution was adjusted to 9.0 with hydrochloric acid.

5'-Triphosphate-3'-O-azidomethylthymine nucleotide of the above formula 3 containing a fluorophore was immobilized on the surface of a solid carrier. The mixed solutions S28-S31 were added to the surface of the solid carrier, respectively (the mixed solution S28 was added in Example 28, the mixed solution S29 was added in Example 29, the mixed solution S30 was added in Example 30, and the mixed solution S31 was added in Example 31), and the mixed solutions S28-S31 were allowed to flow through the 5'-triphosphate-3'-O-azidomethylthymine nucleotide via a channel structure. The time for adding the mixed solutions S28-S31 was 23 s (taking a certain site on the surface of the solid carrier as an example, the time for the mixed solutions S28-S31 to flow through the site was 23 s). During the addition, the mixed solutions S28-S31 were heated to 60 °C and left to stand at this temperature for 2 s and 5 s to reduce -O-azidomethyl in the 5'-triphosphate-3'-O-azidomethylthymine nucleotide into -OH.

The cleavage efficiencies of Examples 28-31 were calculated. The results are shown in Table 7:

Table 7

| Cleavage efficiency (%) | Example 28 | Example 29 | Example 30 | Example 31 |
|---|---|---|---|---|
| Standing for 2 s | 69% | 66% | 67% | 63% |
| Standing for 5 s | 73% | 71% | 71% | 67% |

With reference to Table 7, it is found by comparing Examples 28-31 that, under otherwise identical conditions, when the concentration of Tris buffer is 50-200 mmol/L, the cleavage efficiencies of reducing -O-azidomethyl in the 5'-triphosphate-3'-O-azidomethylthymine nucleotide into -OH are not greatly different, and when the concentration of Tris buffer is 50 mmol/L, the cleavage efficiency is relatively high.

Under otherwise unchanged conditions, the reaction temperature of the mixed solutions S28-S31 was adjusted to 70 °C and the mixed solutions were left to stand for 2 s. The cleavage efficiencies were 85%, 84%, 83%, and 79%, respectively. Comparing with the reaction temperature of 60 °C, it can be found that: the reaction temperature is increased, and the cleavage efficiency is improved.

**Examples 32-35**

A method for reducing azidomethoxy is as follows:
THPP and sodium tetrachloropalladate were placed in Tris containing NaCl to form mixed solutions S32-S35 (the mixed solution in Example 32 was labeled as S32, the mixed solution in Example 33 was labeled as S33, the mixed solution in Example 34 was labeled as S34, and the mixed solution in Example 35 was labeled as S35). The concentrations of Tris in the mixed solutions S32-S35 were regulated as follows: the concentration of Tris in Example 32 was 50 mmol/L, the concentration of Tris in Example 33 was 100 mmol/L, the concentration of Tris in Example 34 was 150 mmol/L, and the concentration of Tris in Example 35 was 100 mmol/L. THPP was 100 mmol/L, sodium tetrachloropalladate was 10 mmol/L, and NaCl was 2.0 mol/L. The pH value of each mixed solution was adjusted to 9.2 with hydrochloric acid.

5'-Triphosphate-3'-O-azidomethylthymine nucleotide of the above formula 3 containing a fluorophore was immobilized on the surface of a solid carrier. The mixed solutions S32-S35 were added to the surface of the solid carrier, respectively (the mixed solution S32 was added in Example 32, the mixed solution S33 was added in Example 33, the mixed solution S34 was added in Example 34, and the mixed solution S35 was added in Example 35), and the mixed solutions S32-S35 were allowed to flow through the 5'-triphosphate-3'-O-azidomethylthymine nucleotide via a channel structure. The time for adding the mixed solutions S32-S35 was 23 s (taking a certain site on the surface of the solid carrier as an example, the time for the mixed solutions S32-S35 to flow through the site was 23 s). During the addition, the mixed solutions S32-S35 were heated to 70 °C and left to stand at this temperature for 0 s to reduce -O-azidomethyl in the 5'-triphosphate-3'-O-azidomethylthymine nucleotide into -OH.

The cleavage efficiencies of Examples 32-35 were calculated to be 85%, 84%, 83%, and 79%, respectively.

Comparing the cleavage efficiencies of Examples 32-35 with those of Examples 27-30 when the mixed solutions were left to stand for 0 s, it is found that: the reduction efficiency of azidomethoxy is improved when the temperature is increased to 70 °C compared with the reaction temperature of 60 °C.

**Examples 36-40**

A method for reducing azidomethoxy is as follows:
THPP and sodium tetrachloropalladate were placed in Tris buffer containing NaCl to form mixed solutions S36-S40 (the mixed solution in Example 36 was labeled as S36, the mixed solution in Example 37 was labeled as S37, the mixed solution in Example 38 was labeled as S38, the mixed solution in Example 39 was labeled as S39, and the mixed solution in Example 40 was labeled as S40), and the content of each component in the mixed solutions S36-S40 was regulated as follows: 100 mmol/L of THPP, 10 mmol/L of Tris, and 2.0 mol/L of NaCl. The concentrations of sodium tetrachloropalladate were as follows: the concentration of sodium tetrachloropalladate in Example 36 was 2.0 mmol/L, the concentration of sodium tetrachloropalladate in Example 37 was 5.0 mmol/L, the concentration of sodium tetrachloropalladate in Example 38 was 10 mmol/L, the concentration of sodium tetrachloropalladate in Example 39 was 20 mmol/L, and the concentration of sodium tetrachloropalladate in Example 40 was 40 mmol/L. The pH value of each mixed solution was adjusted to 9.2 with hydrochloric acid.

5'-Triphosphate-3'-O-azidomethylthymine nucleotide of the above formula 3 containing a fluorophore was immobilized on the surface of a solid carrier. The mixed solutions S36-S40 were added to the surface of the solid carrier, respectively (the mixed solution S36 was added in Example 36, the mixed solution S37 was added in Example 37, the mixed solution S38 was added in Example 38, the mixed solution S39 was added in Example 39, and the mixed solution S40 was added in Example 40), and the mixed solutions S36-S40 were allowed to flow through the 5'-triphosphate-3'-O-azidomethylthymine nucleotide via a channel structure. The time for adding the mixed solutions S36-S40 was 23 s (taking a certain site on the surface of the solid carrier as an example, the time for the mixed solutions S36-S40 to flow through the site was 23 s). During the addition, the mixed solutions S36-S40 were heated to a specified temperature (53 °C/55 °C/60 °C) and left to stand at this temperature for 0 s and 2 s to reduce -O-azidomethyl in the 5'-triphosphate-3'-O-azidomethylthymine nucleotide into -OH.

The cleavage efficiencies of Examples 36-40 at different specified temperatures were calculated. The results are shown in Table 8:

Table 8

| Cleavage efficiency (%) | Example 36 | Example 37 | Example 38 | Example 39 | Example 40 |
|---|---|---|---|---|---|
| Standing at 53 °C for 0 s | - | 54 | 72 | 89 | 21 |
| Standing at 55 °C for 0 s | 51 | 78 | 90 | 95 | 47 |
| Standing at 60 °C for 0 s | 85 | 96 | 99 | 100 | 76 |
| Standing at 60 °C for 2 s | 93 | - | - | - | - |

As can be seen from Table 8, under otherwise identical conditions, when the concentration of sodium tetrachloropalladate is increased, the cleavage efficiency of reducing -O-azidomethyl in the 5'-triphosphate-3'-O-azidomethylthymine nucleotide into -OH is improved; when the ratio of THPP to sodium tetrachloropalladate is 5: 1, the cleavage efficiency is highest; when the concentration ratio of the two is 5:2, an inflection point appears.

**Example 41**

A sequencing method is as follows:

a, four types of 3'-O-azidomethyl-dNTP containing a fluorophore (Dye) of the following formula 3 (B was A, T, G, and C, respectively) were used as substrates, a biochip on which a nucleic acid template is immobilized as a carrier, and a part of the sequence at one end of the nucleic acid template complementarily bound to a primer;

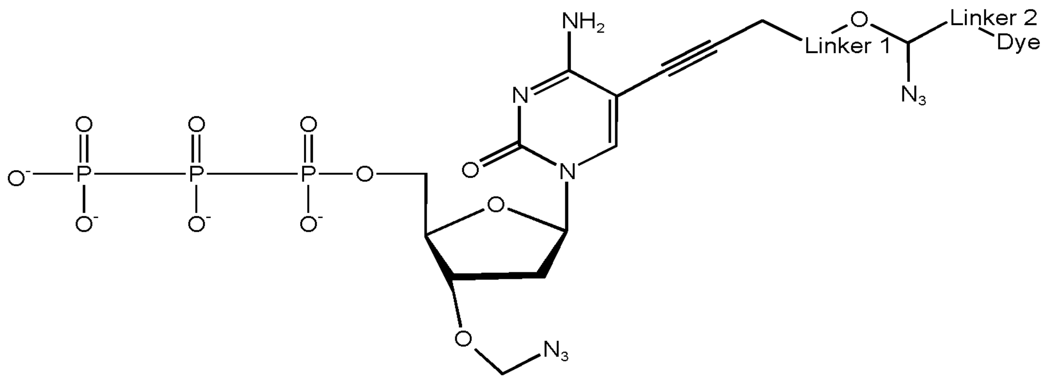

Formula 3

b, 3'-O-azidomethyl-dNTP containing the fluorophore and an extension reagent for a polymerase flowed through the surface of the biochip, and 3'-O-azidomethyl-dNTP containing the fluorophore bound to a complementary strand of the nucleic acid template by adopting single-read sequencing to obtain an extension product;
c, fluorescence of 3'-O-azidomethyl-dNTP containing the fluorophore was activated, and a fluorescence signal generated on the biochip was acquired by a fluorescence microscope after each addition of 3'-O-azidomethyl-dNTP containing the fluorophore;
d, a mixed solution S41 was added, and 3'-O-azidomethyl of 3'-O-azidomethyl-dNTP containing the fluorophore was reduced into 3'-OH-dNTP; in this process, the mixed solution S41 flowed through the surface of the biochip and was in contact with the biochip for 23 s. In the contact process, the mixed solution S41 was heated to 60 °C and left to stand at this temperature for 0 s, wherein the components and concentrations of the mixed solution S41 were as follows: 100 mmol/L of THPP, 10 mmol/L of sodium tetrachloropalladate, 150 mmol/L of ethanolamine, and 2 mol/L of NaCl, and the pH value of the mixed solution S41 was adjusted to 9.2 with hydrochloric acid;
e, the biochip obtained after step d was cleaned with a cleaning reagent; and
g, steps b to e were repeated for 150 cycles.

**Examples 42-45**

A sequencing method is different from that of Example 41 in that: the mixed solutions were heated to 40 °C (Example 42), 45 °C (Example 43), 55 °C (Example 44), and 70 °C (Example 45), respectively.
Q30, phasing, and prephasing (also referred to as prephase) in the sequencing method provided in Examples 42-45 were counted, wherein the phasing was used to record a phasing reaction rate (bases that should have participated

in the reaction in the N-th cycle of sequencing, but participate in the reaction in the N + 1-th cycle of sequencing), and the phasing could reflect the cleavage efficiency of azidomethoxy on nucleotides. Specifically, when azidomethoxy was not completely cleaved, the nucleotides at the end of a sequencing strand at partial sites were nucleotides with blocked polymerization reactivity; then, nucleotides introduced during the next cycle of sequencing could not participate in the polymerization reaction at these sites, and thus sequencing data could not be generated at the sites during this cycle of sequencing, resulting in phasing in the sequencing. Thus, increased phasing indicates a decreased cleavage efficiency; conversely, decreased phasing indicates an increased cleavage efficiency;

the prephasing was used to record a prephasing reaction rate (bases that should have participated in the reaction in the N-th cycle of sequencing, but had participated in the reaction in the N - 1-th cycle of sequencing in advance);

Q30 means the ratio that the accuracy rate of sequenced bases is 99.9%; for example, Q30 ≥ 85 means that the ratio of the sequenced bases with an accuracy rate of 99.9% is greater than or equal to 85%.

The sequencing results in Examples 41-45 are shown in Table 9 below:

Table 9

| Experimental conditions | Experimental conditions | Q30 | phasing | prephasing |
|---|---|---|---|---|
| Example 42 (40 °C) | SE150 | 72.89 | 0.30 | 0.21 |
| Example 43 (45 °C) | SE150 | 79.84 | 0.17 | 0.17 |
| Example 44 (55 °C) | SE150 | 85.13 | 0.14 | 0.09 |
| Example 41 (60 °C) | SE150 | 88.15 | 0.14 | 0.12 |
| Example 45 (70 °C) | SE150 | 87.97 | 0.12 | 0.11 |

**Example 46**

A sequencing method is different from that of Example 41 in that: the pH value of the mixed solution was 9.4. In Example 46, Q30 was 87.97, the phasing was 0.12, and the prephasing was 0.11.

**Example 47**

A sequencing method is as follows:

a, four types of 3'-O-azidomethyl-dNTP containing a fluorophore (Dye) of the above formula 3 (B was A, T, G, and C, respectively) were used as substrates, a biochip on which a nucleic acid template is immobilized as a carrier, and at least a part of the nucleic acid template on the surface of the biochip complementarily bound to a primer;

b, 3'-O-azidomethyl-dNTP containing the fluorophore and an extension reagent for a polymerase flowed through the surface of the biochip, and 3'-O-azidomethyl-dNTP containing the fluorophore bound to a complementary strand of the nucleic acid template by adopting single-read sequencing to obtain an extension product;

c, fluorescence of 3'-O-azidomethyl-dNTP containing the fluorophore was activated, and a fluorescence signal generated on the biochip was acquired by a fluorescence microscope after each addition of 3'-O-azidomethyl-dNTP containing the fluorophore;

d, a mixed solution S47 was added, -O-azidomethyl of -O-azidomethyl-dNTP containing the fluorophore was reduced into 3'-OH-dNTP, and the mixed solution S47 was allowed to flow through the surface of the biochip and be in contact with the biochip for 23 s. In the contact process, the mixed solution S47 was heated to 60 °C and left to stand at this temperature for 0 s, wherein the components and concentrations of the mixed solution S47 were as follows: 100 mmol/L of THPP, 10 mmol/L of sodium tetrachloropalladate, 50 mmol/L of Tris, and 2 mol/L of NaCl, and the pH value of the mixed solution S47 was adjusted to 9.2 with hydrochloric acid;

e, the biochip obtained after step d was cleaned with cystamine at a concentration of 20 mmol/L; and

g, steps b to e were repeated for 150 cycles.

**Comparative Example 6**

A sequencing method is different from that of Example 41 in that the mixed solution used in step d is a mixed solution D6. The components and concentrations of the mixed solution D6 were as follows: 100 mmol/L of THPP, 150 mmol/L of ethanolamine, and 2 mol/L of NaCl.

**Comparative Example 7**

A sequencing method is different from that of Example 41 in that the mixed solution used in step d is a mixed solution D7. The components and concentrations of the mixed solution D7 were as follows: 100 mmol/L of THPP, 50 mmol/L of Tris, and 2 mol/L of NaCl.

The fluorescence images of step c and step e in Example 41 acquired by a fluorescence microscope are shown in FIG. 3, those of step c and step e in Example 47 acquired by the fluorescence microscope are shown in FIG. 4, those of step c and step e in Comparative Example 6 acquired by the fluorescence microscope are shown in FIG. 5, and those of step c and step e in Comparative Example 7 acquired by the fluorescence microscope are shown in FIG. 6. In FIGs. 3-6, the left image is an image acquired in step c, and the right image is an image acquired in step e.

The data of the sequencing methods in Example 41, Example 47, Comparative Example 6, and Comparative Example 7 were output through the sequencing platform. The results are shown in Table 10 below:

Table 10

| | Q30 | phasing | prephasing |
|---|---|---|---|
| Example 41 | 84.92 | 0.12 | 0.13 |
| Example 47 | 86.27 | 0.14 | 0.16 |
| Comparative Example 6 | 81.71 | 0.25 | 0.22 |
| Comparative Example 7 | 88.22 | 0.17 | 0.17 |

Comparing Example 41 with Comparative Example 6, it can be seen that when the buffer system is an ethanolamine system, the addition of palladium to the mixed solution in step d can effectively increase Q30 and decrease the phasing and prephasing under otherwise identical conditions; comparing Example 47 with Comparative Example 7, it can be seen that when the buffer system is a Tris buffer system, the addition of palladium to the mixed solution in step d can decrease Q30, but the phasing and prephasing are decreased, under otherwise identical conditions.

The above description is only for the purpose of illustrating the preferred examples of the present application, and is not intended to limit the scope of the present application. Any modification, equivalent, improvement, and the like made without departing from the spirit and principle of the present application shall fall within the protection scope of the present application.

## Claims

1. A method for reducing an azidomethoxy group, wherein the method comprises:

   mixing a compound I comprising the azidomethoxy group, a palladium compound, and an organic phosphine to give a mixed system, and placing the mixed system under a condition suitable for a chemical reaction to reduce the azidomethoxy group of the compound I into hydroxy,
   wherein the azidomethoxy group has a chemical formula of $-O-CA_1(N_3)-A_2$,
   $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

2. The method according to claim 1, wherein the organic phosphine and the palladium compound in the mixed system are in a molar ratio of 2: 1 to 2000:1.

3. The method according to claim 1, wherein in the mixed system, the palladium compound is selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound.

4. The method according to claim 3, wherein in the mixed system, the palladium compound is selected from at least one of a palladium halate, a palladium halide, an organic palladium carboxylate, and an allylpalladium halide dimer.

5. The method according to claim 3, wherein the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate.

6. The method according to claim 1, wherein in the mixed system, the organic phosphine is selected from at least one of trivalent organic phosphines.

7. The method according to claim 6, wherein the organic phosphine is selected from at least one of tris(3-hydroxypropyl) phosphine, tris(2-carboxyethyl)phosphine, tris(hydroxymethyl)phosphine, triphenylphosphine, and tris(2-hydroxyethyl)phosphine.

8. The method according to any one of claims 1 to 7, wherein the chemical reaction is carried out under an alkaline condition; and/or the chemical reaction is carried out at a temperature of 30-100 °C.

9. The method according to any one of claims 1 to 8, wherein the mixed system further comprises metal ions selected from at least one of sodium ions and potassium ions.

10. The method according to claim 9, wherein the metal ions are derived from at least one of halide salts, carbonates, acetates, phosphates, and borates of the sodium ion and the potassium ion.

11. The method according to claim 9, wherein the mixed system comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

12. The method according to any one of claims 9 to 11, wherein the metal ions in the mixed system have a concentration of 0.5-2.5 mol/L.

13. The method according to any one of claims 1 to 12, wherein the mixed system further comprises a buffer.

14. The method according to claim 13, wherein the buffer is selected from at least one of a primary amine buffer and a tertiary amine buffer.

15. The method according to claim 14, wherein the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N*,*N*-dihydroxyethylglycine.

16. The method according to any one of claims 13 to 15, wherein the buffer in the mixed system has a concentration of 50-200 mmol/L.

17. The method according to any one of claims 1 to 16, wherein the compound I has a structural formula of $A_3$-O-$CA_1$($N_3$)-$A_2$, wherein $A_3$ is selected from one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

18. The method according to claim 17, wherein $A_3$ comprises at least one of ribosyl or an analog thereof, deoxyribosyl or an analog thereof, a base or an analog thereof, a phosphate group, and a detectable group.

19. The method according to claim 18, wherein $A_3$ comprises at least one of the following structures:

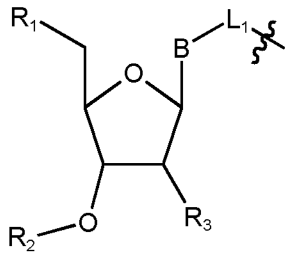

and

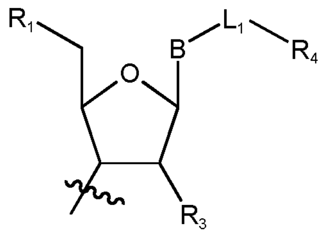

,

wherein B is a base or an analog thereof, $L_1$ is a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ contains a detectable group.

**20.** The method according to any one of claims 17 to 19, wherein at least one of $A_1$ and $A_2$ is hydrogen.

**21.** The method according to any one of claims 17 to 20, wherein $A_2$ is selected from hydrogen, or $A_2$ comprises at least one of a group capable of inhibiting a polymerization reaction of the compound I and a detectable group.

**22.** The method according to any one of claims 17 to 21, wherein $A_1$ is selected from hydrogen, and $A_2$ comprises at least one of a group capable of inhibiting the polymerization reaction of the compound I and a detectable group.

**23.** The method according to claim 22, wherein $A_2$ comprises a group capable of inhibiting the polymerization reaction of the compound I and a detectable group.

**24.** The method according to any one of claims 1 to 23, wherein the compound I is selected from at least one of the following structural formulas 1-1, 1-2, and 1-3:

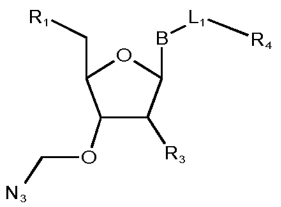

Formula 1-1

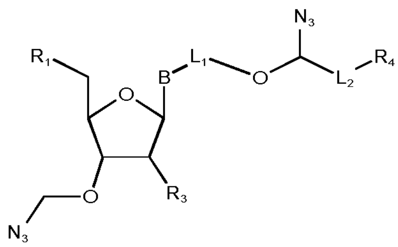

Formula 1-2

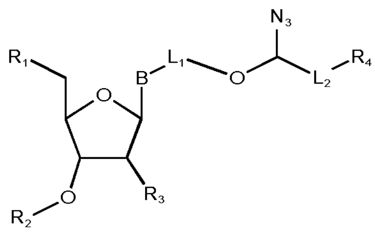

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ contains a detectable group.

**25.** The method according to claim 24, wherein the chemical reaction is carried out at a pH value of 8.0-10.0.

**26.** The method according to claim 25, wherein the chemical reaction is carried out at a pH value of 9.0-9.5.

**27.** The method according to claim 24, wherein the chemical reaction is carried out at a temperature of 40-75 °C.

**28.** The method according to claim 27, wherein the chemical reaction is carried out at a temperature of 45-70 °C.

**29.** The method according to claim 24, wherein the palladium compound and the organic phosphine in the mixed system are in a molar ratio of 5:1 to 50:1.

**30.** The method according to claim 29, wherein the palladium compound and the organic phosphine in the mixed system are in a molar ratio of 5:1 to 20:1.

**31.** The method according to any one of claims 24 to 30, wherein the mixed system comprises a buffer and metal ions selected from at least one of sodium ions and potassium ions.

**32.** The method according to claim 31, wherein the metal ions have a concentration of 1.0-2.5 mol/L.

**33.** The method according to claim 32, wherein the metal ions have a concentration of 1.2-2.0 mol/L.

**34.** The method according to any one of claims 24 to 30, wherein the buffer has a concentration of 50-180 mmol/L.

**35.** The method according to claim 34, wherein the buffer has a concentration of 100-150 mmol/L.

**36.** A sequencing method, comprising the following steps:

(a) under a condition suitable for a polymerization reaction, enabling a compound II to be incorporated into a nucleic acid template to obtain an extension product, wherein the compound II is a nucleotide analog comprising an azidomethyl group and a detectable group, and the compound II has a structural formula of formula 1-1, formula 1-2, or formula 1-3:

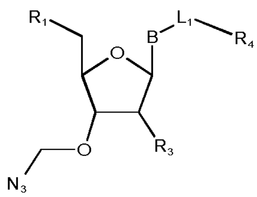

Formula 1-1

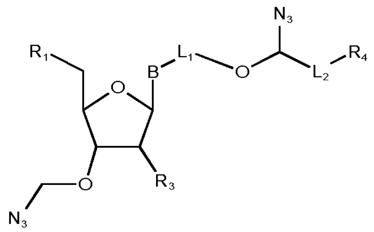

Formula 1-2

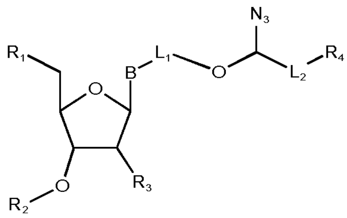

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or -$OR_5$, $R_5$ is H or a chemically cleavable group, and $R_4$ comprises the detectable group;
(b) detecting the detectable group and acquiring a detectable signal generated by the detectable group; and
(c) adding a mixed solution comprising a palladium compound and an organic phosphine and placing the mixed solution under a condition suitable for a cleavage reaction to remove the azidomethyl group on the extension product or the azidomethyl group and the detectable group on the extension product.

**37.** The method according to claim 36, wherein in step (c), the palladium compound and the organic phosphine are added in an amount satisfying: the palladium compound and the organic phosphine in a molar ratio of 2.5:1 to 100:1.

**38.** The method according to claim 37, wherein the palladium compound and the organic phosphine are in a molar ratio of 5:1 to 50:1.

**39.** The sequencing method according to claim 38, wherein the palladium compound and the organic phosphine are in a molar ratio of 5:1 to 20:1.

**40.** The sequencing method according to any one of claims 36 to 39, wherein the palladium compound is selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound.

**41.** The sequencing method according to claim 40, wherein the palladium compound is selected from at least one of a palladium halate, a palladium halide, an organic palladium carboxylate, and an allylpalladium halide dimer.

**42.** The sequencing method according to claim 40, wherein the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate.

**43.** The sequencing method according to any one of claims 36 to 39, wherein the organic phosphine is selected from at least one of trivalent organic phosphines.

**44.** The sequencing method according to claim 43, wherein the organic phosphine is selected from at least one of tris(3-hydroxypropyl)phosphine, tris(2-carboxyethyl)phosphine, tris(hydroxymethyl)phosphine, triphenylphosphine, and tris(2-hydroxyethyl)phosphine.

**45.** The sequencing method according to any one of claims 37 to 39, wherein the cleavage reaction is carried out at a pH value of 8.0-10.0.

**46.** The sequencing method according to claim 45, wherein the cleavage reaction is carried out at a pH value of 9.0-9.5.

**47.** The sequencing method according to any one of claims 37 to 46, wherein the cleavage reaction is carried out at a temperature of 40-75 °C.

**48.** The sequencing method according to claim 47, wherein the cleavage reaction is carried out at a temperature of 45-70 °C.

**49.** The sequencing method according to any one of claims 37 to 46, wherein the mixed solution further comprises metal ions selected from at least one of sodium ions and potassium ions.

**50.** The sequencing method according to claim 49, wherein the metal ions are derived from at least one of halide salts, carbonates, acetates, phosphates, and borates of the sodium ion and the potassium ion.

**51.** The sequencing method according to claim 50, wherein the mixed solution comprises at least one of sodium chloride, potassium chloride, sodium fluoride, sodium bromide, sodium iodide, sodium carbonate, sodium bicarbonate, potassium carbonate, potassium bicarbonate, sodium acetate, sodium phosphate, sodium dihydrogen phosphate, disodium hydrogen phosphate, and sodium borate.

**52.** The sequencing method according to any one of claims 50 to 51, wherein the metal ions in the mixed solution have a concentration of 0.5-2.5 mol/L.

**53.** The sequencing method according to claim 52, wherein the metal ions have a concentration of 1.0-2.5 mol/L.

**54.** The sequencing method according to claim 53, wherein the metal ions have a concentration of 1.2-2.0 mol/L.

**55.** The sequencing method according to any one of claims 49 to 54, wherein the mixed solution further comprises a buffer.

**56.** The sequencing method according to claim 55, wherein the buffer is selected from at least one of a primary amine buffer and a tertiary amine buffer.

**57.** The sequencing method according to claim 56, wherein the buffer is selected from at least one of tris(hydroxymethyl) aminomethane buffer, glycine, ethanolamine, tetraethylethylenediamine, tetramethylethylenediamine, *N*-butyldiethanolamine, diethylaminoethanol, Hepes buffer, and *N*,*N*-dihydroxyethylglycine.

**58.** The sequencing method according to any one of claims 36 to 57, wherein the nucleic acid template binds to the surface of a solid carrier, and the mixed solution flows through the surface of the solid carrier.

**59.** The sequencing method according to claim 58, wherein the solid carrier comprises a channel structure capable of communicating one end of the solid carrier to the other end, the nucleic acid template is immobilized in the channel structure, and the mixed solution flows through the surface of the channel structure.

**60.** The sequencing method according to claim 59, wherein the channel structure has a width of 5-15 mm and a height of 50-200 μm, a flow velocity of the mixed solution is 1000-2000 μL/s, and a volume of flow of the mixed solution is 50-500 μL.

**61.** The sequencing method according to any one of claims 58 to 60, wherein an oligonucleotide is immobilized on the surface of the solid carrier, the sequencing method further comprises, before performing step (a):
flowing a solution comprising the nucleic acid template through the surface of the solid carrier, so that a part of the sequence of the nucleic acid template complementarily binds to the oligonucleotide.

**62.** The sequencing method according to claim 61, further comprising:
after step (c), cleaning the sequencing system after the cleavage reaction with a cleaning reagent.

**63.** The sequencing method according to claim 62, wherein the cleaning reagent comprises a divalent palladium removal reagent, and the divalent palladium removal reagent comprises at least one of allyl, oxygen azidoalkyl, and disulfide bonds.

**64.** The sequencing method according to claim 63, wherein the divalent palladium removal reagent is selected from at least one of ethyl isocyanoacetate, cysteine or a salt thereof, glutathione, potassium isocyanoacetate, and cystamine.

**65.** The sequencing method according to claim 63 or 64, wherein the divalent palladium removal reagent has a concentration of 1-40 mmol/L.

**66.** A kit comprising a cleavage reagent, wherein the cleavage reagent is used for cleaving a compound III comprising an azidomethoxy group so as to reduce the azidomethoxy group in the compound III into hydroxy, the azidomethoxy group has a chemical formula of $-O-CA_1(N_3)-A_2$, and $A_1$ and $A_2$ are each independently selected from one of hydrogen, substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl,
the cleavage reagent comprises a palladium compound and an organic phosphine.

**67.** The kit according to claim 66, wherein the palladium compound and the organic phosphine are in a molar ratio of 2: 1 to 100:1.

**68.** The kit according to claim 66, wherein the palladium compound is selected from one of a Pd(0) compound, a monovalent palladium compound, a divalent palladium compound, a trivalent palladium compound, a tetravalent palladium compound, and a hexavalent palladium compound, or a composition formed from two or more of a Pd(0) compound, a monovalent palladium compound, a divalent palladium compound, a trivalent palladium compound, a tetravalent palladium compound, and a hexavalent palladium compound.

**69.** The kit according to claim 66, wherein the palladium compound is selected from at least one of a palladium halate, a palladium halide, an organic palladium carboxylate, and an allylpalladium halide dimer.

**70.** The kit according to claim 68, wherein the divalent palladium compound is selected from at least one of sodium tetrachloropalladate, allylpalladium(II) chloride dimer, palladium acetate, palladium dichloride, tetraamminepalladium dichloride, sodium tetranitropalladate, palladium dibromide, palladium diiodide, palladium trifluoroacetate, and palladium pivalate.

**71.** The kit according to claim 66, wherein the organic phosphine is selected from at least one of trivalent organic phosphines.

**72.** The kit according to claim 70, wherein the organic phosphine is selected from at least one of tris(3-hydroxypropyl) phosphine, tris(2-carboxyethyl)phosphine, tris(hydroxymethyl)phosphine, triphenylphosphine, and tris(2-hydroxyethyl)phosphine.

**73.** The kit according to any one of claims 66 to 72, wherein the cleavage reagent further comprises metal ions selected from at least one of sodium ions and potassium ions.

**74.** The kit according to claim 73, wherein the metal ions are derived from at least one of halide salts, carbonates, acetates, phosphates, and borates of the sodium ion and the potassium ion.

**75.** The kit according to claim 73, wherein the cleavage reagent comprises at least one of sodium chloride, potassium chloride, sodium carbonate, sodium acetate, sodium phosphate, and sodium borate.

**76.** The kit according to any one of claims 73 to 75, wherein the metal ions in the cleavage reagent have a concentration of 0.5-2.5 mol/L.

**77.** The kit according to any one of claims 67 to 76, wherein the cleavage reagent further comprises a buffer.

**78.** The kit according to claim 77, wherein the buffer is selected from at least one of a primary amine buffer and a tertiary amine buffer.

**79.** The kit according to claim 78, wherein the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N*,*N*-dihydroxyethylglycine.

**80.** The kit according to any one of claims 66 to 79, wherein the compound III is selected from at least one of the following structural formulas 1-1, 1-2, and 1-3:

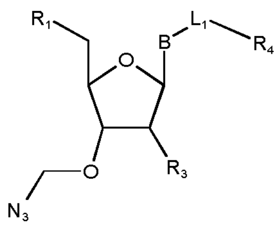

Formula 1-1

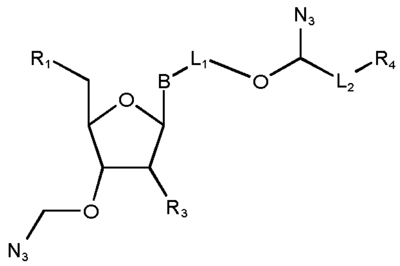

Formula 1-2

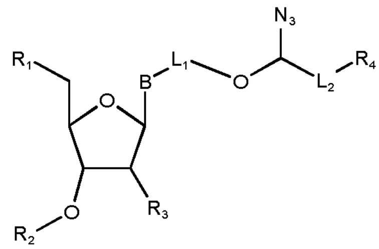

Formula 1-3

,

wherein B is a base or an analog thereof, $L_1$ and $L_2$ are each independently a covalent bond or a covalent linker group, $R_1$ is -OH or a phosphate group, $R_2$ is H or a chemically cleavable group, $R_3$ is H or $-OR_5$, $R_5$ is H or a chemically cleavable group, and $R_4$ contains a detectable group.

**81.** The kit according to claim 80, wherein the palladium compound and the organic phosphine are in a molar ratio of 5:1 to 50:1.

**82.** The kit according to claim 81, wherein the palladium compound and the organic phosphine are in a molar ratio of 5:1 to 20:1.

**83.** The kit according to claim 81 or 82, wherein the cleavage reagent comprises a buffer and metal ions selected from at least one of sodium ions and potassium ions.

**84.** The kit according to claim 83, wherein the metal ions have a concentration of 1.0-2.5 mol/L.

**85.** The kit according to claim 84, wherein the metal ions have a concentration of 1.2-2.0 mol/L.

**86.** The kit according to any one of claims 83 to 85, wherein the buffer has a concentration of 50-180 mmol/L.

**87.** The kit according to claim 86, wherein the buffer has a concentration of 100-150 mmol/L.

**88.** The kit according to any one of claims 66 to 87, further comprising a cleaning reagent, wherein the cleaning reagent comprises a divalent palladium removal reagent, and the divalent palladium removal reagent comprises at least one of allyl, oxygen azidoalkyl, and disulfide bonds.

**89.** The kit according to claim 88, wherein the divalent palladium removal reagent is selected from at least one of ethyl isocyanoacetate, cysteine or a salt thereof, glutathione, potassium isocyanoacetate, and cystamine.

**90.** The kit according to claim 88 or 89, wherein the divalent palladium removal reagent has a concentration of 1-40 mmol/L.

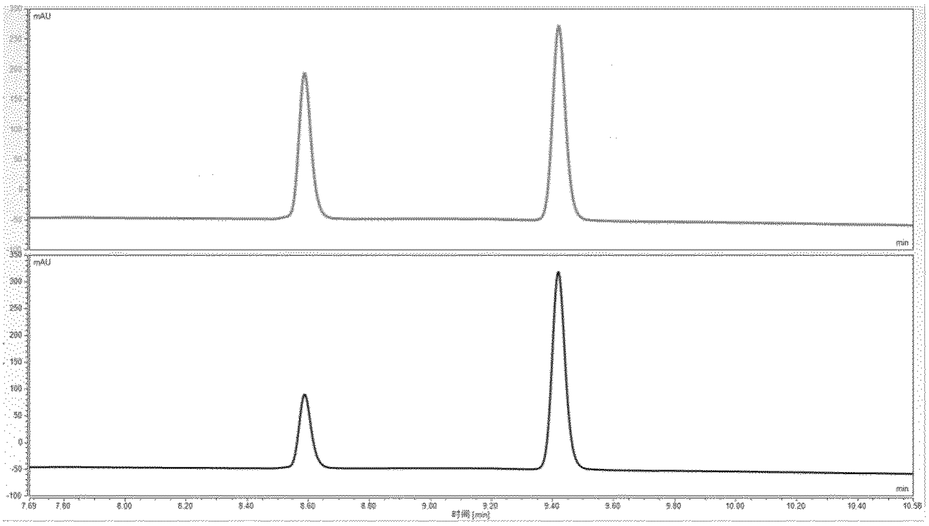


FIG. 1

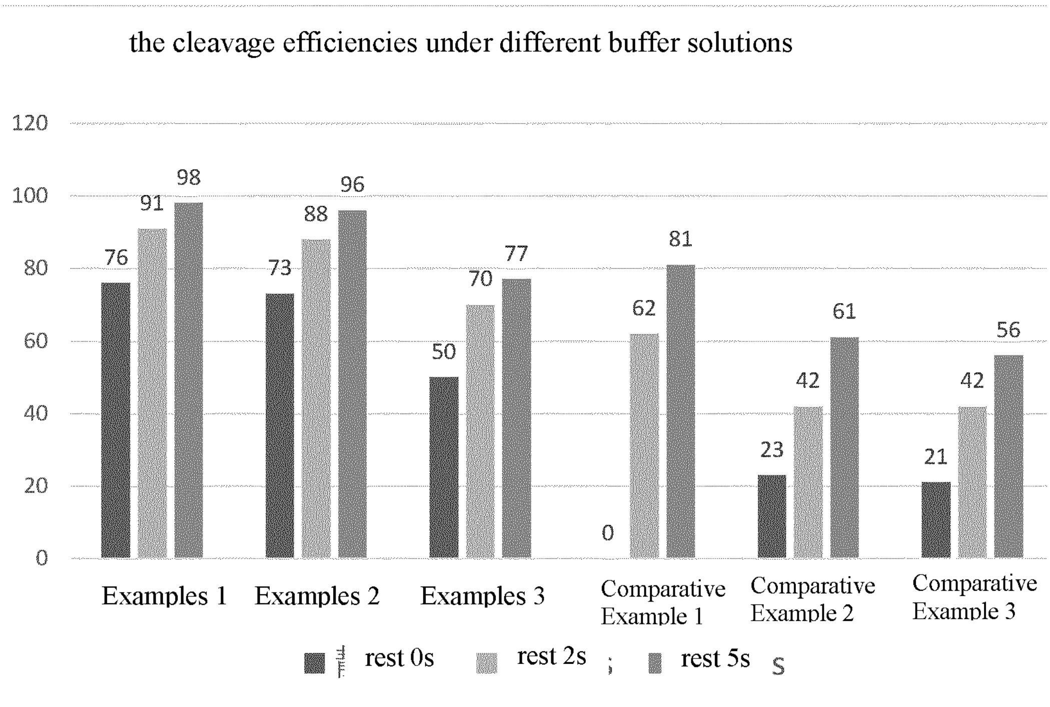


FIG. 2

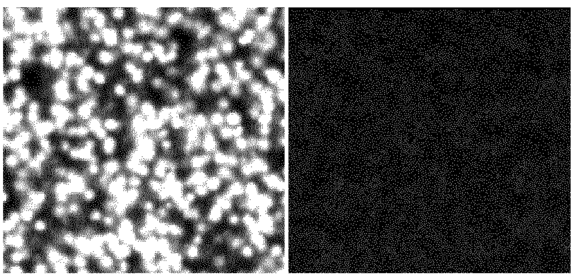

FIG. 3

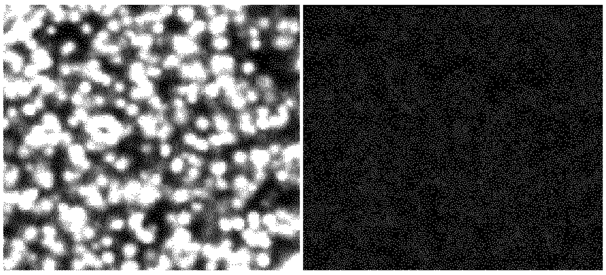

FIG. 4

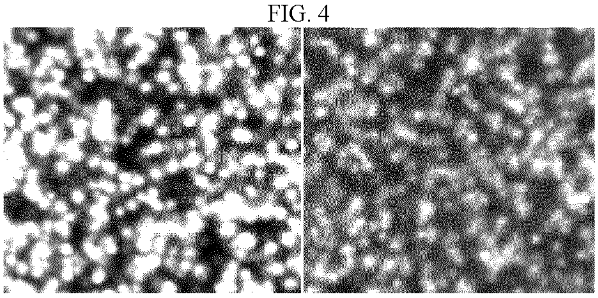

FIG. 5

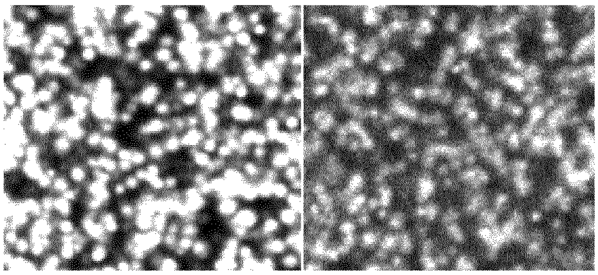

FIG. 6

**INTERNATIONAL SEARCH REPORT**

International application No.
**PCT/CN2022/123981**

**A. CLASSIFICATION OF SUBJECT MATTER**

C07H1/02(2006.01)i；C07H19/10(2006.01)i；C07H19/20(2006.01)i；C07H21/00(2006.01)i；C12Q1/6869(2018.01)i

According to International Patent Classification (IPC) or to both national classification and IPC

**B. FIELDS SEARCHED**

Minimum documentation searched (classification system followed by classification symbols)

C07H, C12Q

Documentation searched other than minimum documentation to the extent that such documents are included in the fields searched

Electronic data base consulted during the international search (name of data base and, where practicable, search terms used)

CNTXT, CNKI, VEN: 叠氮, 膦, 钯, 还原, 测序, 边合成边测序, SBS, azide, phosphine, palladium, reduction, sequence, synthesis

**C. DOCUMENTS CONSIDERED TO BE RELEVANT**

| Category* | Citation of document, with indication, where appropriate, of the relevant passages | Relevant to claim No. |
|---|---|---|
| X | WO 2021259881 A1 (ILLUMINA CAMBRIDGE LIMITED) 30 December 2021 (2021-12-30)<br>claims 1-52, and description, paragraph 93, 100, 103-104, and 131 | 1-90 |
| X | CN 112638925 A (ILLUMINA CAMBRIDGE LIMITED) 09 April 2021 (2021-04-09)<br>claims 1-50, and description, paragraphs 132-139 and 190-212 | 1-90 |
| A | CN 111741967 A (GENEMIND BIOSCIENCES CO., LTD.) 02 October 2020 (2020-10-02)<br>entire document | 1-90 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 July 2023** | **08 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2022/123981**

| Patent document cited in search report | | | Publication date (day/month/year) | Patent family member(s) | | | Publication date (day/month/year) |
|---|---|---|---|---|---|---|---|
| WO | 2021259881 | A1 | 30 December 2021 | US | 2021403500 | A1 | 30 December 2021 |
| | | | | IL | 297992 | A | 01 January 2023 |
| | | | | KR | 20230031837 | A | 07 March 2023 |
| | | | | AU | 2021295081 | A1 | 08 December 2022 |
| | | | | CA | 3177293 | A1 | 30 December 2021 |
| | | | | CN | 115867560 | A | 28 March 2023 |
| | | | | IN | 202217062828 | A | 03 February 2023 |
| CN | 112638925 | A | 09 April 2021 | US | 2022220553 | A1 | 14 July 2022 |
| | | | | WO | 2020136170 | A2 | 02 July 2020 |
| | | | | WO | 2020136170 | A3 | 13 August 2020 |
| | | | | KR | 20210109439 | A | 06 September 2021 |
| | | | | BR | 112020026668 | A2 | 30 March 2021 |
| | | | | CA | 3103634 | A1 | 02 July 2020 |
| | | | | JP | 2022515944 | A | 24 February 2022 |
| | | | | SG | 11202012557 | XA | 28 January 2021 |
| | | | | EP | 3902814 | A2 | 03 November 2021 |
| | | | | AU | 2019412494 | A1 | 07 January 2021 |
| | | | | US | 2020216891 | A1 | 09 July 2020 |
| | | | | US | 11293061 | B2 | 05 April 2022 |
| | | | | HK | 40055929 | A0 | 18 March 2022 |
| | | | | IN | 202017056222 | A | 25 June 2021 |
| | | | | RU | 2020141465 | A | 26 January 2023 |
| CN | 111741967 | A | 02 October 2020 | EP | 3733683 | A1 | 04 November 2020 |
| | | | | EP | 3733683 | A4 | 06 April 2022 |
| | | | | US | 2020283467 | A1 | 10 September 2020 |
| | | | | US | 11512106 | B2 | 29 November 2022 |
| | | | | WO | 2019105421 | A1 | 06 June 2019 |
| | | | | HK | 40031499 | A0 | 12 March 2021 |
| | | | | HK | 40033464 | A0 | 09 April 2021 |

Form PCT/ISA/210 (patent family annex) (July 2022)